# EUROPEAN PATENT APPLICATION

(11) **EP 2 163 243 A1**
(43) Date of publication of application: **17.03.2010**
(21) Application number: 08016137.5
(22) Date of filing: 12.09.2008
(51) Int. Cl.: A61K 9/50, C12N 5/00, A61P 9/00

(54) **Treatment of acute myocardial infarction (AMI) using encapsulated cells encoding and secreting GLP-1 peptides or analogs thereof**

(71) Applicant: Biocompatibles UK Limited, Farnham Surrey GU9 8QL (GB)
(72) Inventor: Wallrapp, Christine Dr., 63762 Grosstheim (DE); Thoenes, Eric, 63654 Büdingen (DE); Lewis, Andrew Lennard, GU 9AA Farnham, Surrey (GB); Stratford, Peter William c/o Biocompatibles UK Ltd., Farnham, Surrey GU9 8QL (GB)
(74) Representative: Peters, Hajo

(57) **Abstract**

The present application refers to the use of cells, e.g. mesenchymal stem cells or mesenchymal stromal cells, or any further suitable cell, encoding and secreting GLP-1, a fragment or variant thereof or a fusion peptide comprising GLP-1 or a fragment or variant thereof, for the treatment of acute myocardial infarction (AMI or MI), wherein the cells, encoding and secreting GLP-1, a fragment or variant thereof or a fusion peptide comprising GLP-1 or a fragment or variant thereof, are encapsulated in a (spherical) microcapsule to prevent a response of the immune system of the patient to be treated. The present application also refers to the use of these (spherical) microcapsule(s) or of a pharmaceutical composition containing these cells or (spherical) microcapsule(s) for the treatment of acute myocardial infarction (AMI or MI).

SEQUENCE LISTING

<110>  Biocompatibles UK Limited

<120>  Treatment of acute myocardial infarction (AMI) using (spherical)
       microcapsules encoding and secreting GLP-1 peptides or analogs thereof

<130>  BI07P007EP

<140>
<141>

<160>  26

<170>  PatentIn version 3.3

<210>  1
<211>  31
<212>  PRT
<213>  Artificial

<220>
<223>  Description of sequence:  synthetic peptide corresponding to
       GLP-1(7-37)

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  15
<212>  PRT
<213>  Artificial

<220>
<223>  Description of sequence: full-length IP-2 sequence having all 15
       amino acids of the naturally occurring IP-2 sequence, human;

<400>  2

Arg Arg Asp Phe Pro Glu Glu Val Ala Ile Val Glu Glu Leu Gly
1               5                   10                  15

<210>  3
<211>  15
<212>  PRT
<213>  Artificial

<220>
<223>  Description of sequence: full-length IP-2 sequence having all 15
       amino acids of the naturally occurring IP-2 sequence, murine;

<400>  3

Arg Arg Asp Phe Pro Glu Glu Val Ala Ile Ala Glu Glu Leu Gly
1               5                   10                  15

<210>  4
<211>  35

<212>  PRT
<213>  Artificial

<220>
<223>  Description of sequence: murine isoform of GLP-2

<400>  4

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp Arg Lys
        35

<210>  5
<211>  35
<212>  PRT
<213>  Artificial

<220>
<223>  Description of sequence: human isoform of GLP-2

<400>  5

His Ala Asp Gly Ser Phe Ser Asp Glu Met Ser Thr Ile Leu Asp Asn
1               5                   10                  15

Leu Ala Thr Arg Asp Phe Ile Asn Trp Leu Ile Gln Thr Lys Ile Thr
            20                  25                  30

Asp Lys Lys
        35

<210>  6
<211>  79
<212>  PRT
<213>  Artificial

<220>
<223>  Description of sequence: SEQ ID No: 6 (ID6syn, CM1) corresponds
       to GLP-1(7-37)-IP2-RR-GLP1(7-37), 79 aa, 8,7 kD

<400>  6

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  7
<211>  83
<212>  PRT
<213>  Artificial

<220>
<223>  Description of sequence: SEQ ID No: 7 (ID7rec, CM2) corresponds
       to GLP-1(7-37)-IP2-RR-GLP2, 83 aa, 9,4 kD

<400>  7

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Asp Gly Ser Phe Ser Asp Glu Met Ser Thr Ile Leu Asp Asn
    50                  55                  60

Leu Ala Thr Arg Asp Phe Ile Asn Trp Leu Ile Gln Thr Lys Ile Thr
65                  70                  75                  80

Asp Lys Lys

<210>  8
<211>  46
<212>  PRT
<213>  Artificial

<220>
<223>  Description of sequence: SEQ ID No:8 (ID8 syn, CM3) corresponds
       to GLP-1(7-37)-IP2, 46 aa, 5,1 kD;

<400>  8

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly
        35                  40                  45

<210>  9
<211>  97
<212>  PRT
<213>  Artificial

<220>
<223>  Description of sequence: artificial

<400>  9

Met Ala Pro Ala Ala Trp Leu Arg Ser Ala Ala Ala Arg Ala Leu Leu
1               5                   10                  15

Pro Pro Met Leu Leu Leu Leu Leu Gln Pro Pro Pro Leu Leu Ala Arg
            20                  25                  30

Ala Leu Pro Pro Asp Val His His Leu His Ala Glu Arg Arg Gly Pro
        35                  40                  45

Gln Pro Trp His Ala Ala Leu Pro Ser Ser Pro Ala Pro Ala Pro Ala
    50                  55                  60

Thr Gln Glu Ala Pro Arg Pro Ala Ser Ser Leu Arg Pro Pro Arg Cys
65                  70                  75                  80

Gly Val Pro Asp Pro Ser Asp Gly Leu Ser Ala Arg Asn Arg Gln Lys
                85                  90                  95

Arg

<210>  10
<211>  79
<212>  PRT
<213>  Artificial

<220>
<223>  Description of sequence: SEQ ID No: 10
       (N-GLP-1(7-37)-IP2(human)-RR-GLP-1(7-37)-C, also designated human
       CM1)

<400>  10

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  11
<211>  83
<212>  PRT

<213>  Artificial

<220>
<223>  Description of sequence: SEQ ID No: 11
       (N-GLP-1(7-37)-IP2(human)-RR-GLP-2-C), also designated human CM2
       herein)

<400>  11

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Asp Gly Ser Phe Ser Asp Glu Met Asn Thr Ile Leu Asp Asn
    50                  55                  60

Leu Ala Ala Arg Asp Phe Ile Asn Trp Leu Ile Gln Thr Lys Ile Thr
65                  70                  75                  80

Asp Arg Lys


<210>  12
<211>  46
<212>  PRT
<213>  Artificial

<220>
<223>  Description of sequence: SEQ ID No: 12, GLP-1(7-37) linked
       without any linker sequence via its C-terminus to human IP2

<400>  12

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly
        35                  40                  45


<210>  13
<211>  815
<212>  DNA
<213>  Artificial

<220>
<223>  Description of sequence: (SEQ ID No:13) represents the
       translated peptide sequence of the construct according to Fig. 1k
       (SEQ ID No: 14)


<220>

<221>  CDS
<222>  (11)..(118)

<220>
<221>  CDS
<222>  (387)..(809)

<400>  13
   gatatccacc atg gcc ccc gcc gcc tgg ctg agg agc gcc gcc gcc agg      49
              Met Ala Pro Ala Ala Trp Leu Arg Ser Ala Ala Ala Arg
              1               5                   10
   gcc ctg ctg cca ccc atg ctg ctg ctg ctg ctg cag ccc cca cct ctg       97
   Ala Leu Leu Pro Pro Met Leu Leu Leu Leu Leu Gln Pro Pro Pro Leu
       15                  20                  25
   ctg gcc cgg gcc ctg ccc ccg gtgagtgccc gccactcgcc gtccgctcct      148
   Leu Ala Arg Ala Leu Pro Pro
   30                  35
   cgctgagggg gcgccgggca cgcgggctgg gcccagcggc gtatccggac gccaagaaac      208
   cagagagcca gccagatgcc aaagggccct gccatgtgcc ggtgcccttt ccctctccat      268
   ttgccctgcc acacagtggg ctggggttgc acgtgtgttt gctgacaggc cacatctcta      328
   actgtgggcc atgtggacct taggcctgac cagaccctca tgtcttcctc cttcccag      386
   gac gtg cac cac ctg cac gcc gag agg cgc ggc cct cag ccc tgg cac      434
   Asp Val His His Leu His Ala Glu Arg Arg Gly Pro Gln Pro Trp His
               40                  45                  50
   gcc gcc ctg cca agc agc cct gcc cct gcc cca gcc acc cag gag gcc      482
   Ala Ala Leu Pro Ser Ser Pro Ala Pro Ala Pro Ala Thr Gln Glu Ala
           55                  60                  65
   ccc agg cct gcc agc agc ctg agg cca ccc agg tgc ggc gtg cct gat      530
   Pro Arg Pro Ala Ser Ser Leu Arg Pro Pro Arg Cys Gly Val Pro Asp
       70                  75                  80
   ccc tcc gat ggc ctg agc gct cgg aat cgg cag aag agg cac gcc gag      578
   Pro Ser Asp Gly Leu Ser Ala Arg Asn Arg Gln Lys Arg His Ala Glu
   85                  90                  95                  100
   ggc acc ttc acc tcc gac gtg agc agc tac ctg gag ggc cag gcc gcc      626
   Gly Thr Phe Thr Ser Asp Val Ser Ser Tyr Leu Glu Gly Gln Ala Ala
                   105                 110                 115
   aag gag ttc atc gcc tgg ctg gtg aag ggc agg ggc cgc agg gac ttc      674
   Lys Glu Phe Ile Ala Trp Leu Val Lys Gly Arg Gly Arg Arg Asp Phe
               120                 125                 130
   cct gag gag gtg gcc atc gtg gag gag ctg ggc cgg cga cac gcc gag      722
   Pro Glu Glu Val Ala Ile Val Glu Glu Leu Gly Arg Arg His Ala Glu
           135                 140                 145
   ggc acc ttc acc tcc gac gtg agc agc tac ctg gag ggc cag gcc gcc      770
   Gly Thr Phe Thr Ser Asp Val Ser Ser Tyr Leu Glu Gly Gln Ala Ala
       150                 155                 160
   aag gag ttc atc gcc tgg ctg gtg aag ggc agg ggc tga gcgcgc      815
   Lys Glu Phe Ile Ala Trp Leu Val Lys Gly Arg Gly
   165                 170                 175

<210>  14
<211>  815
<212>  DNA

<213>  Artificial

<220>
<223>  Description of sequence: DNA sequence of the construct according
      to Fig. 1k

<400>  14
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

agggcacctt cacctccgac gtgagcagct acctggaggg ccaggccgcc aaggagttca     780

tcgcctggct ggtgaagggc aggggctgag cgcgc                                815

<210>  15
<211>  834
<212>  DNA
<213>  Artificial

<220>
<223>  Description of sequence: DNA sequence and sequence of the
      translated peptide according to the construct Fig. 1h


<220>
<221>  CDS
<222>  (11)..(118)

<220>
<221>  CDS
<222>  (387)..(821)

<400>  15
gatatccacc atg gcc ccc gcc gcc tgg ctg agg agc gcc gcc gcc agg        49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg        97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct         148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac 722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg 770
Gly Ser Phe Ser Asp Glu Met Asn Thr Ile Leu Asp Asn Leu Ala Ala
    150                 155                 160

cgc gac ttc atc aac tgg ctg atc cag acc aag atc acc gat cgg aag 818
Arg Asp Phe Ile Asn Trp Leu Ile Gln Thr Lys Ile Thr Asp Arg Lys
165                 170                 175                 180

tga gcgcgctgat atc 834

<210> 16
<211> 834
<212> DNA
<213> Artificial

<220>
<223> Description of sequence: DNA sequence of the construct according
to Fig. 1h

<400> 16
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

gccgcaggga cttccctgag gaggtggcca tcgtggagga gctgggccgg cgacacgccg 720

acggcagctt cagcgacgag atgaacacca tcctggacaa cctggccgcg cgcgacttca 780

tcaactggct gatccagacc aagatcaccg atcggaagtg agcgcgctga tatc 834

<210> 17
<211> 780
<212> DNA
<213> Artificial

<220>
<223> Description of sequence: DNA sequence sequence and translated
      peptide sequence of the construct according to Fig. 1l,

<220>
<221> CDS
<222> (11)..(118)

<220>
<221> CDS
<222> (387)..(776)

<400> 17
gatatccacc atg gcc ccc gcc gcc tgg ctg agg agc gcc gcc gcc agg 49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac 722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg 770
Gly Ser Phe Ser Asp Glu Met Asn Thr Ile Leu Asp Asn Leu Ala Ala
    150                 155                 160

cgc tga tatc 780
Arg
165

<210> 18
<211> 720
<212> DNA
<213> Artificial

<220>
<223> Description of sequence: DNA sequence of the construct according
to Fig. 11

<400> 18
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

acggcagctt cagcgacgag atgaacacca tcctggacaa cctggccgcg cgctgatatc 720

<210> 19
<211> 716

<212> DNA
<213> Artificial

<220>
<223> Description of sequence: DNA sequence and sequence of the

translated peptide according to Fig. 1m

<220>
<221> CDS
<222> (11)..(118)

<220>
<221> CDS
<222> (387)..(710)

<400> 19
gatatccacc atg gcc ccc gcc gcc tgg ctg agg agc gcc gcc gcc agg 49
Met Ala Pro Ala Ala Trp Leu Arg Ser Ala Ala Ala Arg
1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc tga gcgcgc 716
Pro Glu Glu Val Ala Ile Val Glu Glu Leu Gly
        135                 140

<210> 20
<211> 716
<212> DNA
<213> Artificial

<220>
<223> Description of sequence: DNA sequence of the construct

according to Fig. 1m

<400> 20
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

gccgcaggga cttccctgag gaggtggcca tcgtggagga gctgggctga gcgcgc 716

<210> 21
<211> 31
<212> PRT
<213> Artificial

<220>
<223> Description of sequence: artificial

<400> 21

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 22
<211> 10
<212> PRT
<213> Artificial

<220>
<223> Description of sequence: artificial

<400> 22

Arg Arg Asp Phe Pro Glu Glu Val Ala Ile
1               5                   10

<210> 23
<211> 14
<212> PRT
<213> Artificial

<220>
<223> Description of sequence: artificial

<400>  23

Arg Arg Asp Phe Pro Glu Glu Val Ala Ile Val Glu Glu Leu
1               5                   10


<210>  24
<211>  14
<212>  PRT
<213>  Artificial

<220>
<223>  Description of sequence: artificial

<400>  24

Arg Arg Asp Phe Pro Glu Glu Val Ala Ile Ala Glu Glu Leu
1               5                   10


<210>  25
<211>  31
<212>  PRT
<213>  Artificial

<220>
<223>  Description of sequence: sequence according to formula (I)

<220>
<221>  misc_feature
<222>  (31)..(31)
<223>  Xaa = NH2, when sequence is GLP-1(7-36) amide, or Xaa = Gly-OH,
       when sequence is GLP-1(7-37) amide,

<400>  25

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Xaa
            20                  25                  30

<210>  26
<211>  584
<212>  DNA
<213>  Artificial

<220>
<223>  Description of sequence: GLP-1 fusion peptide corresponding to
       Figure 1 e #217


<220>
<221>  CDS
<222>  (31)..(561)

<400>  26
aattcagata attcgatagc cccgggcacc atg gct ccc gct gca tgg ctg aga       54
                                 Met Ala Pro Ala Ala Trp Leu Arg
                                 1               5

tct gcg gcc gcg cgc gcc ctc ctg ccc ccg atg ctg ctg ctg ctg ctc       102
Ser Ala Ala Ala Arg Ala Leu Leu Pro Pro Met Leu Leu Leu Leu Leu
    10                  15                  20

cag ccg ccg ccg ctg ctg gcc cgg gct ctg ccg ccg gac gtc cac cac       150
Gln Pro Pro Pro Leu Leu Ala Arg Ala Leu Pro Pro Asp Val His His

25                  30                  35                  40

ctc cat gcc gag agg agg ggg cca cag ccc tgg cat gca gcc ctg ccc        198
Leu His Ala Glu Arg Arg Gly Pro Gln Pro Trp His Ala Ala Leu Pro
                45                  50                  55

agt agc ccg gca cct gcc cct gcc acg cag gaa gcc ccc cgg cct gcc        246
Ser Ser Pro Ala Pro Ala Pro Ala Thr Gln Glu Ala Pro Arg Pro Ala
            60                  65                  70

agc agc ctc agg cct ccc cgc tgt ggc gtg ccc gac cca tct gat ggg        294
Ser Ser Leu Arg Pro Pro Arg Cys Gly Val Pro Asp Pro Ser Asp Gly
        75                  80                  85

ctg agt gcc cgc aac cga cag aag agg cat gcc gaa ggg acc ttt acc        342
Leu Ser Ala Arg Asn Arg Gln Lys Arg His Ala Glu Gly Thr Phe Thr
    90                  95                  100

agc gat gtg agc tct tat ctg gaa ggc cag gct gcc aag gag ttc att        390
Ser Asp Val Ser Ser Tyr Leu Glu Gly Gln Ala Ala Lys Glu Phe Ile
105                 110                 115                 120

gct tgg ctg gtg aaa ggc cgg gga agg cgg gat ttc cca gag gag gtg        438
Ala Trp Leu Val Lys Gly Arg Gly Arg Arg Asp Phe Pro Glu Glu Val
                125                 130                 135

gcc atc gtg gag gag ctg ggc cgg cga cat gcc gaa ggg acc ttt acc        486
Ala Ile Val Glu Glu Leu Gly Arg Arg His Ala Glu Gly Thr Phe Thr
            140                 145                 150

agc gat gtg agc tct tat ctg gaa ggc cag gct gcc aag gag ttc att        534
Ser Asp Val Ser Ser Tyr Leu Glu Gly Gln Ala Ala Lys Glu Phe Ile
        155                 160                 165

gct tgg ctg gtg aaa ggc cgg gga tga attgccaagg gcgaattatc agg        584
Ala Trp Leu Val Lys Gly Arg Gly
    170                 175

## Description

The present application refers to the use of cells, e.g. mesenchymal stem cells or mesenchymal stromal cells, or any further suitable cell, encoding and secreting GLP-1, a fragment or variant thereof or a fusion peptide comprising GLP-1 or a fragment or variant thereof, for the treatment of acute myocardial infarction (AMI or MI), wherein the cells, encoding and secreting GLP-1, a fragment or variant thereof or a fusion peptide comprising GLP-1 or a fragment or variant thereof, are encapsulated in a (spherical) microcapsule to prevent a response of the immune system of the patient to be treated. The present application also refers to the use of these (spherical) microcapsule(s) or of a pharmaceutical composition containing these cells or (spherical) microcapsule(s) for the treatment of acute myocardial infarction (AMI or MI).

Acute myocardial infarction (AMI or MI), more commonly known as a heart attack, is a common medical condition that occurs when the blood supply to a part of the heart is interrupted, most typically due to rupture of a vulnerable plaque. The resulting ischemia or oxygen shortage causes damage and potential death of heart tissue. AMI is a medical emergency, and the leading cause of death for both men and women all over the world (see e.g. The World Health Report 2004 - Changing History (PDF), World Health Organization, 120-4, ISBN 92-4-156265-X). Important risk factors are a previous history of vascular disease such as atherosclerotic coronary heart disease and/or angina, a previous heart attack or stroke, any previous episodes of abnormal heart rhythms or syncope, older age-especially men over 40 and women over 50, smoking, excessive alcohol consumption, the abuse of certain drugs, high triglyceride levels, high LDL ("Low-density lipoprotein") and low HDL ("High density lipoprotein"), diabetes, high blood pressure, obesity, and chronically high levels of stress in certain persons.

Myocardial infarction is a common presentation of ischemic heart disease. The WHO estimated that in 2002, 12.6 percent of deaths worldwide are due to ischemic heart disease (see, e.g., The World Health Report 2004, supra). Ischemic heart disease is the leading cause of death in developed countries, but third to AIDS and lower respiratory infections in developing countries (see e.g. Cause of Death - UC Atlas of Global Inequality, Center for Global, International and Regional Studies (CGIRS) at the University of California Santa Cruz, retrieved on December 7, 2006).

In the United States, diseases of the heart are the leading cause of death, causing even a higher mortality than cancer (malignant neoplasms) (see e.g. "Deaths and percentage of total death for the 10 leading causes of death: United States": 2002-2003, National Center of Health Statistics, retrieved on April 17, 2007). Coronary heart disease is responsible for 1 in 5 deaths in the U.S. Some 7,200,000 men and 6,000,000 women are living with some form of coronary heart disease. 1,200,000 people suffer a (new or recurrent) coronary attack every year, and about 40% of them die as a result of the attack (see e.g. Heart Attack and Angina Statistics, American Heart Association (2003), retrieved on December 7, 2006) This means that about every 65 seconds, an American dies of a coronary event. Similar statistics are to be expected in Europe.

Immediate treatment for suspected acute myocardial infarction typically includes oxygen, aspirin, glyceryl trinitrate and pain relief, usually morphine sulfate. The patient normally receives a number of diagnostic tests, such as an electrocardiogram (ECG, EKG), a chest X-ray and blood tests to detect elevated creatine kinase or troponin levels (which are chemical markers released by damaged tissues, especially the myocardium). Further treatment may include either medications to break down blood clots that block the blood flow to the heart, or mechanically restoring the flow by dilatation or bypass surgery of the blocked coronary artery. Coronary care unit admission allows rapid and safe treatment of complications such as abnormal heart rhythms.

AMI is a type of acute coronary syndrome, which is most frequently (but not always) a manifestation of coronary artery disease. The most common triggering event is the disruption of an atherosclerotic plaque in an epicardial coronary artery, which leads to a clotting cascade, sometimes resulting in total occlusion of the artery. Atherosclerosis is the gradual build-up of cholesterol and fibrous tissue in plaques in the wall of arteries (in this case, the coronary arteries), typically over decades. Blood stream column irregularities visible on angiographies reflect artery lumen narrowing as a result of decades of advancing atherosclerosis. Plaques can become unstable, rupture, and additionally promote a thrombus (blood clot) that occludes the artery, typically within minutes. When a severe plaque rupture occurs in the coronary vasculature, it may lead to myocardial infarction and usually to a necrosis of downstream myocardium.

If impaired blood flow to the heart lasts long enough, it triggers a process called the ischemic cascade, in which the heart cells typically die (particularly due to necrosis) and do not grow back. A collagen scar forms in its place. Recent studies indicate that apoptosis may also play a role in the process of tissue damage subsequent to myocardial infarction (see Krijnen PA, Nijmeijer R, Meijer CJ, Visser CA, Hack CE, Niessen HW. (2002). "Apoptosis in myocardial ischaemia and infarction". J Clin Pathol 55 (11): 801-11. PMID 12401816). As a result, the patient's heart can be permanently damaged. The scar tissue formed in the ischemic cascade also puts the patient at risk for potentially life threatening arrhythmias. A therapy, which reduces the extent of this ensuing tissue damage, could have significant benefits in improving the function of the heart post AMI and improved long term patient outcomes.

At present, there is a deal of great research in the use of stem cell therapy for the regeneration of the myocardium post MI. Patients who receive stem cell treatment by left ventricular intramyocardial implantation of stem cells derived from their own bone marrow after a myocardial infarction show improvements in left ventricular ejection fraction and end-diastolic volume, which was not seen with placebo. The larger the initial infarct size, the greater the effect of the infusion. However, a suitable therapy has not yet been established. Clinical trials of progenitor cell infusion as a treatment approach to ST elevation MI are proceeding (see e.g. Schachinger V, Erbs S, Elsasser A, Haberbosch W, Hambrecht R, Holschermann H, Yu J, Corti R, Mathey DG, Hamm CW, Suselbeck T, Assmus B, Tonn T, Dimmeler S, Zeiher AM; REPAIR-AMI Investigators (2006), "Intracoronary bone marrow-derived progenitor cells in acute myocardial infarction", N Engl J Med 355 (12): 1210-21, PMID 16990384.).

Additional to the above stem cell approach, there are currently three biomaterial and tissue engineering approaches for the treatment of AMI or MI. However, these approaches are in an even earlier stage of medical research, so many questions and issues need to be addressed prior to establishing a suitable therapy based on these approaches.

The first interesting approach in this field involves polymeric left ventricular restraints in the prevention of heart failure. A second approach utilizes *in vitro* engineered cardiac tissue, which is subsequently implanted *in vivo.*

Another approach entails injecting cells and/or a scaffold into the myocardium to create *in situ* engineered cardiac tissue (see e.g. Christman KL, Lee RJ. "Biomaterials for the Treatment of Myocardial Infarction". J Am Coll Cardiol 2006; 48(5): 907-13. PMJD 16949479). A variant of this approach is the injection of cells to produce factors that will help preserve myocardium post AMI or MI, for instance, by preventing the cardiomyocytes from undergoing apoptosis. In this final approach, Glucagon Like Peptide-1 (GLP-1) has been shown to provide some very promising effects, which may be utilized in the treatment of AMI or MI. A number of recent studies, carried out in this approach, have shown that Glucagon Like Peptide-1 (GLP-1), one of the most potent incretin hormones, has potential beneficial actions on the ischaemic and failing heart (see Bose AK, Mocanu MM, Carr RD, Yellon DM, Myocardial ischaemia-reperfusion injury is attenuated by intact glucagon like peptide-1 (GLP-1) in the in vitro rat heart and may involve the p7Os6K pathway. Cardiovasc Drugs Ther. 2007 Aug;21 (4):253-6; and Nikolaidis LA, Mankad S, Sokos GG, Miske G, Shah A, Elahi D, Shannon RP. Effects of glucagon-like peptide- 1 in patients with acute myocardial infarction and left ventricular dysfunction after successful reperfusion, Circulation, 2004 Mar 2, 109(8) :962-5).

GLP-1 is located on the glucagon gene, a well studied gene (see e.g. White, J.W. et al., 1986 Nucleic Acid Res. 14(12) 4719-4730). The preproglucagon molecule as a high molecular weight precursor molecule is synthesized in pancreatic alpha cells and in the jejunum and colon L cells. Preproglucagon is a 180 amino acid long prohormone and its sequence contains, in addition to glucagon, two sequences of related structure: glucagon-like peptide-1 (GLP-1) and glucagon-like peptide-2 (GLP-2). In the preproglucagon molecule, between GLP-1 and GLP-2 is a 17 amino acid peptide sequence (or rather a 15 amino acid sequence plus the C-terminal RR cleavage site), intervening peptide 2 (IP2). The IP2 sequence (located between GLP-1 and GLP-2 in the precursor molecule) is normally cleaved proteolytically after aa 37 of GLP-1. The preproglucagon module is therefore cleaved into various peptides, depending on the cell, and the environment, including GLP-1 (1-37), a 37 amino acid peptide in its unprocessed form. Generally, this processing occurs in the pancreas and the intestine. The GLP-1 (1-37) sequence can be further proteolytically processed into active GLP-1 (7-37), the 31 amino acid processed form, or GLP-1 (7-36) amide. Accordingly, the designation GLP-1(7-37) means that the fragment in question comprises the amino acid residues from (and including) number 7 to (and including) number 37 when counted from the N-terminal end of the parent peptide, GLP-1. The amino acid sequence of GLP-1 (7-36)amide and of GLP-1 (7-37) is given in formula I (SEQ ID NO: 25):
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-X (I)
which shows GLP-1 (7-36)amide when X is NH₂ and GLP-1 (7-37) when X is Gly-OH.

GLP-1 is a gut hormone and is the most potent endogenous insulinotropic agent with actions that include stimulating adenylate cyclase and protein kinase activity in the beta-cell. Physiologically, together with gastric inhibitory polypeptide from the upper gut, it functions as an incretin hormone lowering the blood glucose level. Accordingly, GLP-1, secreted in response to food intake, has multiple effects on, e.g., the stomach, liver, pancreas and brain that work in concert to regulate blood sugar. Consequently, Glucagon-like peptide GLP-1 (7-36)amide, and its non-amidated analogue GLP-1 (7-37) have attracted considerable interest because of their potent actions on carbohydrate metabolism and its potential applicability to the treatment of diabetes, including type 2 diabetes.

Additionally, GLP-1 has shown promising effects on the treatment of acute myocardial infarction (AMI) or myocardial infarction (MI). As mentioned above, GLP-1 is a naturally occurring incretin with both insulinotropic and insulinomimetic properties that stimulate glucose uptake without the requirements for concomitant glucose infusion. Nikolaidis *et al*. (see Nikolaidis *et al*., 2004, supra) found that when added to standard therapy, GLP-1 infusion improved regional and global LV function in patients with AMI and severe systolic dysfunction after successful primary angioplasty.

In an early study, the safety and efficacy of the administration of GLP-1 was tested during a 72 hour infusion of GLP-1 (1.5 µg/kg per minute) added to a background therapy in 10 patients with AMI and LV fraction (EF) < 40% after successful primary angioplasty compared to 11 control patients was determined (see Nikolaidis et al., Circulation 2004, 109; 962-965). In the context of these experiments, the benefits of GLP-1 were regarded to be independent of AMI location or history of diabetes. Accordingly, GLP-1 infusion, when added to standard therapy, seems to improve regional and global LV function in patients with AMI and severe systolic dysfunction after successful primary angioplasty (see Nikolaidis *et al*., Circulation 2004, supra). Nikolaidis *et al*. also hypothesized that GLP-1 facilitates recovery from myocardial stunning after an ischemic event. In an experimental dog model, GLP-1 was administered upon a 10 minute occlusion of the left ventricular coronary artery, followed by a 24 hour reperfusion. In these experiments, administration of GLP-1 caused an insulinotropic effect, but no hyperglycaemia (see Nikolaidis et al., Journal of Pharmacology and Experimental Therapeutics, January 2005, 312 (1), pp. 303-8).

According to another approach, the role of GLP-1 *in vivo*, particularly GLP-1 (7-36), was investigated by using DPP-IV inhibitors such as valine pyrrolidine (VP) as a means of preventing its degradation (see Bose et al., Diabetes, Vol. 54, January 2005, and Bose et al., Cardiovascular Drugs and Therapy, August 2007, 21 (4), pp. 253-6).
Native GLP-1, particularly GLP-1 (7-36), suffers from a short half life *in vivo.* It is rapidly degraded in plasma within minutes by DPP-IV between residues 8 and 9, resulting in an inactive NH₂-terminally truncated metobolite GLP-1 (9-36). In this context, GLP-1 and valine pyrrolidine (VP) were seen to protect the myocardium from ischemia reperfusion injury in the *in vivo* heart model, demonstrating a significant reduction in infarction compared to VP or saline groups. In the corresponding *in vitro* study, those hearts treated with GLP-1 and VP again demonstrated a significantly reduced infarct size compared with control and VP groups (see also Bose *et al*., 2005 and 2007, supra).

Further data suggested that GLP-1 can exert a direct protective effect to the heart *via* inhibition of apoptosis either directly in target cells expressing GLP-1 receptor or possibly *via* activation of survival factors such as prosurvival signalling pathways (see also Bose et al., Diabetes, Vol. 54, January 2005). In this context, the main target of GLP-1 is the islet, where the hormone stimulates the insulin secretion, promotes beta cell proliferation and neogenesis, and inhibits glucagon secretion. However, GLP-1 receptors are also expressed outside the islets, confirming the likelihood that GLP-1 also plays a role in other organs (see Ahren B. et al, Hormones et metabolisme, Dec 2004, 36 (11-12), pp. 842-5).

GLP-1 has been shown to increase left ventricular (LV) function, myocardial glucose uptake, and GLUT-1 and GLUT-4 translocation during reperfusion to an extent similar to that with insulin. In contrast to insulin, however, the insulinotropic actions of GLP-1 are advantageously dependent on the ambient glucose concentrations, mitigating the risks of hypoglycaemia. GLP-1 has also been shown to have direct effects on the normal heart, reducing contractility but increasing myocardial glucose uptake through a non-Akt-1 dependent mechanism, distinct from the actions of insulin (see Tingcun Zhao et al., The Journal of Pharmacology and Experimental Therapeutics, Vol. 317(3), pp. 1106-1113; No. 3, 2006)

GLP-1 has furthermore been shown to improve left ventricular (LV) function in patients with acute myocardial infarction and left ventricular dysfunction. GLP-1 additionally promotes the activity of phospho-inositide 3-kinase (PI3K) in beta-cells. This kinase has been clearly associated with myocardial protection in the setting of ischemic/reperfusion injury as well as myocardial preconditioning (see Bose et al., Diabetes, Vol. 54, January 2005; and Tingcun Zhao et al., The Journal of Pharmacology and Experimental Therapeutics, Vol. 317, pp. 1106-1113; No. 3, 2006).

In a different approach, Huisamen *et al*. (see Huisamen et al., Cardiovascular Journal of Africa (South Africa), March-April 2008, 19(2), p. 77-83), showed that GLP-1 had an infarct-sparing effect when supported by the presence of the DPP-IV inhibitor valine pyrrolide. GLP-1 could not directly activate Akt (also called protein kinase B) or the extracellular regulated kinases Erk1/2 in hearts or cardiocytes under normoxic conditions, but phosphorylation of the AMP-activated kinase (AMPK) on Thr(172) was enhanced. In addition, the glycolytic enzyme phosphofructokinase-2 was activated dose dependently. During reperfusion after ischaemia, modulation of the phosphorylation of PKB/Akt as well as AMPK was evident. GLP-1 thus directly appears to protect the heart against low-flow ischaemia by enhancing glycolysis.

However, the use of native GLP-1 causes some problems *in vivo* due to its rapid degradation *in vivo* by DPP-IV as already described above. Additionally, GLP-1 undergoes renal excretion. These factors raise the issue, as to which peptide, GLP-1 (7-36) or the NH₂-terminally truncated metabolite GLP-1 (9-36), is the active moiety *in vivo* and as to whether physiological effects are exerted in therapeutic applications by the native GLP-1 or its fragments.

Due to its rapid degradation *in vivo*, GLP-1 was regarded as a suitable tool only for a short-term metabolic control, such as intensive care units potentially useful in patients with acute myocardial infarction, coronary surgery, cerebrovascular events, or septicaemia. For more long-term metabolic control, incretin mimetics such as agonists of the GLP-1 receptor were suggested (see e.g. Nauck M.A., Nov-Hormones et metabolisme, Dec 2004, 36 (11-12), pp. 852-8).

However, irrespective of its fast degradation *in vivo*, GLP-1 appears to provide a good basis as a potential drug in the treatment of coronary diseases, particularly for the treatment of AMI or MI.

Various attempts have therefore been made to synthesize stabilized (against DPP-IV) analogues of naturally occurring GLP-1 (GLP-1(7-37)). In particular, the 8^{th} residue, which *in vivo* is Ala, was replaced by another residue, for instance, Gly, Ser or Thr (Burcelin, R. et al. (1999) Metabolism 48, 252-258). The Gly8 or G8 analogue has been extensively tested, both as synthesized molecule, and produced by cell lines genetically engineered to secrete the mutant polypeptide (Burcelin, R., et al. (1999), Annals of the New York Academy of Sciences 875: 277-285). Various other modifications have been introduced into GLP-1 (7-37) to enhance its *in vivo* stability without compromising its biological activity. However, all of these approaches did not achieve any therapeutic significance due to considerable problems involved.

Nevertheless, none of these approaches allows a long-term provision of GLP-1 *in vivo*. This is due to proteolytical degradation as discussed above, to metabolism of GLP-1 and normal protein degradation typically occurring in the body. Thus, at present, the patient in need of GLP-1 has to receive one or even multiple doses of GLP-1 or its analogs or variants during a long period of time, i.e. as long as he suffers from the disease to be treated, or even worse, for a whole life span. In this period of time, administration typically occurs within short intervals due to the short half life of GLP-1 *in vivo*. Furthermore, doses of GLP-1 have to be administered either by a medical doctor or by the patient himself. This represents a major challenge and burden for patient and environment. In order to circumvent this problem, GLP-1 may be administered by providing cells to a patient containing a nucleic acid encoding and expressing GLP-1. Implantation of such cells would ensure a longer provision of GLP-1 *in vivo* and, due to secretion of GLP-1 from the grafted cells, provide GLP-1 directly at the site of interest.

WO 99/53064 discloses a strategy for creating a multimeric GLP-1 expression cassette which can be incorporated into a variety of cell types which are publicly available immortalised cell lines and dividing primary cell cultures. Examples include EGF-responsive neurospheres, bFGF-responsive neural progenitor stem cells from the CNS of mammals, while the worked example uses baby hamster kidney (BHK) cells. The implanted transfected cells were said to have been used successfully to treat diabetic mice, allowing glucose control equivalent substantially to non-diabetic controls. However, this kind of implantation technique does not comply with the requirements for a routine treatment for e.g. diabetes patients or have been used successfully in AMI or MI patients.

Moreover, it is known in the art that the immune system typically recognizes foreign cells and triggers an immune response in order to protect the organism from such external material. Implantation of cells capable of expressing GLP-1 or any of its variants or derivatives may thus lead to an immune response in the organism. Such a defence response may cause considerable and undesirable side effects during treatment and may lead to severe complications or even death of the treated organism.

One strategy to circumvent this problem may be the use of autologous cells, i.e. cells which are derived from the patient itself, which are genetically altered to transiently express GLP-1, particularly GLP-1(7-36). Such a procedure, however, is typically limited to the cells of the specific patient and requires a long-term preparation in a laboratory prior to use. AMI or MI, however, requires a therapy within a few minutes or hours subsequent to the myocardial infarction, i.e. a short term provision of an efficient medicament.

In summary, at present there is no efficient AMI or MI therapy available in the art, which allows providing an efficient therapy directly subsequent to occurrence of the myocardial infarction in a patient to be treated. In other words, the prior art fails to provide a therapy which reflects the entire spectrum of beneficial effects known for GLP-1, e.g. its activity to powerfully reduce the damages caused by ischemia or oxygen shortage and potential death of heart tissue without the need of repeated administration of GLP-1 peptide(s) and/or the risk of an undesired immune response against e.g. implanted GLP-1 expressing allogenic cells.

Therefore, it is an objective of the present invention to provide an efficient therapy for AMI or MI using GLP-1 based peptide molecules or analogues thereof, which are biologically active *in vivo* over a long-lasting time period without the need of repeated administration of GLP-1 peptide(s) and/or the risk of evoking an undesired immune response.

The object underlying the present invention is solved by the attached claims, particularly by the use of cells, e.g. mesenchymal stem cells or mesenchymal stromal cells, or any further cell, that may be used in the context of the present invention, encoding and secreting GLP-1, a fragment or variant thereof or a fusion peptide comprising GLP-1 or a fragment or variant thereof, for the treatment of AMI or MI, wherein the cells, encoding and secreting GLP-1, a fragment or variant thereof or a fusion peptide comprising GLP-1 or a fragment or variant thereof, are encapsulated in a (spherical) microcapsule to prevent a response of the immune system of the patient to be treated.

### (Spherical) Microcapsule

The (spherical) microcapsule preferably comprises a (spherical) core (i.e. the core may be spherical or not) and at least one surface coating:
- wherein the (spherical) core comprises or consists of (a mixture of) cross-linked polymers and cells, e.g. mesenchymal stem cells or mesenchymal stromal cells, or any further cell, that may be used in the context of the present invention, encoding and secreting GLP-1, a fragment or variant thereof or a fusion peptide comprising GLP-1 or a fragment or variant thereof, as defined herein; and
- wherein the at least one surface coating comprises or consists of cross-linked polymers.

In the context of the present invention, the total diameter of the (spherical) microcapsule as used herein may vary considerably depending on the specific treatment, wherein the diameter of the core of the (spherical) microcapsule as well as the diameter of the at least one surface coating(s), of course, influence the total diameter of the (spherical) microcapsule and depend at least in part on each other. For the treatment of AMI or MI, the inventors of the present application have surprisingly found, that a total diameter (particle size) of the (spherical) microcapsule of about 120 µm to about 300 µm, more preferably a total diameter of about 150 µm to about 250 µm, even more preferably a total diameter of about 165 µm to about 225 µm, and most preferably a total diameter of about 180 µm to about 200 µm, e.g. about 180, 185, 190 or 200 µm, is particularly advantageous for the treatment of AMI or MI. (Spherical) microcapsules, comprising such a total diameter, typically retain in the myocardium at the site of injection and do not migrate into the surrounding tissue. This allows providing a continuous expression of GLP-1 and/or its variants or analogs at the site of injection during treatment for a sufficient time to provide the entire spectrum of beneficial effects known for GLP-1, e.g. its activity to powerfully reduce the damages caused by ischemia or oxygen shortage and potential death of heart tissue.

With respect to the definition "spherical", it is of critical importance to embed the encapsulated cells, e.g. mesenchymal stem cells or mesenchymal stromal cells, or any further cell, which may be used in the context of the present invention, e.g. autologous cells, entirely in the polymer matrix when preparing (spherical) microcapsules for the use according to the present invention. In this context, the term "spherical" is understood in its broadest meaning. A spherical particle is preferably understood to have a sphere-like shape, whereby the shape may be symmetrical or asymmetrical, e.g. a (spherical) microcapsule or core may have ellipsoidal shape. In a less preferred embodiment the microcapsule or core used according to the present invention may not be spherical within the above meaning, but may have an arbitrary shape with e.g. protruding or invading segments on the surface of the microcapsule. Where ever in the present disclosure "spherical" microcapsules or cores are mentioned, "non-spherical" microcapsules or cores may be provided, prepared or used as well.

### (Spherical) Microcapsule - (spherical) core

According to one embodiment, the (spherical) core of the (spherical) microcapsule, as used according to the present invention, typically comprises or consists of cross-linked polymers and cells, e.g. mesenchymal stem cells or mesenchymal stromal cells, or any further cell, that may be used in the context of the present invention, encoding and secreting GLP-1, a fragment or variant thereof or a fusion peptide comprising GLP-1 or a fragment or variant thereof.

In the context of the present invention, the cross-linked polymers of the (spherical) core of the (spherical) microcapsule, form a scaffold structure embedding the cells, e.g. mesenchymal stem cells or mesenchymal stromal cells, or any further cell, that may be used in the context of the present invention, in its cavities. These cells may be embedded in the scaffold structure individually or, typically, as aggregates, e.g. as (a pool of) aggregated cells of about 10 to about 10,000 cells, e.g. about 10 to about 500, about 10 to about 1000 or about 10 to about 10000 cells. Preferably, the (spherical) core comprises a homogenous distribution of the cross-linked polymers and of embedded cells as defined herein. Preferably, the core, including the scaffold structure and the embedded cells as defined herein, is prepared according to a method as disclosed below.

The embedded cells, e.g. mesenchymal stem cells or mesenchymal stromal cells, or any further cell as defined herein, that may be used for the (spherical) microcapsule in the context of the present invention, may be present in the core in a concentration of about 1 x 10⁵, about 1 x 10⁶ or about 1 x 10⁷ cells/ml cross-linked scaffold polymer to about 5 x 10⁸ cells/ml cross-linked scaffold polymer, more preferably in a concentration of about 1 x 10⁵, about 1 x 10⁶, or about 1 x 10⁷ cells/ml cross-linked scaffold polymer to 1 x 10⁸ cells/ml cross-linked scaffold polymer and most preferably in a concentration of about 1 x 10⁵, about 1 x 10⁶, or about 2 x 10⁷ cells/ml cross-linked scaffold polymer to 6 x 10⁷ cells/ml cross-linked scaffold polymer.

In the context of the present invention, the core of the (spherical) microcapsule used according to the present invention typically comprises a diameter (particle size) of not more than the diameter of the total diameter of the (spherical) microcapsule, i.e., the core of the (spherical) microcapsule used according to the present invention typically comprises a diameter of not more than about 100 µm to about 200 µm, more typically a diameter of not more than about 115 µm to about 185 µm, even more typically a diameter of not more than about 130 µm to about 170 µm, and most typically a diameter of not more than about 145 µm to about 155 µm, e.g. not more than about 120, 125, 130, 135, 140, 145, 150 or 155 µm. Preferably, the diameter of the core of the (spherical) microcapsule, as used according to the present invention, has a diameter, which is preferably about 1 to about 80 µm less than the total diameter of the (spherical) microcapsule as defined above, more preferably about 15 to about 70 µm less than the total diameter of the (spherical) microcapsule as defined above, and most preferably about 30 to about 60 µm less than the total diameter of the (spherical) microcapsule as defined above. In other words, the diameter of the core of the (spherical) microcapsule, as used according to the present invention, may have a size of about 50 µm, of about 60 µm, of about 70 µm, of about 80 µm, of about 90 µm, of about 100 µm, of about 110 µm, of about 120 µm, of about 125 µm, of about 130 µm, of about 135 µm, of about 140 µm, of about 145 µm, of about 150 µm, of about 155 µm, of about 160 µm, of about 165 µm, of about 1 70 µm, of about 1 75 µm, of about 180 µm, of about 185 µm, of about 190 µm, of about 195 µm, of about 200 µm, of about 205 µm, of about 210 µm, of about 215 µm, or even of about 220 µm, or may comprise any range selected from two of any of the above mentioned specific values.

### (Spherical) Microcapsule - surface coating

Cells, e.g. mesenchymal stem cells or mesenchymal stromal cells, or any further cell, that may be used in the context of the present invention for the (spherical) core, being located at the core periphery or cells protruding out of the scaffold structure may evoke immunological problems, since the immune system will recognize these microcapsules as foreign components and, thus, these microcapsules will be attacked by the immune system. Although this effect may be avoided by lowering the cell concentration in the initial solution, the present invention allows improving the efficacy of the microcapsule by increasing the core's cell number. The higher the concentration of encapsulated cells, the smaller the total volume of the resultant microcapsules to be transplanted, i.e. the more efficient the microcapsules may work at the site of injection. In order to avoid immunological problems when using high concentrations of cells in the core the invention provides at least one surface coating applied on the (spherical) core of the (spherical) microcapsule as used according to the present invention. This surface coating does not allow an immune response to occur, even if cells are located very closely to the core periphery, since these cells are not accessible for the host's immune system due to the surface coating acting as barrier. This surface coating is typically composed of a cross-linked polymer as defined above without containing any cells. According to a particular embodiment the afore defined (spherical) core is coated with at least one or more than one surface coatings, e.g. 1, 2, 3, 4, 5, 5-10 or more surface coating(s), more preferably 1, 2 or 3 surface coating(s). Typically, each surface coating comprises a uniform thickness around the core. The thickness of the surface coating(s) of the (spherical) microcapsule, as used according to the present invention, may be varied almost arbitrarily and is typically in a range of about 1 to about 80 µm, more preferably in a range of about 15 to about 70 µm, and most preferably in a range of about 20 to about 40 µm, e.g. about 30 µm.

### (Spherical) Microcapsule - Polymers of the (spherical) core and the surface coating

Any (cross-linkable) polymer known in the art as being suitable for encapsulation may be used for the formation of the (spherical) core and, independent from each other, the at least one surface coating(s) of the (spherical) microcapsule, as defined according to the present invention. Preferably, such polymers are used, which, on the one hand, are permeable in their cross-linked state for supply of oxygen and nutrients, and, on the other hand, allow diffusion of the peptide encoded and secreted by the core cells from the microcapsule into the patient's tissue or body fluids. Furthermore, the cross-linked polymers prevent intrusion of components of the body's immune system through the matrix. By way of example, polymers may be used such as synthetic, semi-synthetic and natural water-soluble (bio)polymers, e.g. from natural polymers such as selected proteins or polymers based on proteins (e.g. collagens, albumins etc.), polyamino acids (e.g. poly-L-lysine, poly-L-glutamic acid, etc.), polysaccharides and their derivatives (e.g. carboxyl methyl cellulose, cellulose sulfate, agarose, alginates including alginates of brown algae (e.g. of species Laminarales, Ectocarpales, Fucales), carrageenans, hyaluronic acid, heparin and related glycosamino sulfates, dextranes and its derivatives, chitosan and its derivatives). Synthetic polymers may also be used such as e.g. aliphatic polyesters (e.g. polylactic acid, polyglycolic acid, polyhydroxybuyrates, etc.), polyamides, polyanhydrides, polyorthoesters, polyphosphazenes, thermoplastic polyurethanes, polyvinyl alcohols, polyhydroxyethylmethacrylates, polymethylmethacrylates and polytetrafluoroethylenes, etc. Furthermore, block polymers may be used herein accordingly, i.e. polymers derived by combination of two or more of the aforementioned polymers. Such block polymers may be selected by a skilled person depending on the desired properties, e.g. pore size, cross-linking status, toxicity, handling, biocompatibility, etc. Any of the above polymers is defined as a "chemically different polymer" in the context of the present invention, i.e. each of these polymers typically does not exhibit an identical molar mass and structure with any other of the above polymers. In contrast, "chemically identical polymers" means, that the polymers exhibit an identical molar mass and structure. Finally, mixtures of the above polymers are also encompassed herein, wherein the amounts of polymers contained in such a mixture may be selected by a skilled person depending on the desired properties, e.g. as outlined above. In this respect, mixtures of polymers may be regarded as chemically identical to another polymer mixture ("chemically identical polymers"), if the overall molar mass of the resultant polymer mixture and the corresponding molar percentage of the single polymers of the mixture are identical to the other polymer mixture.

Preferably, alginate is used according to present invention as a polymer for the formation of the (spherical) core and/or of the surface coating(s) due to their biocompatibility and their cross-linking properties. From a chemical point of view, alginates are anionic polysaccharides derived from homopolymeric groups of β-D-mannuronic acid and α-L-guluronic acid, separated by heteropolymeric regions of both acids. Alginates are water soluble and form high viscosity solutions in the presence of monovalent cations such as sodium or potassium. A cross-linked water insoluble hydrogel is formed upon interaction of single alginate chains with bi-, tri- or multivalent cations (such as calcium, barium or polylysin). Preferably, purified alginates (e.g. according to DE 198 36 960, the disclosure of which is incorporated herein by reference) are used for encapsulation, more preferably potassium or sodium alginates in physiological saline solution. Such alginates typically exhibit an average molar mass of about 20 kDa to about 10,000 kDa, more preferably a molar mass of about 100 kDa to about 1,200 kDa. Alginates used for the formation of the core and/or of the surface coating(s) of the microcapsule preferably, as used according to the present invention, may be provided as a solution, more preferably as an aqueous solution, e.g. the viscosity of a 0.2% (w/v) aqueous alginate solution of the alginate to be used may be in the range of about 2 to about 50 mPa s, more preferably in the range of about 3 to about 10 mPa s.

If alginates are used according to the present invention, those, which are rich in α-L-guluronic acid are preferred. Alginates suitable for preparing (spherical) microcapsules as used according to the present invention are obtainable by extraction from certain algae species including, without being limited thereto, brown algae, e.g. Laminarales, Ectocarpales, Fucales, etc., and other species of algae containing alginates. Alginates may be isolated from fresh algae material or dried material according to any method for preparing alginates known to a skilled person.

The above defined cross-linked polymers used for preparation of the (spherical) core and at least one surface coating of the (spherical) microcapsule, as used according to the present invention, may be identical or different. According to a first embodiment the cross-linked polymers used for preparation of the core and at least one surface coating may comprise chemically identical polymers in identical or differing concentrations. Preferably, the polymers present in the core and at least one surface coating are prepared using a non-cross-linked polymer solution selected from any of the polymers a defined above. In the polymer solution, the non-cross-linked polymers are typically present in a concentration of about 0.1 % (w/v) to about 8 % (w/v) of the non-cross-linked polymer, more preferably in a concentration of about 0.1 % (w/v) to about 4 % (w/v), even more preferably in a concentration of about 0.5 % (w/v) to about 2.5 % (w/v) and most preferably in a concentration of about 1 % (w/v) to about 2 % (w/v). If alginates as disclosed above are used as polymers the concentration of the polymer solution for preparing the (spherical) core and at least one surface coating of the microcapsule, as used according to the present invention, furthermore may be selected from 0.1 to 4% (w/v), preferably from 0.4 to 2% (w/v). Different alginate concentrations may be used for preparing the (spherical) core and at least one surface coating of the microcapsules, as used according to the present invention. Preferably, the non-cross-linked polymers used for preparation of the core and/or at least one surface coating comprise chemically identical polymers, more preferably in identical concentrations, e.g. in concentrations as defined above with polymers as defined above. In this context the term "% (w/v)" refers to the concentration of non-cross-linked polymers and is typically determined on the basis of a certain amount of a polymer in its dry form *versus* the total volume of the polymer solution, e.g. after solubilising the non-cross-linked polymer in a suitable solvent (before the cross-linkage). However, the above concentrations may also be indicated in the corresponding "% v/v" concentrations, if applicable, e.g. if polymers are used, which are present in a fluid aggregate state at standard conditions (room temperature, normal pressure, etc.).

According to a second embodiment the cross-linked polymers used for preparation of the core and/or the at least one surface coating may comprise chemically different polymers in identical or differing concentrations. Thereby, concentrations may be chosen as defined above. Furthermore, polymers may be chosen from polymers as defined above, including e.g. natural polymers, synthetic polymers, and combination of polymers, i.e. block polymers.

Additionally, the polymers in each surface coating may be identical or different, i.e. the cross-linked polymers of each surface coating may comprise chemically identical or different polymers in identical or differing concentrations, e.g. the (spherical) microcapsule, as used according to the present invention, may comprise at least one surface coating, as defined above, consisting of any polymer as defined above, and an additional external surface coating consisting of polycations, e.g. polyamino acids as defined above, e.g. poly-L-lysine, poly-L-glutamic acid, etc. Difference in nature of the polymers used may further be due to different molecular weight of the polymers used and/or due to different cross-linkage of identical polymers, etc.

### (Spherical) microcapsule - Cells of the (spherical) core

As defined above, the core of the (spherical) microcapsule, as used according to the present invention, further comprises cells, e.g. mesenchymal stem cells or mesenchymal stromal cells, or any further cell, that may be used in the context of the present invention, encoding and secreting GLP-1, a fragment or variant thereof or a fusion peptide comprising GLP-1 or a fragment or variant thereof. These GLP-1 peptide encoding and secreting cells may be selected from any type of cells being capable to express and secrete GLP-1. Such cells are typically obtainable by stably transfecting a cell with a nucleic acid or rather a vector containing a nucleic acid coding for GLP-1, a fragment or variant thereof or a fusion peptide comprising GLP-1 or a fragment or variant thereof, as defined below.

Suitable cells may be chosen from (non-differentiated) stem cells including totipotent, pluripotent, or multipotent stem cells. Stem cells used in the present context preferably comprise embryonic stem cells or stem cells derived from the ectoderm, the mesoderm or the endoderm, or adult stem cells such as (human) mesenchymal stem cells or mesenchymal stromal cells (MSC, hMSC) (e.g. derived from human bone marrow or from fat tissue), hematopoietic stem cells, epidermal stem cells, neural stem cells and immature fibroblasts, including fibroblasts from the skin (myofibroblasts), etc. These (undifferentiated) stem cells are typically capable of symmetric stem cell division, i.e. cell division leading to identical copies. Stem cells maintain the capacity of transforming into any cell type. Moreover, stem cells are capable of dividing asymmetrically leading to a copy of the stem cell and another cell different from the stem cell copy, e.g., a differentiated cell.

Mesenchymal stromal cells may additionally produce a set of endogenous trophic factors that support the cytoprotective effect of GLP-1. Biologically active factors for this paracrine cytoprotective mechanism of the mesenchymal stromal cells may be e.g. the cytokines GRO, IL-6, IL-8, MCP-1 and the growth factors VEGF, GDNF and Neurotrophin-3. According to a particularly preferred embodiment, the cells in the core of the spherical microcapsules therefore secrete endogenous proteins or peptides as paracrine factors that are released through the capsule in therapeutic levels selected from VEGF, IL6, IL8, GDNF, NT3, and MCP1, etc.

The core of (spherical) microcapsules, as used according to the present invention, may alternatively contain cells which are chosen from (differentiated) cells, e.g., obtainable from the above stem cells or stromal cells, e.g., cells of the connective tissue family, e.g., (mature) fibroblasts, cartilage cells (chondrocytes), bone cells (osteoblasts/osteocytes, osteoclasts), fat cells (adipocytes), or smooth muscle cells, or blood cells including lymphoid progenitor cells or cells derived therefrom, e.g., NK cells, T-cells, B-cells or dendritic cells, or common myeloid progenitor cells or cells derived therefrom, e.g., dendritic cells, monocytes, macrophages, osteoclasts, neutrophils, eosinophils, basophils, platelets, megakaryocytes or erythrocytes, or macrophages, neuronal cells including astrocytes, oligodendrocytes, etc., or epithelial cells, or epidermal cells. These differentiated cells are typically capable of symmetric cell division, i.e. cell division leading to identical copies of the differentiated parent cell. Moreover, in some cases these differentiated cells may be capable of dividing asymmetrically leading to an identical copy of the parent cell and another cell different from the parent cell, i.e. a cell being further differentiated than the parent cell. Alternatively, in some cases differentiated cells as defined above may be capable of differentiating further without the need of cell division, e.g., by adding selective differentiation factors.

Furthermore, cells embedded in the core of the (spherical) microcapsule, as used according to the present invention, may either be cells taken from the patient to be treated himself (autologous cells) or may be taken from allogenic cells (e.g. taken from an established cell line cultivated *in vitro*, e.g., HEK293 cells, hTERT-MSC cells, etc.). Due to the surface coating embedding the core in the (spherical) microcapsule, as used according to the present invention, it allows the use of allogenic cells without evoking any undesired immune response by the patient to be treated.

Cells embedded in the core of the (spherical) microcapsule, as used according to the present invention, may furthermore be a combination of (differentiated and/or non-differentiated) cell types as defined above. The core of the (spherical) microcapsule, as used according to the present invention, may contain, e.g., human mesenchymal stem cells or human mesenchymal stromal cells, wherein a portion of these cells may be differentiated *in vitro* or *in vivo* into a cell type as defined above, e.g. adipocytes (suitable for transplantation into fat tissue), etc. Accordingly, various cell types (derived e.g. from a specific stem cell type) may be allocated in the core, e.g. sharing a common lineage.

In summary, cells for preparing the core of the (spherical) microcapsule, as used according to the present invention, may be selected from non-differentiated or differentiated cells. According to one embodiment non-differentiated cells as defined above may be preferred. Such non-differentiated cells may provide advantageous properties, e.g. a prolonged effect of the (spherical) microcapsules, as used according to the present invention, e.g. the prolonged capability to express and secrete GLP-1 peptides, e.g. due to a longer life span of such non-differentiated cells. In an alternative embodiment, differentiated cells as defined above may be preferred for preparing the core of the (spherical) microcapsule, as used according to the present invention, since they typically do not proliferate any more and, thus, do not lead to any undesired proliferation of cells within the core of the (spherical) microcapsule, as used according to the present invention. Specific differentiation of cells may be carried out by a skilled person *in vitro* according to methods known in the art by adding selected differentiation factors to precursor cells. Preferably, cells are differentiated in such a way that a vast majority of cells (or at least 90%, more preferably at least 95 % and most preferably at least 99%) embedded in the core of the (spherical) microcapsule, as used according to the present invention, belongs to the same cell type. In particular, mesenchymal stem cells as defined above may be differentiated *in vitro*, e.g., into osteoblasts, chondrocytes, adipocytes such as fat cells, neuron-like cells such as brain cells, etc., and used herein accordingly. As to whether non-differentiated or differentiated cells are used for preparing the core of the (spherical) microcapsule, as defined herein, may be dependent on specific requirements of the disease to be treated, e.g. the site of affliction, the administration mode, the tissue chosen for implant, etc. A selection of appropriate cells may be carried out by a skilled person evaluating these criteria.

Furthermore, cells used for preparing the core of the (spherical) microcapsule, as defined herein, may be immortalised or non-immortalised cells, preferably immortalised cells. If immortalised cells are used, these cells preferably retain their capability of symmetric and/or asymmetric cell division as discussed above. According to the present invention cells are defined as immortal when they exceed the double life span of normal cells (i.e. of non-immortalised cells). The maximum life span of normal diploid cells *in vitro* varies dependent on the cell type (e.g. foetal *versus* adult cell) and culture conditions. Thus, the maximum life span of cultured normal cells *in vitro* is approximately 60-80 population doublings. For example, keratinocytes may divide around 80 times, fibroblasts more than 50 times, and lymphocytes about 20 times. Normal bone marrow stromal cells may exhibit a maximum life span of 30-40 population doublings. Preferably, a cell line used for preparation of the core of an (spherical) microcapsule, as used according to the present invention, may continuously grow past 350 population doublings and may still maintain a normal growth rate characteristic of young cells.

Methods for immortalising cells are widely known in the art and may be applied here accordingly (see e.g. WO 03/010305 or WO 98/66827, which are incorporated herein by reference). An exemplary method (according to WO 03/010305) comprises e.g. following steps:
a) culturing cells, e.g., stem cells, in particular stem cells derived from human bone marrow (e.g. (human) mesenchymal stem cells (MSC, hMSC)), in accordance with standard conventional cell culturing methods known to the skilled person;
b) transducing said cell cultures with a retroviral vector, comprising at least a fragment of the human telomerase reverse transcriptase (hTERT) gene or a variant thereof, by
   b1) culturing a packaging cell line (e.g. PA317 cells, PG13 cells, Phenix, etc.), wherein the packaging cell line are cells in which the retroviral vector is produced,
   b2) constructing a retroviral vector (e.g. derived from Moloney murine leukaemia virus, etc.), wherein the retroviral vector comprises at least a fragment of the catalytic subunit of the human telomeric repeat (hTRT) gene or a variant thereof, more preferably a hTERT cDNA fragment, e.g. a 3452 base pair EcoRI fragment from pGRN145 (Geron Corporation),
   b3) transfecting said packaging cell line, with said retroviral vector,
   b4) transducing said packaging cell line with said transfected cells, preferably by centrifuging the cells with the retroviral vector,
   b5) transducing cultured cells according to step a) above with the packaging cells of step b4), said cells comprising said retroviral vector.
c) obtaining an immortal cell line, wherein said immortalised cell line has substantially identical characteristics and properties compared to the cells of step a). As a result the inserted polynucleotide sequence derived from the human telomeric subunit (hTRT)gene may be transcribed and translated to produce a functional telomerase. One of skill will recognize that due to codon degeneracy a number of polynucleotide sequences will encode the same telomerase. In addition, telomerase variants are included, which have sequences substantially identical to a wildtype telomerase sequence and retain the function of the wildtype telomerase polypeptide (e.g. resulting from conservative substitutions of amino acids in the wildtype telomerase polypeptide).

### Modifications of the cells embedded in the core of the spherical microcapsules encoding and secreting the GLP-1 peptides

According to a particularly specific embodiment, the cells embedded in the core of the (spherical) microcapsule encoding and secreting the GLP-1 peptides and GLP-1 fusion peptides as defined herein may be further modified or engineered to additionally secrete a factor selected from the group consisting of anti-apoptotic factors, growth factors, VEGF and erythropoietin (EPO), anti-platelet factors, anti-coagulant factors, anti-thrombotic drugs, anti-angiogenic factors, or any further factor exhibiting cardioprotective function, etc.

According to one specific embodiment, the cells embedded in the core of the (spherical) microcapsule encoding and secreting the GLP-1 peptides and GLP-1 fusion peptides as defined herein may be engineered to additionally secrete erythropoietin (EPO). Erythropoietin (also known as EPO, epoetin or procrit) is an acidic glycoprotein hormone of approximately 34,000 dalton molecular weight occurring in multiple forms, including alpha, beta, omega and asialo. Erythropoietin stimulates red blood cell production. It is produced in the kidney and stimulates the division and differentiation of committed erythroid precursors in the bone marrow and elsewhere. Generally, erythropoietin is present in very low concentrations in plasma when the body is in a healthy state, in which tissues receive sufficient oxygenation from the existing number of erythrocytes. This normal low concentration is enough to stimulate replacement of red blood cells that are lost normally through aging. The amount of erythropoietin in the circulation is increased under conditions such as hypoxia, when oxygen transport by blood cells in the circulation is reduced. Hypoxia may be caused by loss of large amounts of blood through haemorrhage, destruction of red blood cells by overexposure to radiation, reduction in oxygen intake due to high altitudes or prolonged unconsciousness, or various forms of anaemia or ischemia. In response to tissues undergoing hypoxic stress, erythropoietin will increase red blood cell production by stimulating the conversion of primitive precursor cells in the bone marrow into proerythroblasts which subsequently mature, synthesize haemoglobin and are released into the circulation as red blood cells. When the number of red blood cells in circulation is greater than needed for normal tissue oxygen requirements, erythropoietin in circulation is decreased. Preferably, erythropoietin is used as an additional factor contained in the cells to induce production of red blood cells to combat anaemia. (See, e.g., Bottomley *et al*. (2002) Lancet Oncol. 3:145). Erythropoietin has also been suggested to be useful in controlling bleeding in patients with abnormal haemostasis. (See e.g., U.S. Pat. No. 6,274,158). Recombinant human erythropoietin (rHuEpo or epoetin [alpha]) is commercially available as EPOGEN(R) (epoetin alfa, recombinant human erythropoietin) (Amgen Inc., Thousand Oaks, Calif.) and as PROCRIT(R) (epoetin alfa, recombinant human erythropoietin) (Ortho Biotech Inc., Raritan, N.J.). EPO may increase the hematocrit values in patients suffering from AMI or MI. The normal ranges for hematocrit values of erythropoietin are 37-48 percent for women and 42-52 percent for men. (See Case Records of the Massachusetts General Hospital: normal reference laboratory values. (1992) N. Eng. J. Med. 327:718). Of course, the safety and efficacy of use of erythropoietin to increase hematocrit levels in patients with cardiovascular disease, especially those suffering from renal failure, must be further evaluated. Preferably, erythropoietin is typically provided at a concentration or for a duration that will not induce red blood cell formation or alternatively, increase the hematocrit in a subject, e.g., between about 1 pM and less than 1000 µM, including less than 900 µM, less than 700 µM, less than 500 µM, less than 300 µM, less than 100 µM, or less than 50 µM. In other embodiments, erythropoietin is administered as a function of the subject's body weight. Erythropoietin may typically be provided at a concentration of between about 1 U/kg to 10,000 U/kg of a subject's body weight, including less than 7,500 U/kg, 5,000 U/kg, 2500 U/kg, 1000 U/kg, 750 U/kg, 500 U/kg, 250 Ug/kg, 100 Ug/kg, 50 U/kg, 25 U/kg, 10 U/kg, 5 U/kg, or 1 U/kg. In this context, erythropoietin serum concentration is normally within the range of 5-50 mU/ml. For patients suffering from MI or AMI or other conditions associated thereto, erythropoietin is preferably provided either at a concentration of 50-100 U/kg depending on symptom, body weight, sex, animal species and the like. It is generally assumed that treatment options holding the blood concentration at about 1-100 mU/ml will be preferred. Also preferably, erythropoietin is typically provided at a concentration that does not increase the hematocrit in a survivor, wherein the erythropoietin is administered in a single dose within 1, 2 or 3 hours of the myocardial infarction, for an extended period of time.

According to one further specific embodiment, the cells embedded in the core of the (spherical) microcapsule encoding and secreting the GLP-1 peptides and GLP-1 fusion peptides as defined herein may be engineered to additionally secrete VEGF.

According to another specific embodiment, the cells embedded in the core of the (spherical) microcapsule encoding and secreting the GLP-1 peptides and GLP-1 fusion peptides as defined herein may be engineered to additionally secrete antiapoptotic factors. Such factors may include, without being limited thereto, APC (apoptosis repressor with caspase recruitment domain), Bcl-2, Bcl-xL, Che-1/AATF, clusterin, insulin, Mcl-1, NF-kB-dependent antiapoptotic factors, serotonin, survivin, etc. Furthermore, any factor, which acts as an inhibitory factor to an apoptotic factor known in the art, and which may thus be regarded as antiapoptotic factors, is encompassed herewith. Such factors are preferably encoded by a nucleic acid and secreted by the cells encoding and secreting the GLP-1 peptides and GLP-1 fusion peptides as defined herein. In this context, such antiapoptotic factors may be directed against at least one of the following apoptotic factors or apoptosis related proteins including AIF, Apaf e.g. Apaf-1, Apaf-2, Apaf-3, oder APO-2 (L), APO-3 (L), Apopain, Bad, Bak, Bax, Bcl-2, Bcl-x_{L}, Bcl-x_{S}, bik, CAD, Calpain, Caspase e.g. Caspase-1, Caspase-2, Caspase-3, Caspase-4, Caspase-5, Caspase-6, Caspase-7, Caspase-8, Caspase-9, Caspase-10, Caspase-11, ced-3, ced-9, c-Jun, c-Myc, crm A, cytochrom C, CdR1, DcR1, DD, DED, DISC, DNA-PK_{CS}, DR3, DR4, DR5, FADD/MORT-1, FAK, Fas (Fas-ligand CD95/fas (receptor)), FUCE/MACH, FLIP, fodrin, fos, G-Actin, Gas-2, gelsolin, granzyme A/B, ICAD, ICE, JNK, lamin A/B, MAP, MCL-1, Mdm-2, MEKK-1, MORT-1, NEDD, NF-ₖₐₚₚₐB, NuMa, p53, PAK-2, PARP, perforin, PITSLRE, PKCdelta, pRb, presenilin, prlCE, RAIDD, Ras, RIP, sphingomyelinase, thymidinkinase from herpes simplex, TRADD, TRAF2, TRAIL-R1, TRAIL-R2, TRAIL-R3, transglutaminase, etc.

### (Spherical) microcapsule - GLP-1 peptides secreted by cells contained in the core of the (spherical) microcapsule

A GLP-1 peptide encoded and secreted by a cell contained in the core of the (spherical) microcapsule, as defined above, may be selected from any known GLP-1 peptide sequence. In this context, cells embedded in the core of the (spherical) microcapsule are typically transfected prior to preparing the core with nucleic acid sequences encoding such a GLP-1 peptide such that these cells express and secrete the GLP-1 peptide. Preferably a GLP-1 peptide encoded and secreted by a cell contained in the (spherical) microcapsule may be selected from a group consisting of a peptide comprising aa 7 - 35 of GLP-1 and a peptide showing a homology of at least 80 %, 90 %, 95 % or even 99 % with this peptide. More preferably, the peptide GLP-1 may be selected from group consisting of a peptide comprising aa 1 - 37 of GLP-1, a peptide comprising aa 7 - 35, 36 or 37 of GLP-1, GLP-1 (7-36)amide and a peptide showing a homology of at least 80 %, 90 %, 95 % or even 99 % with any of these peptides, including modified peptides. In this context, a "modified GLP-1 peptide" is intended to mean any GLP-1 variant or a GLP-1 fragment, including combinations, e.g. a fragment of a variant. Variants and fragments are categorized as modifications of the unmodified GLP-1 sequence, e.g. GLP-1(7-35, 36 or 37). Within the meaning of the present invention any variant or fragment has to be functional, e.g. has to exert the same or a similar biological activity as the unmodified (GLP-1) peptide. The term "activity" refers to the biological activity (e.g. one or more of the biological activities comprising receptor binding, activation of the receptor, exhibition of beneficial effects known for GLP-1, e.g. its activity to powerfully reduce the damages caused by ischemia or oxygen shortage and potential death of heart tissue as mentioned above in connection with the effects of GLP-1 as described in the prior art, which may be compared under the same conditions for the naturally occurring GLP-1 peptide as defined herein and any fragment or variant thereof. Preferably, a variant or fragment of a GLP-1 peptide as defined herein exerts at least 25% activity of a GLP-1 (7-35, 36 or 37), more preferably at least 50% (biological) activity, even more preferably 60, 70, 80 or 90% (biological) activity and most preferably at least 95 or 99% (biological) activity of a GLP-1 (7-35, 36 or 37) as defined above.

According to a particularly preferred embodiment, the GLP-1 peptide as defined above or a GLP-fusion peptide as defined below does not include at its N-terminus the naturally occurring amino acids 1 to 6 of a (native) GLP-1 (1-37) sequence as defined above. Even more preferably, the GLP-1 peptide as defined above or a GLP-fusion peptide as defined below does not include at its N-terminus the naturally occurring amino acids 1, 2, 3, 4, 5 and/ or 6 of a native GLP-1 (1-37) sequence as defined above. This proviso preferably refers to GLP-1 peptides as defined above, e.g. selected from the group consisting of a peptide comprising aa 1 - 37 of GLP-1, a peptide comprising aa 7 - 35, 36 or 37 of GLP-1, GLP-1 (7-36)amide and a peptide showing a homology of at least 80 %, 90 %, 95 % or even 99 % with any of these peptides, including modified peptides, and to fusion GLP-1 fusion peptides containing such GLP-1 peptides. However, this proviso does not exclude, that such a GLP-1 peptide as defined herein or a GLP-1 fusion peptide as defined herein, comprises a N-terminal sequence (or C-terminal) modification or additional amino acids or peptides fused thereto, e.g. signal peptides, etc.

According to an alternative embodiment, a GLP-1 peptide encoded and secreted by cells embedded in the core of the (spherical) microcapsule may be selected from a GLP-1 fusion peptide or a variant or fragment thereof. The GLP-1 fusion peptides as defined herein have at least two components, e.g. components (I) and (II), components (I) and (III) or components (I), (II) and (III), exhibit GLP-1's biological activity as defined herein and, simultaneously, confer stability to component (I) of GLP-1 fusion peptides by a C-terminal elongation.

Component (I) of GLP-1 fusion peptides as defined herein contains a sequence having at least 80 %, more preferably at least 85 % and even more preferably at least 90 % sequence homology with SEQ ID NO: 1. SEQ ID NO:1 represents the native amino acid sequence of GLP-1(7-37) (length of 31 amino acids), which is strictly conserved among mammalians. According to a particularly preferred embodiment, component (I) of GLP-1 fusion peptides as defined herein contains a sequence being identical to SEQ ID NO: 1.

Component (II) of the GLP-1 fusion peptide encoded and secreted by cells embedded in the core of the (spherical) microcapsule, as defined herein, (or more generally any GLP-1 peptide including fragments or variants of fusion peptides) typically contains a peptide sequence having at least nine amino acids. Component (II) of the GLP-1 fusion peptide furthermore may contain at least one proline residue in its sequence. Proline residues are common amino acids within a β-turn forming tetrameric amino acid sequence. Thus, component (II) of the GLP-1 fusion peptide may form a β-turn like structure. A β -turn structure is a typical secondary structure element of proteins or peptides. It is typically formed by a stretch of four amino acids, which reverts the direction of the peptide's or protein's backbone chain direction. If present in the GLP-1 fusion peptide, the proline residue is commonly located at position 2 or 3, preferably at position 2, of a tetrameric β-turn sequence occurring in component (II) of the GLP-1 fusion peptide.

Cells embedded in the core of the (spherical) microcapsule, as defined herein, are typically transfected prior to preparing the core with nucleic acid sequences encoding a GLP-1 fusion peptide such that these cells encode, express and secrete the GLP-1 fusion peptide. Particularly preferred in this context is a GLP-1 fusion peptide as defined herein, wherein component (II) is a peptide sequence containing a sequence according to SEQ ID NO: 22 (RRDFPEEVAI) (all peptide sequences given in the one-letter-code) or a sequence having at least 80% sequence homology with SEQ ID NO: 22. SEQ ID NO: 22 is a partial sequence of the full-length IP-2 (intervening peptide 2) sequence, which contains the 10 N-terminal amino acids of the 15 amino acid long full-length IP-2 sequence. IP-2 is a preferred example of a component (II) as used herein. Accordingly, other stronger preferred sequences being contained in component (II) are longer partial amino acid sequences of IP-2, such as the 14 N-terminal amino acid sequence occurring in humans (SEQ ID NO: 23 (RRDFPEEVAIVEEL)) or its murine counterpart (SEQ ID NO: 24 (RRDFPEEVAIAEEL)) or a sequence having at least 80% sequence homology with SEQ ID NOs: 23 or 24. Most preferred as elements being contained in component (II) of the fusion peptide are full-length IP-2 sequences having all 15 amino acids of the naturally occurring IP-2 sequence (SEQ ID NO: 2 (RRDFPEEVAIVEELG), human, or SEQ ID NO: 3 (RRDFPEEVAIAEELG), murine) or a sequence having at least 80% sequence homology with SEQ ID NOs: 2 or 3. Within the scope of the present invention are also all mammalian isoforms of IP2 (natural variants of IP2 among mammalians). More than one copy of a sequence being included into component (II) may be provided, e.g. 2, 3 or even more copies of IP2 or a fragment or variant of IP2.

Accordingly, a GLP-1 fusion peptide, encoded and secreted by cells embedded in the core of the (spherical) microcapsule, as defined herein, preferably contains sequences according to SEQ ID NO: 8 (HAEGTFTSDVSSYLEGQAAKEFIAWLVKGRGRRDFPEEVAIAEELG), i.e. GLP-1(7-37) linked without any linker sequence *via* its C-terminus to murine IP2 or according to SEQ ID NO: 12 (HAEGTFTSDVSSYLEGQAAKEFIAWLVKGRGRRDFPEEVAIVEELG), i.e. GLP-1(7-37) linked without any linker sequence *via* its C-terminus to human IP2. Variants or fragments thereof having a sequence homology of at least 80 % with SEQ ID NOs: 8 and 12 or fragments or variants thereof may be used herein as well. Preferred GLP1-fusion peptides in this context may further comprise sequences according to SEQ ID NOs: 13, 14, 19 and 20.

Without being bound to any theory, it is concluded by the inventors of the present invention that the instability of GLP-1 (7-35, 36 or 37), if secreted *in vivo* by cells embedded in the core of the implanted (spherical) microcapsule, as used according to the present invention, into the patients surrounding tissue, is due to its unprotected 3-dimensional structure. Proteases may cleave the GLP-1 (7-35, 36 or 37) peptide and abolish its physiological activity rapidly *in vivo*. By linking a peptide sequence to the C-terminus of GLP-1 (7-35, 36 or 37) its structure gains stability towards enzymatic degradation. Such gain in stability may be enhanced, if the additional C-terminal peptide sequence (being contained in component (II) of the fusion peptide according to the invention) folds back, e.g. due to the presence of a β-turn structural element formed by its primary structure and providing rigidity to component (II). The GLP-1 peptide as defined above, by virtue of its C-terminal peptide extension preferably containing a β-turn structural element, is found to have improved resistance to DPP-IV inactivation. The C-terminal peptide is either not cleaved from the GLP-1(7-35, 36 or 37) sequence prior to acting on its receptor in target cells or it may be cleaved enzymatically to form GLP-1 (7-35, 36 or 37) *in vivo*. Irrespective of the exact form of the GLP-1 peptide bound at the site of the GLP-1 receptor, an GLP-1 peptide as defined above exerts its function as an active insulinotropic compound.

GLP-1 peptide sequences, which are considered to be suitable for being contained in component (II) of a GLP-1 fusion peptide as defined above due to a primary structure forming a β-turn element, may readily be identified by adequate, e.g., spectroscopic methods, e.g. circular dichroism, or other methods known to the skilled person.

Component (II) and component (I) of a GLP-1 fusion peptide, encoded and secreted by cells embedded in the core of the (spherical) microcapsule as defined herein, may be directly linked or linked *via* a linker sequence. Preferably, both components are directly linked with each other. In case they are linked *via* a linker (or spacer), the linker is preferably a peptide linker. The peptide linker typically has a length of 1 to 10 amino acids, preferably 1 to 5, even more preferably 1 to 3 amino acids, in some cases the linker sequence may be even longer comprising 11 to 50 amino acids. The peptide linker may be composed of various (naturally occurring) amino acid sequences. Preferably, the peptide linker will introduce some structural flexibility between components to be linked. Structural flexibility is achieved e.g. by having a peptide linker containing various glycine or proline residues, preferably at least 30%, more preferably at least 40% and even more preferably at least 60 % proline and glycine residues within the linker sequence. Irrespective of the specific sequence the peptide linker may preferably be immunologically inactive.

GLP-1 fusion proteins, encoded and secreted by cells embedded in the core of the (spherical) microcapsule as defined herein, may additionally contain a component (III). Generally, component (III) comprises at least four amino acid residues, preferably at least 10 additional amino acid residues, more preferably at least 20, or most preferably at least 30. In functional terms, component (III) is intended to further enhance the stability of a GLP-1 peptide as defined above. Component (III) is expected not to interfere with the biological function of the GLP-1 fusion peptide which is approximately comparable to the biological activity of GLP-1 (7-37). Generally spoken, any C-terminal elongation of component (I) as defined herein, whether it is component (II), component (III) or a combination of components (II) and (III) as defined herein, enhances stability of component (I), i.e. a GLP-1 (7-37) or its fragment or variant as defined herein.

Preferably, component (III) of the GLP-1 fusion peptide as defined above, comprises at least 4, preferably at least 10, more preferably at least 20 additional amino acid residues of the N-terminal sequence of an isoform of GLP-2 of any mammalian organism (other naturally occurring variant of GLP-2 among mammalian), e.g. murine or human isoforms as shown in SEQ ID NOs: 4 and 5. GLP-2 occurs in pro-glucagon and is also involved in carbohydrate metabolism. In the context of the present invention, the term "GLP-2 peptide" preferably means GLP-2 (1-33, 34, or 35), whereas "modified GLP-2 peptide" is intended to mean any GLP-2 fragment or variant, or a fragment or variant of GLP-2(1-33, 34 or 35). Variants or fragments are categorized as modifications of the unmodified sequence, e.g. GLP-2(1-33, 34 or 35). As with the biologically active sequence included in component (I) (GLP-1 peptide), component (III) may also comprise variants or fragments of naturally occurring forms of GLP-2. Alternatively, component (III) may also comprise at least 4, preferably at least 10, more preferably at least 20 additional amino acid residues of the (N-terminal) sequence of GLP-1(7-37), correspondingly including all mammalian isoforms or - as disclosed herein - all functional fragments or variants thereof. Generally speaking, component (III) may contain any form of a GLP-1 peptide or a modified GLP-1 peptide, which is disclosed herein as suitable for component (I) of the GLP-1 fusion peptide. In a further alternative, component (III) may also contain chimeric forms of GLP-1 (7-37) and GLP-2. A chimeric form may be produced by coupling GLP-1 (7-37) and GLP-2 (or fragments or variants) with each other and by subsequently introducing this chimeric form as component (III) into the GLP-1 fusion peptide. Preferably, the chimeric form is composed of a partial sequence of GLP-1 (7-37) and a partial sequence of GLP-2 linked together. E.g. the chimeric form may include the N-terminal 5 to 30 amino acids of GLP-1 and the C-terminal 5 to 30 amino acids of GLP-2 or vice versa, e.g. amino acids 7 or 8 to 22, 23, 24, 25, 26, 27, or 28 of GLP-1 (7-37) and amino acid sequence from position 15, 16, 17, 18, 19, 20, 21, 22, 23 or 24 to e.g. the C-terminus of GLP-2.

If modifications of naturally occurring forms of GLP-2 or GLP-1 (7-37), respectively, are contained as component (III), component (III) preferably contains the sequence of SEQ ID NOs: 1, 4 or 5, respectively, or a sequence having at least 80% sequence homology with any of SEQ ID NOs: 1, 4 or 5.

In another embodiment, component (III) of the GLP-1 fusion peptide encoded and secreted by cells embedded in the core of the (spherical) microcapsule as defined herein, may contain a plurality of sequences as described above. E.g. component (III) may contain at least two, preferably 2, 3, or 4 copies of GLP-1 (7-37) and/or GLP-2 or at least two copies of sequences having at least 80% sequence homology with SEQ ID NOs: 1, 4 or 5. Also, component (III) may contain more than one copy of a chimeric version of GLP-1 (7-37) or GLP-2, as disclosed above, e.g. eventually forming a combination of chimeric version(s) together with GLP-1 (7-37) and/or GLP-2 or its modifications with at least 80 % sequence homology. A GLP-1 fusion peptide encoded by nucleic acids transfected into cells used according to the present invention for preparing the core of the (spherical) microcapsule, as used according to the present invention, may also comprise two or more, preferably two, components (III), which may e.g. be (1) linked by its N-terminus to the C-terminus of component (I) or (II) and (2) linked by its C-terminus to the N-terminus of component (I) *via* a linker or directly. If two components (III) are provided, these may be identical or different.

According to a preferred embodiment, cells (embedded in the core of the (spherical) microcapsules, as used according to the present invention) are preferred, which are transfected with nucleic acids encoding GLP-1 fusion peptides containing three components (I), (II) and (III). Specific embodiments containing all of these components are selected from a group consisting of: SEQ ID NO: 6 (N-GLP-1(7-37)-IP2(murine)-RR-GLP-1(7-37)-C, also designated murine CM1 herein), SEQ ID NO: 7 (N-GLP-1(7-37)-IP2(murine)-RR-GLP2-C, also designated murine CM2 herein), SEQ ID NO: 10 (N-GLP-1(7-37)-IP2(human)-RR-GLP-1(7-37)-C, also designated human CM1), and SEQ ID NO: 11 (N-GLP-1(7-37)-IP2(human)-RR-GLP-2-C), also designated human CM2 herein) or a sequence having at least 80% sequence homology with SEQ ID NOs: 6, 7, 10, or 11 or a fragment or variant thereof. All sequences according to SEQ ID NOs: 6, 7, 10 and 11 contain an RR-Linker (two arginine residues) at the C-terminus of IP2 (component (II)), which may alternatively also be discarded. Component (I) in each of the embodiments according to SEQ ID NOs: 6, 7, 10 or 11 is GLP-1 (7-37), whereas component (III) (in each of these embodiments linked to the C-terminus of component (II)) is either GLP-1(7-37) or GLP-2. Preferred GLP1-fusion peptides in this context may further comprise sequences according to SEQ ID NOs: 15, 16, 17, 18 and 26.

In another preferred embodiment of the present invention, a GLP-1 fusion peptide encoded and secreted by cells embedded in the core of the (spherical) microcapsule, as defined herein, contains in addition to component (I) a component (III) (without any component (II) as defined above) which is either linked to the C-terminus of component (I) and/or to the N-terminus of component (I). Preferably, component (III) is located at the C-terminus of component (I). Irrespective of whether component (III) is linked to the N-terminus of component (I) (by its C-terminus) or to the C-terminus of component (I) (by its N-terminus), the coupling may be direct or indirect *via* a linker sequence. With regard to the linker sequence it is referred to the above disclosure for a linker connecting component (I) and component (II).

In an alternative preferred embodiment of the present invention, a GLP-1 fusion peptide encoded and secreted by cells embedded in the core of the (spherical) microcapsule, as defined herein, contains in addition to components (I) and (II) a component (III) which is either linked to the C-terminus of component (II) and/or to the N-terminus of component (I). Preferably, component (III) is located at the C-terminus of component (II). Irrespective of whether component (III) is linked to the N-terminus of component (I) (by its C-terminus) or to the C-terminus of component (II) (by its N-terminus), the coupling may be direct or indirect *via* a linker sequence. With regard to the linker sequence it is again referred to the above disclosure for a linker connecting component (I) and component (II).

The GLP-1 fusion protein embedded in the core of the (spherical) microcapsule, as used according to the present invention, may furthermore comprise in addition to any of the afore mentioned combinations of components of the fusion protein (i.e. components (I) and (II), components (I) and (III) or components (I), (II) and (III)) a carrier protein, in particular transferrin or albumin, as component (IV). Such a component (IV) may be linked to the N-and/or C-terminus of any of the afore mentioned combinations of components of the fusion protein, i.e. components (I) and/or (II), components (I) and/or (III) or components (I), (II) and/or (III), either directly or using a linker as defined above.

In a specific embodiment of the invention, the GLP-1 (fusion) peptide as defined herein and as secreted from cells embedded in the (spherical) microcapsules as used herein, contains as component (I) and/or (III) a modified GLP-1 peptide comprising the amino acid sequence of the following formula II:
Xaa7-Xaa8-Glu-Gly-Thr-Phe-Thr-Ser-Asp-Xaa16-Ser-Xaa18-Xaa19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is Ser, Lys or Arg; Xaa19 is Tyr or Gln; Xaa20 is Leu or Met; Xaa22 is Gly, Glu or Aib; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent.

In still another specific embodiment of the invention component (I) and/or (III) of the GLP-1 (fusion) peptide as used for the (spherical) microcapsules, as used according to the present invention, contains a modified GLP-1 peptide comprising the amino acid sequence of the following formula III:
Xaa7-Xaa8-Glu-Gly-Thr-Phe-Thr-Ser-Asp-Val-Ser-Xaa8-Tyr-Leu-Glu-Xaa22-Xaa23-Ala-Ala-Xaa26-Glu-Phe-Ile-Xaa30-Trp-Leu-Val-Xaa34-Xaa35-Xaa36-Xaa37,
wherein Xaa7 is L-histidine, D-histidine, desamino-histidine, 2-amino-histidine, -hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent.

In a particular preferred embodiment the (spherical) microcapsule of the invention contains a GLP-1 (fusion) peptide, component (I) and/or (III) of which contain a (modified) GLP-1 peptide, which is selected from GLP-1 (7-35), GLP-1 (7-36), GLP-1 (7-36)-amide, GLP-1 (7-37) or a variant, analogue or derivative thereof. Also preferred are GLP-1 (fusion) peptides comprising in their components (I) and/or (III) a modified GLP-1 peptide having a Aib residue in position 8 or an amino acid residue in position 7 of said GLP-1 peptide, which is selected from the group consisting of D-histidine, desamino-histidine, 2-amino-histidine, hydroxy-histidine, homohistidine, N-acetyl-histidine, α-fluoromethyl-histidine, α-methyl-histidine, 3-pyridylalanine, 2-pyridylalanine and 4-pyridylalanine.

Both embodiments of components (I) and/or (III) of the GLP-1 (fusion) peptide as defined above by formulae II and III and usable for the present invention may be combined with the disclosure given above for GLP-1 (fusion) peptide. In other words, general formulae II and III may be combined e.g. with the disclosure given above for component (II), linkers, process of manufacturing, etc.

### Modifications of GLP-1 peptides and GLP-1 fusion peptides

The GLP-1 peptides and GLP-1 fusion peptides as defined above, encoded and secreted by cells contained in the core of the (spherical) microcapsule as defined above, may occur in various modified forms. These modified forms are disclosed in the following and described in more detail and comprise, e.g. fragments, variants, etc., of GLP-1 peptides or fusion peptides as defined herein.

A "fragment" of a GLP-1 peptide encoded and secreted by a cell as embedded in the (spherical) microcapsule, as used according to the present invention, refers to any subset of the above disclosed GLP-1 peptides including GLP-1 fusion peptides, that is, a shorter peptide which retains the desired biological activity. Fragments may readily be prepared by removing amino acids from either end of the molecule and testing the resultant for its biological properties as defined above for GLP-1. Proteases for removing one amino acid at a time from either the N-terminal end and/or the C-terminal end of a polypeptide are known, and so determining fragments which retain the desired biological activity involves only routine experimentation. Conclusively, fragments may be due to deletions of amino acids at the peptide termini and/or of amino acids positioned within the peptide sequence.

Additionally, the GLP-1 peptide as defined herein which a biological activity as defined above, be it a fusion peptide itself, a functional variant and/or fragment thereof, can also contain additional amino acid residues flanking the GLP-1 peptide. As long as the resultant molecule retains its resistance or stability towards proteases and its ability to act as defined above, one can determine whether any such flanking residues affect the basic characteristics of the core peptide, e.g. by its beneficial effects known for GLP-1, e.g. its activity to powerfully reduce the damages caused by ischemia or oxygen shortage and potential death of heart tissue, by routine experimentation. The term "consisting essentially of", when referring to a specified sequence, means that additional flanking residues can be present which do not affect the basic characteristic of the specified GLP-1 peptide. This term does not comprehend substitutions, deletions or additions within the specified sequence.

A "variant" of a GLP-1 peptide encoded and secreted by a cell as embedded in the (spherical) microcapsule, as used according to the present invention, refers to a molecule which is substantially similar to either the entire GLP-1 peptide defined above or a fragment thereof. Variant peptides may be conveniently prepared using methods well known in the art. Of course, such variant of a GLP-1 peptide would have similar beneficial effects known for GLP-1, e.g. its activity to powerfully reduce the damages caused by ischemia or oxygen shortage and potential death of heart tissue as the corresponding naturally-occurring GLP-1 peptide. E.g. amino acid sequence variants of the GLP-1 peptides defined above can be prepared by mutations in the DNA sequences which encode the synthesized variants. Such variants include, for example, deletions from, or insertions or substitutions of, residues within the amino acid sequence. Any combination of deletion, insertion, and substitution may also be contained in GLP-1 peptides encoded and secreted by a cell as embedded in the (spherical) microcapsule as defined herein, provided that the final construct possesses the desired activity. Obviously, the mutations that will be made in the DNA encoding the variant peptide must not alter the reading frame and preferably will not create complementary regions that could produce secondary mRNA structure.

The types of substitutions which may be contained in the GLP-1 peptide as used for preparing the (spherical) microcapsule as defined above, may be based on analysis of the frequencies of amino acid changes between a homologous protein/peptide of different species. Based upon such analysis, conservative substitutions may be defined herein as exchanges within one of the following five groups:
I. Small, aliphatic, non-polar or slightly polar residues: Ala, Ser, Thr, Pro, Gly;
II. Polar, negatively-charged residues and their amides: Asp, Asn, Glu, Gln;
III. Polar, positively-charged residues: His, Arg, Lys;
IV. Large, aliphatic non-polar residues: Met, Leu, Ile, Val, Cys;
V. Large aromatic residues: Phe, Try, Trp.

Within the foregoing groups, the following substitutions are considered to be "highly conservative": Asp/Glu; His/Arg/Lys; Phe/Tyr/Trp; Met/Leu/Ile/Val. Semi-conservative substitutions are defined to be exchanges between two of groups (I) - (IV) above which are limited to supergroup (A), comprising (I), (II), and (III) above, or to supergroup (B), comprising (IV) and (V) above. Substitutions are not limited to the genetically encoded or even the naturally-occurring amino acids.

In general, variants of the GLP-1 peptides (and GLP-1 fusion peptides) defined above may also contain amino acid substitutions, made e.g. with the intention of improving solubility (replacement of hydrophobic amino acids with hydrophilic amino acids).

In one embodiment the (modified) GLP-1 peptide, being encoded and secreted by a cell as embedded in the (spherical) microcapsule, as defined above, including a GLP-1 fusion peptide as defined above (occurring in component (I) and/or (III) of the GLP-1 fusion peptide), is characterized by one or more substitution(s) at positions 7, 8, 11, 12, 16, 22, 23, 24, 25, 27, 30, 33, 34, 35, 36, or 37 of the GLP-1 peptide. As an example for the following nomenclature [Arg34-GLP-1 (7-37)] designates a GLP-1 analogue wherein the naturally occurring lysine at position 34 has been substituted with arginine.

Specifically, a GLP-1 peptide or component (I) and/or (III) of a GLP-1 fusion peptide as defined herein may correspond to variants of GLP-1 (7-35, 36, 37 or 38) including, for example, Gln9-GLP-1 (7-37), D-Gln9-GLP-1(7-37), acetyl-Lys9-GLP-1 (7-37), Thr16-Lys18-GLP-1 (7-37), and Lys18-GLP-1 (7-37), Arg34-GLP-1 (7-37), Lys38-Arg26-GLP-1 (7-38)-OH, Lys36-Arg26-GLP-1 (7-36), Arg26,34-Lys38-GLP-1 (7-38), Arg26,34-Lys38-GLP-1 (7-38), Arg26,34-Lys38-GLP-1 (7-38), Arg26,34-Lys38-GLP-1 (7-38), Arg26,34-Lys38-GLP-1 (7-38), Arg26-Lys38-GLP-1(7-38), Arg26-Lys38-GLP-1(7-38), Arg26-Lys38-GLP-1 (7-38), Arg34-Lys38-GLP-1 (7-38), Ala37-Lys38-GLP-1 (7-38), and Lys37-GLP-1 (7-37).

In a particular preferred embodiment of the invention the GLP-1 peptide as encoded and secreted by a cell as embedded in the (spherical) microcapsule, as used according to the present invention, including a GLP-1 fusion peptide as defined above (with respect to component (I) or (III)) is/contains a (modified) GLP-1 peptide, which is selected from GLP-1 (7-35), GLP-1 (7-36), GLP-1 (7-36)-amide, GLP-1 (7-37) or a fragment or variant thereof.

Preferably, a variant of the GLP-1 peptide as defined above (including a variant of a GLP-1 fusion peptide (with respect to component (I) or (III))), encoded and secreted by a cell embedded in the (spherical) microcapsule as defined herein, will have preferably a "core" sequence. Such a "core" sequence is the same as that of the "native" sequence, e.g. GLP-1 (7-37) or GLP-2 or biologically active fragment thereof or any IP2 isoform, which has an amino acid sequence having at least 70% identity to the native amino acid sequence and retains the biological activity thereof. More preferably, such a "core" sequence exhibits at least 80% identity, at least 90% identity, or most preferably at least 95% identity to the native sequence.

The term "sequence identity" as defined herein means that the sequences are compared as follows. To determine the percent identity of two amino acid sequences, the sequences can be aligned for optimal comparison purposes (e.g., gaps can be introduced in the sequence of a first amino acid sequence). The amino acids at corresponding amino acid positions can then be compared. When a position in the first sequence is occupied by the same amino acid as the corresponding position in the second sequence, then the molecules are identical at that position. The percent identity between the two sequences is a function of the number of identical positions shared by the sequences, e.g. where a particular peptide is said to have a specific percent identity to a reference polypeptide of a defined length, the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Of course, other polypeptides will meet the same criteria. Such a determination of percent identity of two sequences can be accomplished using a mathematical algorithm. A preferred, non-limiting example of a mathematical algorithm utilized for the comparison of two sequences is the algorithm of Karlin et al. (1993), PNAS USA, 90:5873-5877. Such an algorithm is incorporated into the NBLAST program, which can be used to identify sequences having the desired identity to the amino acid sequence of the invention. To obtain gapped alignments for comparison purposes, Gapped BLAST can be utilized as described in Altschul et al. (1997), Nucleic Acids Res, 25:3389-3402. When utilizing BLAST and Gapped BLAST programs, the default parameters of the respective programs (e. g., NBLAST) can be used. The sequences further may be aligned using Version 9 of the Genetic Computing Group's GAP (global alignment program), using the default (BLOSUM62) matrix (values-4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the claimed sequence. The described methods of determination of the percent identity of two amino acid sequences can be applied correspondingly to nucleic acid sequences.

The GLP-1 peptides encoded by and secreted from cells embedded in the (spherical) microcapsules as defined above, particularly GLP-1 fusion peptides, may be protected against proteolytic cleavage as outlined above. They may be preferably protected against DPP-IV. GLP-1 peptides as defined herein as well as their fragments and variants, particularly GLP-1 fusion peptides, may contain a sequence of GLP-1, e.g. GLP-1 (7-35, 36 or 37) (in case of GLP-1 fusion peptides as part of component (I) and/or (III)), resistant to the DPP-IV.

Resistance of a peptide to degradation by dipeptidyl aminopeptidase IV may be determined e.g. by the following degradation assay: Aliquots of the peptides are incubated at 37°C with an aliquot of purified dipeptidyl aminopeptidase IV for 4-22 hours in an appropriate buffer at pH 7-8 (buffer not being albumin). Enzymatic reactions are terminated by the addition of trifluoroacetic acid, and the peptide degradation products are separated and quantified using HPLC or LC-MS analysis. One method for performing this analysis is: The mixtures are applied onto a Zorbax300SB-C18 (30 nm pores, 5 µm particles) 150 x 2.1 mm column and eluted at a flow rate of 0.5 ml/min with a linear gradient of acetonitrile in 0.1 % trifluoroacetic acid (0%-100% acetonitrile over 30 min). Peptides and their degradation products may be monitored by their absorbance at 214 nm (peptide bonds) or 280 nm (aromatic amino acids), and are quantified by integration of their peak areas. The degradation pattern can be determined by using LC-MS where MS spectra of the separated peak can be determined. Percentage intact/degraded compound at a given time is used for estimation of the peptides DPP-IV stability.

A GLP-1 (fusion) peptide as defined herein and as secreted from cells embedded in the (spherical) microcapsules as defined above, is defined as DPP-IV stabilized when it is 10 times more stable than the non-modified peptide sequence of GLP-1 (7-37) based on percentage intact compound at a given time. Thus, a DPP-IV stabilized GLP-1 peptide is preferably at least 10, more preferably at least 20 times more stable than e.g. GLP-1 (7-37). Stability may be assessed by any method known to the skilled person, e.g. by adding DPP-IV to a solution of the peptide to be tested and by determining the degradation of the peptide (see above), e.g. over a period of time, by e.g. a spectroscopic method, Western-Blot analysis, antibody screening etc. In parallel, a GLP-1 peptide (e.g. a fragment and/or variant or a GLP-1 fusion peptide) as defined above is defined as a compound, which exerts the effect of GLP-1(7-37) by e.g. binding to its native receptor (GLP-1 receptor). Preferably, a GLP-1 (fusion) peptide as defined above has a binding affinity to the GLP-1 receptor, which corresponds to at least 10%, preferably at least 50% of the binding affinity of the naturally occurring GLP-1 peptide. The binding affinity may be determined by any suitable method, e.g. surface plasmon resonance, etc. Moreover, it is preferred, if the GLP-1 (fusion) peptide as defined herein, evokes formation of intracellular cAMP by its binding to its extracellular receptor, which transmits the signal into the cell.

For *in vitro* control purposes the GLP-1 peptide or GLP-1 fusion peptide as defined herein may be isolated from the cells from which it is expressed, for instance using conventional separation techniques. Thus cells may be grown under appropriate conditions, for instance including support and nutrients, *in vitro*, and secreted protein, i.e. the GLP-1 peptide as defined above, is recovered from the extracellular medium. The sequences engineered into cells thus preferably include signal (peptide) sequences (see below) allowing secretion of the GLP-1 peptide as defined above (see below). The cells preferably express a protease capable of cleaving the signal sequences, either naturally endogenously or after transfection of introduced into the cell by genetic engineering methods. In an alternative, the engineered gene sequences encoding a GLP-1 peptide as defined herein do not include such signal peptide sequences, whereby the intracellularly expressed GLP-1 peptide will typically not be secreted, and is recovered from cells by processes involving cell lysis. In such methods the coding sequences may include purification tags allowing efficient extraction of the product peptide from the medium; tags may be cleaved off to release isolated GLP-1 peptide. However, this alternative is generally irrelevant to cells of a (spherical) microcapsule, as used according to the present invention, which are implanted into the patient and require delivery of a GLP-1 peptide into the surrounding tissue.

The GLP-1 peptide(s) and GLP-1 fusion peptide(s) as defined herein, encoded and secreted nucleic acids contained in cells embedded in the core of the (spherical) microcapsule as used herein, may furthermore contain or be conjugated to at least one synthetic polymer or a natural polymer, e.g. polyamino acids. At least one polymer constituent is typically covalently coupled to the fusion peptide subunit. "Conjugated" in the meaning of the present invention is intended to mean "chemically coupled". "Chemically coupled" is intended to mean coupled *via* covalent or non-covalent bonding. While covalent bonding is preferred, the polymer constituent may also be linked to the fusion peptide *via* complexation without covalent linkage, e.g. *via* hydrogen bonding or electrostatic, hydrophobic, etc., interaction. The entire complex containing the fusion peptide and the polymer is termed hereinafter "GLP-1 conjugate complex" or "GLP-1 conjugate molecule".

Thereby, the GLP-1 conjugate molecule is even further protected against the proteolytic degradation *in vivo* as already defined above, mainly due to proteolytic endopeptidase IV activity. The conjugate complex having or comprising a GLP-1 (fusion) peptide of at least two components (I) and (II) and the synthetic polymer exhibits GLP-1's biologically activity and, simultaneously, confers stability to the GLP-1 as its component (I) by a C-terminal elongation. Accordingly, by conjugating the fusion peptide to a polymer its *in vivo* stabilisation is increased considerably.

The polymer used herein for coupling the GLP-1 (fusion) peptide as defined herein may be a physiologically acceptable polymer which includes polymers which are soluble in an aqueous solution or suspension and have no negative impact, such as side effects, to mammals upon administration of the fusion peptide in a pharmaceutically effective amount. There is no particular limitation to the physiologically acceptable polymer used according to the present invention. The polymer may be of synthetic nature or may be a naturally occurring polymer (e.g. a protein).

One, two, or three polymer constituents may be covalently attached to the fusion peptide subunit, with one polymer constituent being preferred, to form the GLP-1 conjugate molecule. However, in specific embodiments more than three polymer constituents may be provided per fusion peptide subunit. The constituents may be covalently coupled to either component (I) or component (II) of the fusion peptide or both of them. It is preferred to couple at least one of the polymer constituents to component (II). If one or more polymer constituent(s) is/are coupled to component (I) it/they is/are preferred to be coupled to the N-terminus or to the side chains of serine, threonine, tyrosine, aspartate, glutamate, lysine or arginine residues. Preferably, the side chains of one or more of residues Thr 11, Thr 13, Asp15, Ser 17, Ser 18, Tyr 19, Glu 21, Lys 26, Glu 27, Lys 34, Arg 36 of component (I) are used for coupling purposes. If the naturally occurring sequence of IP2 is used as component (II) one or more of its Arg, Glu and Asp residues will modified at their side chains by a polymer constituent(s).

The N-terminal binding of a polymer, in particular PEG, may offer advantages in purification of the conjugate molecule. It is also believed that the N-terminal binding of a polymer may better preserve the bioactivity compared with random polymer binding to any other residue, e.g. any other lysine residue. Accordingly, in a preferred embodiment, at least one polymer constituent is located at the N-terminus of the fusion peptide. If PEGylation is used, PEGylation of terminal or side chain carboxyl groups or the ε-amino group of lysine occurring in the inventive peptide, confers resistance to oxidation and is also within the scope of the present invention.

More generally, the synthetic polymer of a GLP-1 (fusion) peptide, encoded and secreted by cells as used in the core of the herein defined (spherical) microcapsule is preferably selected from alkylene glycols, such as polyethylene glycol (PEG), polypropylene glycol (PPG), copolymers of ethylene glycol and propylene glycol, polyoxyethylated polyol, polyolefinic alcohol, polyvinylpyrrolidone, polyhydroxyalkyl methacrylamide, polyhydroxyalkyl methacrylate, such as polyhydroxyethylene methycrylate, polyacrylate, polysaccharides, poly([alpha]-hydroxy acid), polyvinyl alcohol, polyphosphazene, polyoxazoline, poly(N-acryloylmorpholine), polyvinylethyl ether, polyvinlyacetal, polylactic glycolic acid, polylactic acid, lipid polymer, chitin, hyaluronuic acid, polyurethyne, polysialic acid, cellulose triacetate, cellulose nitrate and combinations of any of the foregoing. Preferably natural polymers are peptide or proteins linked *via* side chains to the GLP-1 (fusion) peptide or *via* terminal groups (amino and/or carboxy), preferably albumin and transferrin.

Any of the above described embodiments or features may be combined with each other, if not indicated otherwise.

### Nucleic acids encoding GLP-1 peptides and GLP-1 fusion peptides

The GLP-1 peptides as defined above are produced in cells as defined above, i.e. encoded and secreted in cells embedded in the core of the (spherical) microcapsule(s) as used according to the present invention. For this purpose, GLP-1 peptides as defined above or its fragments or variants are encoded by nucleic acid sequences being contained in these cells. These nucleic acid sequences may occur naturally in the cells or may be introduced into the cells by cell transfection techniques prior to the preparation of the (spherical) microcapsule. According to the present invention any suitable nucleic acid sequence coding for a GLP-1 peptide as defined above may be used. Due to degeneracy of the genetic code a plurality of nucleic acid sequences may code for such a GLP-1 peptide. According to a preferred embodiment of the present invention a nucleic acid sequence used for transfection of cells as defined herein may comprise any nucleic acid sequence coding for the GLP-1 peptide(s) as defined above and additional (functional) nucleotide sequences. The present invention preferably provides a nucleic acid sequence suitable for transfection of a cell as defined herein which may code (a) for the entire GLP-1 aa sequence (GLP-1(1-37) or functional GLP-1 (7-35, 36 or 37) (variant) sequences or any other GLP-1 peptide, including GLP-1 fusion peptides as defined above, (b) optionally for a protease cleavage sequence at the N-terminus of the GLP-1 sequence according to (a) and, optionally, for a signal peptide sequence upstream from (b). Preferably, the signal (peptide) sequence is selected from a sequence as defined below.

The nucleic acid sequence as defined above may be contained in a vector. This vector may be used to transfect a cell as defined herein suitable for preparing the (spherical) microcapsule as used according to the present invention. Typically, such a vector, in particular an expression vector, contains at least one nucleic acid sequence as defined above. A "vector" within the meaning of the present invention advantageously comprises at least one nucleic acid sequence encoding a GLP-1 peptide as defined above and, if necessary, additional elements suitable for directing expression of the encoded GLP-1 peptide sequences. One class of vectors as used herein utilizes DNA elements that provide autonomously replicating extrachromosomal plasmids derived from animal viruses (e.g. bovine papilloma virus, polyomavirus, adenovirus, or SV40, etc.). A second class of inventive vectors as used herein relies upon the integration of the desired gene sequences into the host cell chromosome.

### Vectors comprising nucleic acids encoding GLP-1 peptides and GLP-1 fusion peptides

Vectors, as defined above, may be used to transfect a cell as defined herein, are typically prepared by inserting at least one GLP-1 peptide encoding nucleic acid sequence into suitable (empty) vectors. Such suitable (empty) vectors are known to a skilled person and may be reviewed e.g. in "Cloning Vectors" (Eds. Pouwels P. H. et al. Elsevier, Amsterdam-New York-Oxford, 1985, ISBN 0 444 904018). Suitable (empty) vectors are also intended to include any vector known to a skilled person, such as plasmids, phages, viruses such as SV40, CMV, Baculo virus, Adeno virus, Sindbis virus, transposons, IS-elements, phasmids, phagemides, cosmides, linear or circular DNA. For integration in mammalian cells linear DNA is typically used. Preferably, the vector type used for the present invention corresponds to the specific host cell requirements. Suitable commercially available expression vectors, into which the inventive nucleic acids may be inserted, include pSPORT, pBluescriptIISK, the baculovirus expression vector pBlueBac, and the prokaryotic expression vector pcDNAII, all of which may be obtained from Invitrogen Corp., San Diego, CA.

A vector as defined herein suitable for transfecting a cell which may be used as constituent of the (spherical) microcapsule as defined above, typically combines the GLP-1 encoding nucleic acid sequences with other regulatory elements, which e.g. control expression of the encoded (inventive) amino acid sequences. Such regulatory elements are e.g. 1) specific to a tissue or region of the body; 2) constitutive; 3) glucose responsive; and/or 4) inducible/regulatable. Regulatory elements herein are preferably selected from regulation sequences and origins of replication (if the vectors are replicated autonomously). Regulation sequences in the scope of the present invention are any elements known to a skilled person having an impact on expression on transcription and/or translation of GLP-1 encoding nucleic acid sequences. Regulation sequences include, apart from promoter sequences so-called enhancer sequences, which may lead to an increased expression due to enhanced interaction between RNA polymerase and DNA. Further regulation sequences of inventive vectors are transcriptional regulatory and translational initiation signals, so-called "terminator sequences", etc. or partial sequences thereof.

Generally, any naturally occurring promoter may be contained in an expression vector suitable for transfecting a cell which may be used for preparing the (spherical) microcapsule as used herein. Such promoters may be selected from any eukaryotic, prokaryotic, viral, bacterial, plant, human or animal, e.g. mammalian promoters. Suitable promoters include, for example, the cytomegalovirus promoter, the lacZ promoter, the gal 10 promoter and the AcMNPV polyhedral promoter, promoters such as cos-, tac-, trp-, tet-, trp-tet-, Ipp-, lac-, Ipp-lac-, laclq-, T7-, T5-, T3-, gal-, trc-, ara-, SV40-, SP6, I-PR- or the I-PL-promoter, advantageously being found in gram-negative bacteria. Additionally, promoters may be obtained from gram-positive promoters such as amy and SPO2, yeast promoters, such as ADC1, MFa, AC, P-60, CYC1, GAPDH or mammalian promoters such as the cytomegalovirus (CMV) promoter, muscle-specific promoters including mammalian muscle creatine kinase (MCK) promoter, mammalian desmin promoter, mammalian troponin I (TNNI2) promoter, or mammalian skeletal alpha-actin (ASKA) promoter, or liver type pyruvate kinase promoters, particularly those fragments which run (-183 to +12) or (-96 to +12) (Thompson, et al. J Biol Chem, (1991). 266:8679-82.; Cuif, et al., Mol Cell Biol, (1992). 12:4852-61); the spot 14 promoter (S14, -290 to +18) (Jump, et al., J. Biol Chem, (1990). 265:3474-8); acetyl-CoA carboxylase (O'Callaghan, et al., J. Biol Chem, (2001). 276:16033-9); fatty acid synthase (-600 to +65) (Rufo, et al., J Biol Chem, (2001). 28:28); and glucose-6-phosphatase (rat and human) (Schmoll, et al., FEBS Left, (1996). 383:63-6; Argaud, et al., Diabetes, (1996). 45:1563-71), or promoters from CaM-Kinasell, Nestin, L7, BDNF, NF, MBP, NSE, beta-globin, GFAP, GAP43, tyrosine hydroxylase, Kainat-receptor-subunit 1, glutamate-receptor-subunit B, or human ubiquitin promoter B (ubiB human), human ferritin H promoter (FerH), etc. Particularly preferred promoters are of human or mammalian origin. Finally, synthetic promoters may be used advantageously. Promoter sequences, as contained in an inventive vector, may also be inducible for *in vitro* control purposes, to allow modulation of expression (e.g. by the presence or absence of nutrients or other inducers in the growth medium). One example is the lac operon obtained from bacteriophage lambda plac5, which can be induced by IPTG. Finally, a promoter as defined above may be linked with a GLP-1 encoding nucleic acid sequence as defined herein such that the promoter is positioned 5' "upstream" of the GLP-1 encoding nucleic acid sequence. Preferably, human promoters are used, e.g. the human ubiquitin promoter B (ubiB human) or the human ferritin H promoter (FerH).

Enhancer sequences for upregulating expression of GLP-1 encoding nucleic acid sequences as defined herein are preferably another constituent of a vector or an expression as defined above. Such enhancer sequences are typically located in the non-coding 3' region of the vector. Enhancer sequences as employed in a vector as defined above may be obtained from any eukaryotic, prokaryotic, viral, bacterial, plant, human or animal, e.g. mammalian hosts, preferably in association with the corresponding promoters as defined above. Enhancer elements which will be most useful in the present invention are those which are glucose responsive, insulin responsive and/or liver specific. Enhancer elements may include the CMV enhancer (e.g., linked to the ubiquitin promoter (Cubi)); one or more glucose responsive elements, including the glucose responsive element (G1RE) of the liver pyruvate kinase (L-PK) promoter (-172 to -142); and modified versions with enhanced responsiveness (Cuif *et al*., supra; Lou, et al., J. Biol Chem, (1999). 274:28385-94); G1RE of L-PK with auxiliary L3 box (-172 to -126) (Diaz Guerra, et al., Mol Cell Biol, (1993). 13:7725-33; modified versions of G1RE with enhanced responsiveness with the auxiliary L3 box; carbohydrate responsive element (ChoRE) of S 14 (-1448 to -1422), and modifications activated at lower glucose concentrations (Shih and Towle, J Biol Chem, (1994). 269:9380-7; Shih, et al., J Biol Chem, (1995). 270:21991-7; and Kaytor, et al., J Biol Chem, (1997). 272:7525-31; ChoRE with adjacent accessory factor site of S 14 (-1467 to -1422) [*et al*., supra]; aldolase (+1916 to +2329)(Gregori et al., J Biol Chem, (1998). 273:25237-43; Sabourin, et al., J. Biol Chem, (1996). 271:3469-73; and fatty acid synthase (-7382 to -6970) (Rufo, *et al*., supra.), more preferably insulin responsive elements such as glucose-6-phosphatase insulin responsive element (-780 to -722) [Ayala et al., Diabetes, (1999). 48:1885-9; and liver specific enhancer elements, such as prothrombin (940 to -860) [Chow et al., J Biol Chem, (1991) 266: 18927-33; and alpha-1-microglobulin (-2945 to -2539) [Rouet et al., Biochem J, (1998). 334:577-84), Muscle-specific enhancers such as mammalian MCK enhancer, mammalian DES enhancer, and vertebrate troponin I IRE (TNI IRE, herein after referred to as FIRE) enhancer. Finally, a SV40 enhancer sequence may also be included.

Enhancer elements may further be used along with promoters as defined above for upregulating expression of GLP-1 encoding nucleic acid sequences as defined herein, e.g. such promoter/enhancer combinations include e.g. the cytomegalovirus (CMV) promoter and the CMV enhancer, the CMV enhancer linked to the ubiquitin promoter (Cubi), the group of liver-specific enhancer elements comprising human serum albumin [HSA] enhancers, human prothrombin [HPrT] enhancers, alpha-1 microglobulin [A1MB] enhancers, and intronic aldolase enhancers used in combination with their corresponding promoters, or HSA enhancers used in combination with a promoter selected from the group of a CMV promoter or an HSA promoter, enhancer elements selected from the group consisting of human prothrombin [HPrT] and alpha-1 microglobulin [A1MB] used in combination with the CMV promoter enhancer elements selected from the group consisting of human prothrombin [HPrT] and alpha-1 microglobulin [A1MB] used in combination with the alpha-1-anti trypsin promoter, etc.

Furthermore, a vector as defined above suitable for transfecting a cell which may be used as constituent of the (spherical) microcapsule as used according to the present invention, may contain transcriptional and/or translational signals, preferably transcriptional and/or translational signals recognized by an appropriate host, such as transcriptional regulatory and translational initiation signals. Transcriptional and/or translational signals may be obtained from any eukaryotic, prokaryotic, viral, bacterial, plant, preferably human or animal, e.g. mammalian hosts, preferably in association with the corresponding promoters as defined above. A wide variety of transcriptional and translational regulatory sequences may be employed therefore, depending upon the nature of the host to the extent that the host cells recognizes the transcriptional regulatory and translational initiation signals associated with a GLP-1 encoding nucleic acid sequence. The 5' region adjacent to the naturally occurring GLP-1 encoding nucleic acid sequence may be retained and employed for transcriptional and translational regulation in an inventive vector. This region typically will include those sequences involved with initiation of transcription and translation, such as the TATA box, capping sequence, CAAT sequence, and the like. Typically, this region will be at least about 150 base pairs long, more typically about 200 bp, and rarely exceeding about 1 to 2 kb.

Transcriptional initiation regulatory signals suitable for a vector as defined above may be selected that allow to control repression or activation such that expression of the GLP-1 encoding or nucleic acid sequences as defined above can be modulated. One such controllable modulation technique is the use of regulatory signals that are temperature-sensitive in order to repress or initiate expression by changing the temperature. Another controllable modulation technique is the use of regulatory signals that are sensitive to certain chemicals. These methods are preferably to be used in *in vitro* procedures, e.g. when preparing the necessary constructs. Furthermore, transcriptional initiation regulatory signals may be use herein, which allow control repression or activation of expression *in vivo* without any further means from outside the cell, e.g. to obtain a transient expression in the encapsulated cells. Such transcription and/or translational signals include e.g. transcriptional termination regulatory sequences, such as a stop signal and a polyadenylated region. Furthermore, transcriptional termination regulatory sequences may be located in the non-coding 3' region of a vector as defined above containing the GLP-1 encoding nucleic acid sequence. Suitable termination sequences can include, for example, the bovine growth hormone, SV40, lacZ, EF1 alpha and AcMNPV polyhedral polyadenylation signals.

The expression vectors suitable for transfecting a cell which may be used for preparing the (spherical) microcapsule as used according to the present invention, may also include other sequences for optimal expression of the GLP-1 encoding or nucleic acid sequences as defined herein. Such sequences include those encoding signal (peptide) sequences, i.e. which encode N-terminally located peptide sequences that provide for passage of the secreted protein into or through a membrane; which provide for stability of the expression product; and restriction enzyme recognition sequences, which provide sites for cleavage by restriction endonucleases. All of these materials are known in the art and are commercially available (see, for example, Okayama (1983), Mol. Cell. Biol., 3: 280).

As defined herein "a signal sequence" is a signal (peptide) sequence which typically comprises about 15 to 30 amino acids located at the N-terminus of the expressed GLP-1 (fusion) peptide and enables the GLP-1 peptide to be secreted, i.e. to pass through a cell membrane. Such a signal sequence may include the signal sequence normally associated with the wild type GLP-1 precursor protein (i.e., the signal sequence(s) of the full length proglucagon precursor molecule), as well as signal (peptide) sequences which are not normally associated thereto, i.e. which are heterologous to the wild type GLP-1 precursor protein (i.e., the signal sequence(s) of the full length proglucagon precursor molecule). A "signal sequence" as defined herein can be, for example, a signal peptide sequence or a leader sequence (e.g. a secretory signal (and leader) sequence). Furthermore, signal (peptide) sequences as defined herein preferably provide for cleavage of the (GLP-1) precursor peptide by a protease, e.g. a signal sequence protease. Upon cleavage of the signal sequence from the (GLP-1) precursor peptide by the protease a biologically active GLP-1 peptide as defined above is produced. Such a signal sequence generally comprises a region which encodes a cleavage site recognized by a protease for cleavage. Alternatively, a region which encodes a cleavage site recognized by a protease for cleavage can be introduced into the signal sequence. Furthermore, additional (one or more) sequences which encodes a cleavage site recognized by a protease for cleavage can be added to the signal sequence.

Examples of signal sequences which can be encoded by a vector as defined above include a signal sequence derived from a secreted protein such as GLP-1 or other than GLP-1, such as a cytokine, a clotting factor, an immunoglobulin, a secretory enzyme or a hormone (including the pituitary adenylate cyclase activating polypeptide (PACAP)/glucagon superfamily) and a serum protein. For example, a signal sequence as defined herein can be derived from secreted matrix metalloproteinases (MMP), e.g. a stromelysin leader sequence, from secreted human alkaline phosphatase (SEAP), pro-exendin, e.g. a proexendin-4 leader sequence, pro-helodermin, pro-glucose-dependent insulinotropic polypeptide (GIP), proinsulin-like growth factor (IGF1), preproglucagon, alpha-1 antitrypsin, insulin-like growth factor 1, human factor IX, human lymphotoxin A (Genbank Accession no. BAA00064), or human clusterin (Genbank Accession No. AAP88927). Particular examples of signal sequences as defined herein are sequences which include a coding region for a signal for precursor cleavage by signal peptidase, furin or other prohormone convertases (e.g., PC3). For example, a signal which is cleaved by furin (also known as PACE, see U.S. Pat. No. 5,460,950), other subtilisins (including PC2, PC1/PC3, PACE4, PC4, PC5/PC6, LPC/PC7IPC8/SPC7 and SKI-1; Nakayama, Biochem. J., 327:625-635 (1997)); enterokinase (see U.S. Pat. No. 5,270,181) or chymotrypsin can be introduced into the signal sequence as defined above. The disclosure of each of these documents is hereby incorporated herein by reference. Furin is a ubiquitously expressed protease that resides in the trans-golgi and processes protein precursors before their secretion. Furin cleaves at the COOH-terminus of its consensus recognition sequence, Arg-X-Lys-Arg or Arg-X-Arg-Arg, (Lys/Arg)-Arg-X-(Lys/Arg)-Arg and Arg-X-X-Arg, such as an Arg-Gln-Lys-Arg. These amino acid sequences are a signal for precursor cleavage by the protease furin. Thus, a heterologous signal sequence can also be synthetically derived from a consensus sequence compiled from signal sequences (e.g., a consensus sequence compiled from secreted proteins that are cleaved by signal peptidase).

Additionally to regulation sequences as defined above, an autonomously replicating vector as defined above typically comprises an origin of replication. Suitable origins of replication include, without being limited thereto, e.g. ColE1, pSC101, SV40, pMPI (ori pMPI) and M13 origins of replication, etc.

Preferably, a vector as defined above, suitable for expression of the GLP-1 encoding or nucleic acid sequences of the cells of the (spherical) microcapsules as defined above, may additionally contain a suicide gene. In the context of the present invention "a suicide gene" is preferably capable to stop the therapy with (spherical) microcapsules, as used herein, by killing the suicide gene harbouring cell contained in the core of the (spherical) microcapsule upon administering a specific substance, e.g. a suicide gene suitable for the present invention may be activated by administering an exogenous activator that typically does not occur in the human or animal body. In this case, typically the suicide gene initiates a cascade causing the cell to undergo an apoptotic event. Alternatively, a suicide gene suitable for the present invention may metabolize an administered exogenous non-toxic prodrug that typically does not occur in the human or animal body. Metabolism of the exogenous non-toxic prodrug preferably renders the prodrug to a cell toxin. The suicide gene may be contained on the same vector encoding the GLP-1 peptide as defined above or alternatively on a second vector. Furthermore, the suicide gene may be regulated by control and regulatory elements of any kind, e.g. control and regulatory elements such as promoters, enhancers, etc. as mentioned herein as constituents of expression vectors, or by their naturally occurring control and regulatory elements. Preferably, suicide genes are selected according to the present invention, which allow any of the above control mechanisms, e.g. suicide genes selected from cytosin deaminase (CD), uracil phosphoribosyl transferase (UPRTase), HSV thymidine kinase (HSV-Tk), suicide genes which may be induced by addition of tetracycline such as the bacterial Tet repressor protein (TetR), etc. As a particular example the cytosine desaminase (CD) may be used. The cytosine desaminase (CD) typically occurs in a variety of organisms and is capable of transforming 5-fluorocytosin (5-FC) into 5-fluorouracil (5-FU), which represents a common chemotherapeutic agent. 5-Fluorouracil (5-FU) is highly toxic for the organism whereas its prodrug 5-fluorocytosin (5-FC) is not toxic to cells. 5-Fluorouracil (5-FU) is subsequently phosphorylated by cellular kinases and is capable of abrogating the cells RNA synthesis. Thus, the prodrug 5-fluorocytosin (5-FC) represents an excellent tool for inducing suicide of a specific cell. Furthermore, 5-Fluoro-dUMP acts as antifolate agent and inhibits the enzyme thymidylat synthase, which catalyses methylation of dUMP to dTMP in the *de novo* synthesis path of desoxyribonukleotides. Thereby, inhibition of DNA synthesis in the cell may be inhibited. Also preferably, the HSV-1 thymidin kinase (ATP: Thymidin-5-phosphotransferase) and its corresponding prodrug ganciclovir (GCV) may be used. The guanosin analog GCV is specifically phosphorylated and inhibits elongation of DNA synthesis and thus leads to suicide of the cell.

Transfection of the vectors as defined above or, alternatively, of naked GLP-1 peptide encoding nucleic acids into suitable cells used for preparation of (spherical) microcapsules as defined above, may be accomplished by any method known to a skilled person (see e.g. Maniatis et al. (2001) Molecular Cloning: A laboratory manual, Cold Spring Harbor Laboratory Press, Cold Spring Harbor, NY). If vectors are transfected into suitable cells as defined above, the vector is preferably present in the form of a plasmid DNA, which carries a GLP-1 peptide encoding nucleic acid. The plasmid DNA is preferably a circular plasmid DNA. Suitable transfection methods include, without being limited thereto, e.g. electroporation techniques including modified electroporation techniques (e.g. nucleofection), calcium phosphate techniques, e.g. the calcium phosphate co-precipitation method, the DEAE-Dextran method, the lipofection method, e.g. the transferring-mediated lipofection method, etc. Preferably, transfection is carried out with plasmid DNA carrying a vector as defined above using a modified electroporation technique (e.g. nucleofection).

### Method(s) for preparing the (spherical) microcapsules

The present invention also provides a method for preparing the (spherical) microcapsules as used according to the present invention. These (spherical) microcapsules are preferably prepared according to two or more method steps. According to a method step 1) a core is prepared as disclosed above. According to a method step 2) the core as prepared according to method step 1) is encased by a surface coating. Further optional steps may comprise repetition of method step 2) for the preparation of additional surface coatings. Preferably, a step identical to method step 2) is carried out for each of such additional surface coatings. Further optional steps may include washing steps subsequent to preparation of the spherical microcapsule.

Typically, a core as disclosed above is prepared according to method step 1) for preparing (spherical) microcapsules, as used according to the present invention. Such a core is composed of cross-linked polymer and GLP-1 encoding and secreting cells, which have been transfected according to a method as disclosed above. According to method step 1), a mixture (suspension) of the soluble form of the polymer, e.g. the soluble form of an alginate (e.g. potassium or sodium alginate in physiological saline solution), and of GLP-1-peptide encoding and secreting cells is typically prepared, preferably in a concentration as defined above for the (spherical) core, e.g. of 1 x 10⁵ up to 6 x 10⁷cells, per ml polymer solution.

As a typical technique the homogenic cell/polymer suspension (e.g. cell/alginate suspension) is pressed *via* an air injected spray nozzle, consisting of three channels, which are arranged concentrically as three concentric rings around a common centre: an inner channel, an intermediate channel and an outer channel (air ring). Preferably hollow needles are used for the inner channel having an inner diameter of 50 µm up to 2,000 µm. The intermediate channel typically has an inner diameter of 60 µm to 4,000 µm, and the outer channel (air ring) preferably has an inner diameter of 100 µm to 5,000 µm. Exclusively the inner channel and the outer channel (air ring) are used in method step 1) for preparing the core of the (spherical) microcapsule, as used according to the present invention. Thus, a spray nozzle merely consisting of two channels (an inner and an outer channel) may be used in method step 1) as well. Typically, no material flows through the intermediate channel, if an air injected spray nozzle with three channels is used. The suspension of the cell/polymer solution is typically pressed with a speed of 10 µl/min to 5ml/min through the inner channel leading to droplets at the outlet of the channel, which tear off due to the air flow provided by the outer channel (air ring), having a speed of typically 0.5 l/min to 10 l/min. Droplets containing cells and non-cross-linked polymer solution fall down into a cross-linker containing solution (precipitation bath), which is typically positioned in a distance of about 4 cm to about 60 cm under the outlet of the air injected spray nozzle. The droplet preferably rounds during dropping down, thereby receiving a substantially spherical geometrical form. The cross-linker effects ionical cross-linking of the polymers and the core of the spherical (water insoluble) microcapsule is initially formed having a diameter as defined above for the (spherical) core. The diameter of the core of the (spherical) microcapsule is dependent on size and geometry of the chosen channels used in method step 1). The cross-linker containing solution (precipitation bath) is preferably composed of bivalent cations, e.g. calcium or barium ions (5-100 mM) or other bivalent or multivalent cations, if alginates are used as polymers. Furthermore, the precipitation bath preferably contains a buffer substance (e.g. 1mM - 10mM histidine) and sodium chloride (e.g. 290 mOsmol ± 50 mOsmol). Other suitable cross-linkers and buffers known in the art may be used herein, if other polymers than alginates are used.

Method step 1) provides the core of the (spherical) microcapsule composed of cross-linked polymers and cells as defined above. Subsequent to method step 1) optional method step(s) may include a washing step. The core of the (spherical) microcapsule, as used according to the present invention, is e.g. washed with a physiological saline solution or any other suitable washing solution and, if applicable, the core is incubated in a sodium sulfate solution, preferably in a sodium sulfate solution according to US 6,592,886, the disclosure of which is incorporated herein by reference. Separation of the cores of the (spherical) microcapsules, as used according to the present invention, from the precipitation bath and/or the washing bath is typically is carried out using a centrifuge or any other suitable method.

According to method step 2) the core of the (spherical) microcapsule, as used according to the present invention, prepared by method step 1) is coated with a surface coating substantially of cross-linked polymer. Accordingly, the core of the (spherical) microcapsule, prepared by step 1), is added to a polymer solution containing non-crosslinked polymers as disclosed above comprising no cells. Preferably, the polymers are provided in their non-cross-linked form in a concentration as defined above. Typically, this mixture containing the polymer solution and the core of the (spherical) microcapsule is pressed through the inner channel of the above-described air injected spray nozzle, e.g. with a speed of 15 µl/min to 2 ml/min, preferably 10 µl/min to 5 ml/min. Simultaneously, a pure non-cross-linked polymer solution without cells, preferably a solution comprising about 0.1% to about 4% (w/v) polymer, e.g. an alginate solution without any cells, is pressed through the intermediate channel with a speed of typically 15 µl/min to 2 ml/min, preferably 10 µl/min to 5 ml/min. Thereby, droplets are formed at the end of the intermediate channel, containing the core and a surface of non-polymerized polymer. These droplets tear off due to the air flow provided *via* the outer channel (air ring) having a speed of typically 0.5 l/min to 10 l/min. The polymer concentration of the core of the (spherical) microcapsule, the polymer solution, into which the core of the (spherical) microcapsules is added, and the polymer concentration of the surface coating may differ (see above). The droplets containing the core of the (spherical) microcapsules (prepared according to method step 2) fall into a solution containing the cross-linker (precipitation bath) as defined above. During dropping down, the droplet preferably rounds to an approximately spherical geometrical form. The cross-linker affects an ionic cross-linkage of the polymers analogous to method step 1). Thereby, water insoluble (spherical) microcapsules are formed having a diameter as defined above, preferably of total diameter (particle size) of the (spherical) microcapsule of about 100 µm to about 200 µm, more preferably a total diameter of about 115 µm to about 185 µm, even more preferably a total diameter of about 130 µm to about 1 70 µm, and most preferably a total diameter of about 145 µm to about 155 µm, e.g. about 150 µm. The total diameter of (spherical) microcapsules obtainable by method step 2) is dependent from size and geometry of the chosen channels, as used herein. In order to prepare (spherical) microcapsules as defined above, with more than one surface coating, i.e. the (spherical) microcapsules containing the core as defined above and 2, 3, 4, 5, 5-10 or more surface coatings, method step 2) may be repeated as often as necessary. Those further surface coatings are defined within the above diameter ranges.

Subsequent to method step 2) one or more optional washing steps may follow as defined above.

### Method of treatment and uses of the cells and (spherical) microcapsules

According to a further aspect of the invention a method of treatment of AMI or MI in an animal, preferably a mammal such as human being, is provided by administration of cells, e.g. mesenchymal stem cells or mesenchymal stromal cells, or any further cell, that may be used in the context of the present invention, encoding and secreting GLP-1, a fragment or variant thereof or a fusion peptide comprising GLP-1 or a fragment or variant thereof, wherein these cells are encapsulated in a (spherical) microcapsule as defined above to prevent a response of the immune system of the patient to be treated. Preferably, the (spherical) microcapsule as well as all its components as used in the inventive method, e.g., polymers of the polymer matrix of the core or the surface coating, etc., is as defined above. The method of treatment according to the invention can be used for human and also for veterinary medical purposes.

Treatment in the context of the present invention preferably comprises treatment or prevention of ischemic heart disease or acute coronary syndromes and the treatment or prevention of conditions associated therewith. Non-limiting examples of such diseases or conditions include AMI or MI, ST elevation MI (STEMI), cardiomyopathy (including ischemic cardiomyopathy), unstable angina, congestive heart failure and ventricular dysfunction, heart failure, endothelium dysfunction disorders, optionally hypertension, or any diseases or conditions related thereto.

A method of treatment or prevention of AMI or MI as defined above including treatment or prevention of a disease or condition associated with AMI or MI furthermore comprises administering the cells, encapsulated in a (spherical) microcapsule as defined herein or the (spherical) microcapsule as defined herein, or administering the pharmaceutical composition containing such (spherical) microcapsule, to a patient in need thereof. A patient in need thereof is typically a, e.g., an animal, preferably a mammal, such as a human being. Administration in the context of the above method of treatment typically occurs in a "safe and effective" amount of the active agent, i.e. the cells, encapsulated in a (spherical) microcapsule as defined herein, or the (spherical) microcapsule as defined herein. As used herein, "safe and effective amount" means an amount of these cells, encapsulated in a (spherical) microcapsule as defined herein, or the (spherical) microcapsule as defined herein, that is sufficient to significantly induce a positive modification of a disease or disorder as mentioned herein. At the same time, however, a "safe and effective amount" is small enough to avoid serious side-effects that is to say to permit a sensible relationship between advantage and risk. The determination of these limits typically lies within the scope of sensible medical judgment. In the context of the present invention the expression "safe and effective amount" preferably means an amount of the cells, encapsulated in a (spherical) microcapsule as defined herein, or the (spherical) microcapsule as defined herein that is suitable to exert beneficial effects known for GLP-1, e.g. its activity to powerfully reduce the damages caused by ischemia or oxygen shortage and potential death of heart tissue without the need of repeated administration of GLP-1 peptide(s) and/or the risk of an undesired immune response against e.g. implanted GLP-1 expressing allogenic cells. A "safe and effective amount" of the cells, encapsulated in a (spherical) microcapsule as defined herein, or the (spherical) microcapsule as defined herein, will furthermore vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the administering doctor.

Typically, (spherical) microcapsules as contained in the inventive pharmaceutical composition secrete about 0.2 µg GLP-1 peptide as defined herein per day per ml of (spherical) microcapsules. Thus, a dosage range may be e.g. in the range from about 0.01 µg to 20 mg of secreted biologically active GLP-1 peptide per day (even though higher amounts in the range of 1-100 mg are also contemplated), such as in the range from about 0.01 µg to 10 mg per day, preferably in the range from 0.01 µg to 5 mg per day and even more preferably in the range from about 0.01 µg to 1 mg per day and most preferably in the range from about 0.01 µg to 500 µg per day.

Administration in the context of the above method of treatment typically occurs by providing the cells, encapsulated in a (spherical) microcapsule as defined herein, or the (spherical) microcapsule as defined herein, or the pharmaceutical composition containing such (spherical) microcapsule, into a specific administration site in the patient to be treated.

Such a specific administration site is typically the heart muscle or tissue, the myocardium or myocardial tissue, particularly the infarct area, the surrounding area and/or the periinfarct zone, e.g. all as detected by conventional means in the art such as electrocardiography or NOGA electromechanical mapping or MRI. Administration sites also include arterioles feeding the heart muscle or tissue, arterioles feeding the myocardium or myocardial tissue, particularly arterioles feeding the infarct area, the surrounding area and/or the periinfarct zone, etc. Administration sites furthermore include any surface of the heart muscle or tissue, particularly of the infarct area, the surrounding area and/or the periinfarct zone, which may be treated, without being limited thereto, by epicardial injection after or during open chest surgery, where infarct may be discriminated by eye. Following infarct, the tissue rapidly becomes jelly-like and loses physical integrity. Injection of the cells, encapsulated in a (spherical) microcapsule as defined herein, or the (spherical) microcapsule as defined herein, or the pharmaceutical composition containing such (spherical) microcapsule, may occur also into this liquefied jelly-like area of the infarct or in any further region as defined above.

Administration of the cells, encapsulated in a (spherical) microcapsule as defined herein, or the (spherical) microcapsule as defined herein, or the pharmaceutical composition containing such (spherical) microcapsule, into a specific administration site as defined above may be carried out using different modes of administration. The cells, encapsulated in a (spherical) microcapsule as defined herein, or the (spherical) microcapsule as defined herein, or the pharmaceutical composition containing such (spherical) microcapsule, can be administered, for example, systemically or locally. Routes for systemic administration in general include, for example, transdermal or parenteral routes, including intravenous, subcutaneous, and/or intraarterial injections. Routes for local administration in general include, e.g., topical administration routes but also transdermal, intramuscular, subcutaneous, intracardial, intramyocardial and/or pericardial injections. More preferably, the cells, encapsulated in a (spherical) microcapsule as defined herein, or the (spherical) microcapsule as defined herein, or the pharmaceutical composition containing such (spherical) microcapsule, may be administered by an intradermal, subcutaneous, or intramuscular route, more preferably by an intracardial, intramyocardial and/or pericardial injection.

Other modes of administration, which may be suitable for treatment of any of the afore mentioned diseases or disorders, include transplantation of the cells as defined herein, encoding and secreting GLP-1, a fragment or variant thereof or a fusion peptide comprising GLP-1 or a fragment or variant thereof, wherein these cells, are encapsulated in a (spherical) microcapsule as defined above, or of (spherical) microcapsules as defined herein, (preferably formulated in a suitable form, e.g. by addition of suitable pharmaceutical carriers, e.g. in the form of gels, capsules, tablets, etc.). Cells encapsulated in a (spherical) microcapsule as defined herein, may be directly delivered to the affected site of the heart (an administration site as defined above) by interventional means, e.g. using a catheter to navigate to the affected area and implant the beads by injection into the myocardial tissue. Implantation could be performed during routine angioplasty post AMI. Implantation may also occur into the affected area of myocardium of a mammalian AMI or post AMI patient by direct injection into the heart tissue, or by intravascular delivery through the arterioles feeding the affected heart tissue.

Without being limited thereto, the cells as defined herein, encoding and secreting GLP-1, a fragment or variant thereof or a fusion peptide comprising GLP-1 or a fragment or variant thereof, wherein these cells, are encapsulated in a (spherical) microcapsule as defined above, or the (spherical) microcapsules as defined herein may be administered e.g. *via* injection by applying an appropriate injection needle such as injection needles having a size of from 12 to 26 G, more preferably of from 18 to 22 G or e.g. by transplanting the cells or the (spherical) microcapsules as defined herein, preferably formulated in a suitable form, using surgical devices, such as scalpels, injection needles as defined above, etc. According to a particular example, which shall not be regarded as limiting to the present embodiment, a patient in need thereof, suffering from AMI or MI or any disease associated thereto or disclosed herein may receive a intramuscular injection or implantation of the cells or the (spherical) microcapsules as defined herein into a site of administration as defined above, etc.

Treating or preventing AMI or MI diseases and disorders as defined above using (spherical) microcapsules as defined above having cells embedded in its core encoding and secreting GLP-1 or such cells or a pharmaceutical composition comprising these (spherical) microcapsules) preferably results from the beneficial effects of GLP-1, e.g. its activity to (powerfully) reduce the damages caused by ischemia or oxygen shortage and potential death of heart tissue. Such beneficial effects include e.g. improved regional and global LV function in patients with AMI and severe systolic dysfunction after successful primary angioplasty, etc. The *in situ* cardioprotective effect of (spherical) microcapsules encoding and secreting GLP-1 is particularly due to the local secretion of a glucagon-like peptide-1 (GLP-1) fusion protein and other paracrine factors that are continuously delivered to the site of injury.

According to the knowledge of the present inventors, without being bound thereto, the *in situ* cardioprotective effect of (spherical) microcapsules encoding and secreting GLP-1 is at least in part based on the fact that the present invention successfully utilizes properties of GLP-1 which have the potential for it to exert a direct cardioprotective effect. In this regard and in addition to its incretin actions, GLP-1 has been shown to reduce pancreatic beta-cell apoptosis. The localization of the GLP-1 receptor in the heart and the demonstration that GLP-1 promotes the activity of PI3K in beta-cells (a kinase that has been clearly associated with myocardial protection in the setting of ischemic/reperfusion injury as well as preconditioning), and thus allows one to hypothesize a novel and independent action of GLP-1 in the setting of ischemia/reperfusion. GLP-1 additionally induces an increased level of cAMP in cardiomyocytes, which, in turn, activates protein kinase A. GLP-1 has an antiapoptotic action on insulin-secreting cells mediated by cAMP and PI3K. Activation of PI3K leads to the phosphorylation and inactivation of the proapoptotic peptide BAD by causing it to bind to 14-3-3 proteins. BAD is a proapoptotic member of the Bcl-2 family that can displace Bax from binding to Bcl-2 and Bcl-xl, resulting in cell death. Western blot results of the investigators confirmed phosphorylation of BAD at serine 136 by GLP-1. Therefore, it appears as a surprising result, that GLP-1 has a direct antiapoptotic effect on cardiac muscle in model used. Furthermore, elevated levels of cAMP have previously been thought to be detrimental in ischemic cardiomyocytes. Nevertheless, the amount of cAMP produced may play a role in determining divergent signalling pathways that lead to antiapoptotic pathways. The cAMP produced may also be located in particular microdomains, described as compartmentalization, that restrict its actions. GLP-1-mediated increases in cAMP (comparable to isoproterenol) failed to cause any inotropic or lusitropic effect, supporting the suggestion for such compartmentalization. Compartmentalization of G protein-coupled signalling has been the subject of numerous reports, and it is increasingly recognized that spatiotemporal regulation of protein kinase A activity involves regulation of discrete cAMP pools. GLP-1 has also been shown to increase blood pressure and heart rate in rats, although the prior art failed to demonstrate any hemodynamic changes. This may be due to the differences in dose, method of delivery, or species. GLP-1 has been shown to have effects on the central control of blood pressure and pulse. Additionally, recombinant GLP-1 has previously been shown in a porcine model of myocardial ischemia to prevent the accumulation of pyruvate and lactate but failed to show any decrease in the infarction. Nevertheless, the inventors of the present invention surprisingly could demonstrate myocardial protection by GLP-1 or a fusion peptide of GLP-1 as shown herein. In conclusion, the inventors of the present invention have surprisingly found for the first time a cardioprotective effect of GLP-1 in pig heart, and, furthermore, a new insight into this possible therapeutic potential for GLP-1 agonists, a class of drugs currently undergoing trials in the treatment of type 2 diabetes, wherein no immunoreactions are exhibited by administering a (spherical) microcapsule as defined herein.

The invention furthermore encompasses use of cells as defined herein, encoding and secreting GLP-1, a fragment or variant thereof or a fusion peptide comprising GLP-1 or a fragment or variant thereof, wherein these cells, are encapsulated in a (spherical) microcapsule as defined above, for the manufacture of a product, e.g. a pharmaceutical composition or a kit, for the treatment of AMI or MI in an animal, preferably a mammal, such as a human being. The cells as used in such a treatment may be cells as defined above, e.g. mesenchymal stem cells or mesenchymal stromal cells, or any further cell, that may be used in the context of the present invention, encoding and secreting GLP-1, a fragment or variant thereof or a fusion peptide comprising GLP-1 or a fragment or variant thereof, wherein these cells, are encapsulated in a (spherical) microcapsule to prevent a response of the immune system of the patient to be treated.

Another aspect of the present invention is a pharmaceutical composition containing cells as defined herein, encoding and secreting GLP-1, a fragment or variant thereof or a fusion peptide comprising GLP-1 or a fragment or variant thereof, wherein these cells, are encapsulated in a (spherical) microcapsule as defined above, or containing (spherical) microcapsules as defined herein. Such a pharmaceutical composition may be applied to a patient suffering from the above disorders, preferably to the administration sites as defined above in a mode as defined above.

Preparation of pharmaceutical compositions which contain cells as defined herein, encoding and secreting GLP-1, a fragment or variant thereof or a fusion peptide comprising GLP-1 or a fragment or variant thereof, wherein these cells, are encapsulated in a (spherical) microcapsule as defined above, or containing (spherical) microcapsules as defined herein, as an "active ingredient", is generally well understood in the art, as e.g. exemplified by US Patents 4,608,251; 4,601,903; 4,599,231; 4,599,230; 4,596,792; and 4,578,770, all incorporated herein by reference.

Typically, pharmaceutical compositions are prepared as injectables either as liquid solutions or suspensions, preferably containing water (aqueous formulation) or may be emulsified. The term "aqueous formulation" is defined as a formulation comprising at least 50 % w/w water. Likewise, the term "aqueous solution" is defined as a solution comprising at least 50% w/w water, and the term "aqueous suspension" is defined as a suspension comprising at least 50 % w/w water.

For intramuscular, intravenous, cutaneous or subcutaneous injection, or any further injection at the site of affliction as defined above, the cells or (spherical) microcapsules as defined above will be in the form of a parenterally acceptable aqueous solution which is pyrogen-free and has suitable pH, isotonicity and stability. Liquid pharmaceutical compositions generally include a liquid vehicle such as water. Preferably, the liquid vehicle will include a physiological saline solution, dextrose ethanol or other saccharide solution or glycols such as ethylene glycol, propylene glycol or polyethylene glycol or combinations thereof may be included. Further examples include other isotonic vehicles such as physiological salt solutions, e.g. Ringers solution or Lactated Ringer's solution.

If the inventive pharmaceutical composition comprises an aqueous solution of cells or (spherical) microcapsules as defined above, and e.g. a buffer, said (spherical) microcapsule is typically present in the pharmaceutical composition in a concentration from 0.1 mg/ml or above, and said pharmaceutical composition usually has a pH from about 2.0 to about 10.0, preferably from about 7.0 to about 8.5.

It is possible that other ingredients may be present in the inventive pharmaceutical composition. Such additional ingredients may include wetting agents, emulsifiers, antioxidants, bulking agents, pH buffering agents (e.g. phosphate or citrate or maleate buffers), preservatives, surfactants, stabilizers, tonicity modifiers, cheating agents, metal ions, oleaginous vehicles, proteins (e.g. human serum albumin, gelatin or proteins) and/or a zwitterion (e.g. an amino acid such as betaine, taurine, arginine, glycine, lysine and histidine). Such ingredients are selected by a skilled person according to the specific requirements of the cells embedded in the core of the (spherical) microcapsule, as used according to the present invention, i.e. the ingredients are not cytotoxic and ensure *via*bility of the cells. Furthermore, such ingredients may stabilize GLP-1 peptides already encoded and secreted by the cells embedded in the core of the (spherical) microcapsule, as used according to the present invention.

With regard to buffers these are preferably selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethane, hepes, bicine, tricine, malic acid, succinate, maleic acid, fumaric acid, tartaric acid, aspartic acid or mixtures thereof. Each one of these specific buffers constitutes an alternative embodiment of the invention.

The use of all of the afore-mentioned additives in pharmaceutical compositions containing cells as defined herein and/or the (spherical) microcapsule as used according to the present invention, is well-known to the skilled person, in particular with regard to concentration ranges of the same. For convenience reference is made to Remington: The Science and Practice of Pharmacy, 19th edition, 1995.

Inventive pharmaceutical compositions containing cells, encoding and secreting GLP-1 as defined herein, and/or (spherical) microcapsules as defined above, are preferably administered in a manner as defined above for treatments in general. Such administrations are preferably compatible with the dosage formulation, and comprise preferably a safe and effective amount of the active ingredients as defined above, i.e. such amount which is regarded as safe but therapeutically effective. The quantity of cells, encoding and secreting GLP-1 as defined herein and/or (spherical) microcapsules as defined above, to be administered with an inventive pharmaceutical composition (or, if required, alone), depends on the subject and the disease to be treated, including, e.g., the severity of the patient's disease. Suitable dosage ranges depend on the amount of biologically active GLP-1 peptide secreted by the (spherical) microcapsules (as contained in the inventive pharmaceutical composition) during a predetermined time period and typically range in the order of one to several hundred micrograms (GLP-1) per day as defined above.

The present invention may furthermore comprise combinations of the above described embodiments and features if not described otherwise and is not intended to be limited to these particularly defined single embodiments.

The invention is illustrated further in the accompanying Figures. However, it is not intended to limit the scope of the invention to the content of the Figures as shown in the following.

### Description of Figures:

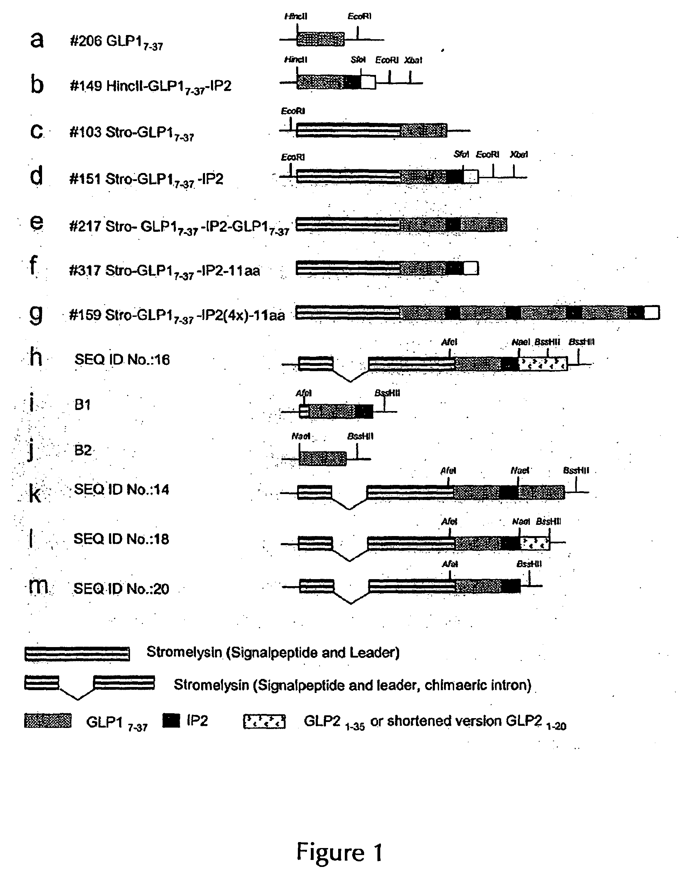

- Fig. 1:: shows a non-limiting overview over exemplary constructs a - m (see also Example 1), which may be contained in cells used for preparation of the (spherical) microcapsules, as used according to the present invention.

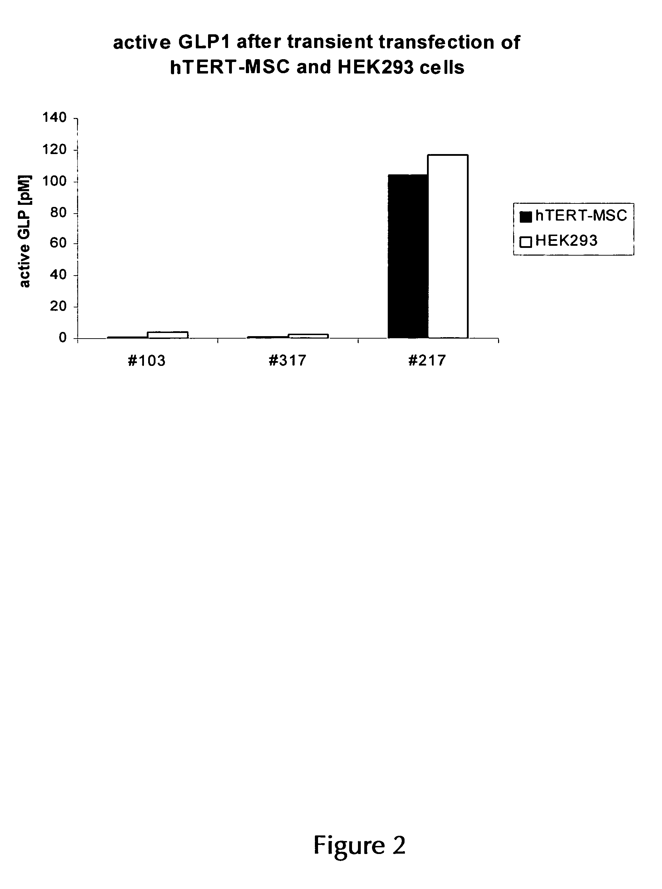

- Fig. 2:: depicts the results of transient expression of different GLP-1 constructs in hTERT-MSC and HEK293 cells and of active GLP-1 after transient transfection (see also Example 2). Only marginal active GLP-1 levels can be found in the monomeric GLP-1 constructs #103 and #317 (having just one copy of GLP-1(7-37)). An enormous gain in expression was observed in the dimeric GLP-1 construct #217 (having GLP-1 (7-37) as component (I) and as component (III)) both in hTERT-MSC and in HEK293 cells.

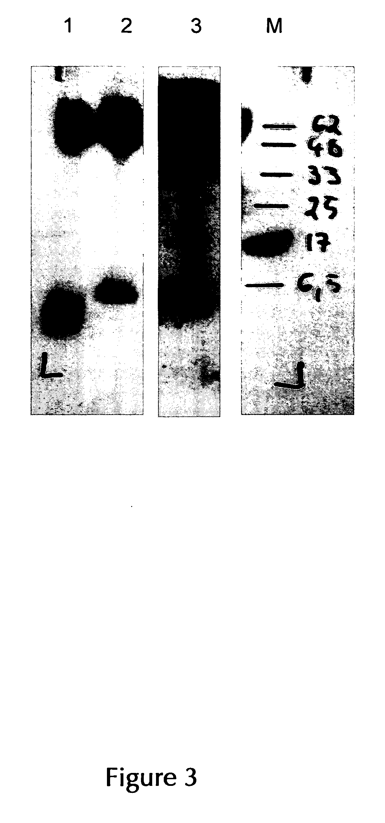

- Fig. 3:: shows a Western Blot Analysis of a cell culture supernatant from GLP-1 secreting cells (see also Example 3). Lane 1: 100 ng synthetic GLP-1(7-37) dissolved in supernatant of mock transfected hTERT-MSC cells; Lane 2: supernatant of hTERT-MSC cells (clone 79TM217/13) secreting dimeric GLP-1 from construct #217; Lane 3: supernatant of AtT20 cells (clone 81-A-217/3) secreting dimeric GLP-1 from construct #217; Lane M: prestained protein marker [kDa]). The results show that peptides as defined herein containing GLP-1(7-37) and a C-terminal appendix (2 and 3 in Fig. 3) are secreted from the transfected cell lines and can be detected using an anti-GLP-1 antibody, which binds to the mid-molecular epitopes of GLP-1 (7-37).

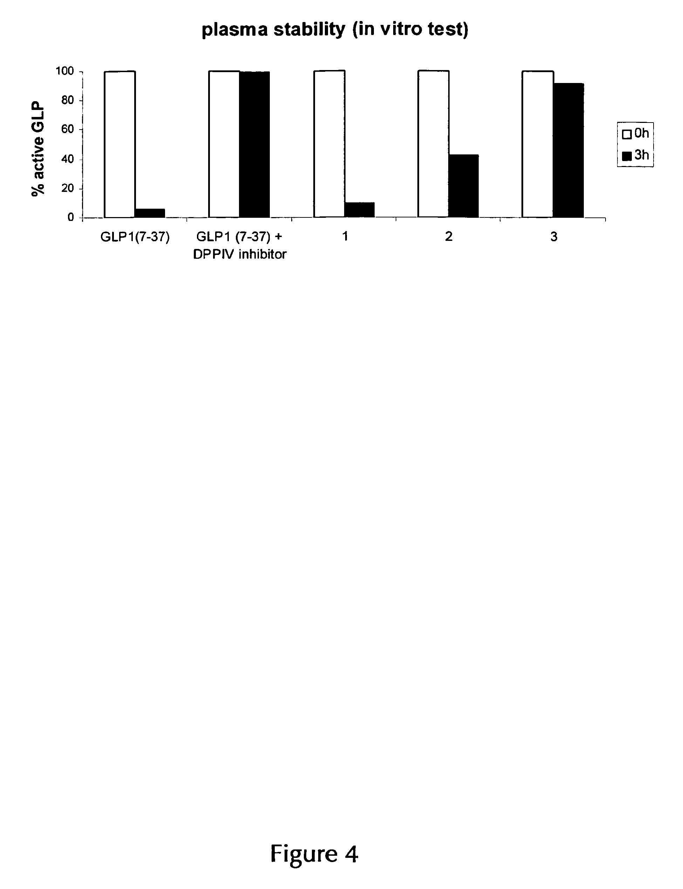

- Fig. 4:: describes plasma stability tests (*in vitro*) carried out with GLP-1 peptides as used according to the present invention. Therefore, HEK293 cells were transiently transfected with constructs (1) #103 GLP-1 (7-37), (2) #317 GLP-1(7-37)-IP2-extended with 11 AA and (3) #217 GLP-1(7-37)-IP2-GLP-1(7-37). HEK293 cells are effective hosts for the gene construct (see also Example 4).

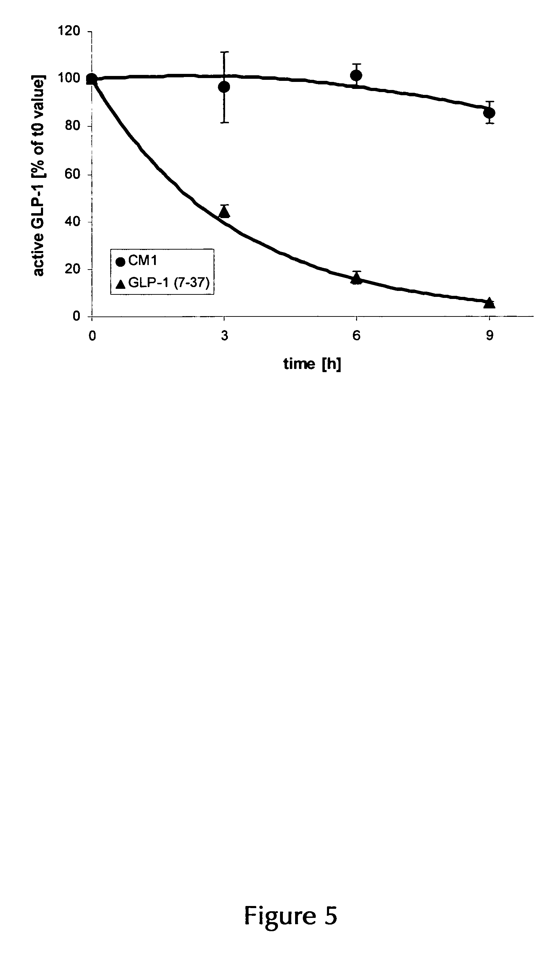

- Fig. 5:: describes a plasma stability kinetic (*in vitro*) carried out with supernatant of stably transfected hTERT-MSC cell clone 79TM217/18K5 secreting GLP-1 peptide CM1 produced by construct #217 GLP-1(7-37)-IP2-GLP-1(7-37) and synthetic GLP-1(7-37) as control. The results are obtained from three independent experiments. Active GLP-1 was measured using the GLP-1 (active) ELISA (Linco).

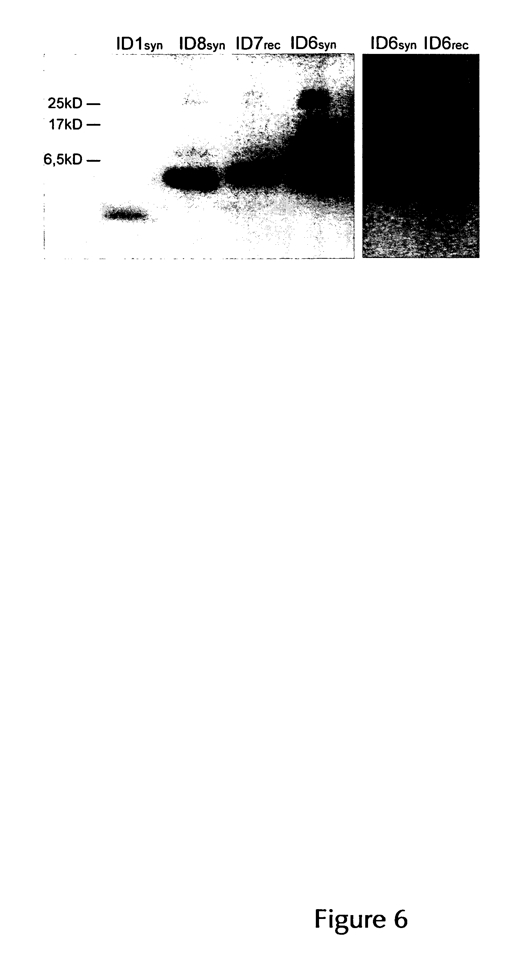

- Fig. 6:: shows a Western Blot for the peptides indicated below. The following values are given: SEQ ID NO: 1 (ID1syn) corresponds to GLP-1 (7-37), 31 aa, 3,3 kD; SEQ ID NO:8 (ID8 syn, CM3) corresponds to GLP-1 (7-37)-IP2, 46 aa, 5,1 kD; SEQ ID NO: 7 (ID7rec, CM2) corresponds to GLP-1(7-37)-IP2-RR-GLP2, 83 aa, 9,4 kD; SEQ ID NO: 6 (ID6syn, CM1) corresponds to GLP-1(7-37)-IP2-RR-GLP1(7-37), 79 aa, 8,7 kD (see also Example 5).

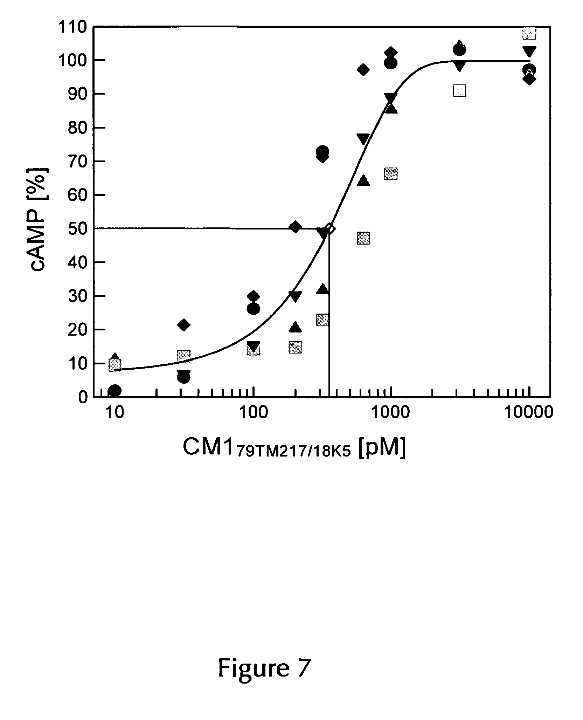

- Fig. 7:: illustrates dose response curves for GLP-1 receptor mediated cAMP increase in the bioassay cell line 111CHO349/18. Stimulation was done with serially diluted conditioned medium of 79TM217/18K5 cells secreting CM1 produced by construct #217 GLP-1 (7-37)-IP2-GLP-1 (7-37). No detectable cAMP response was found in the parental hMSC-TERT cell line. The graph was prepared from five independent experiments. The peptide dose that produces a half maximal effect (ED50) in the cAMP bioassay has been determined to be 353 pM (see also Example 6).

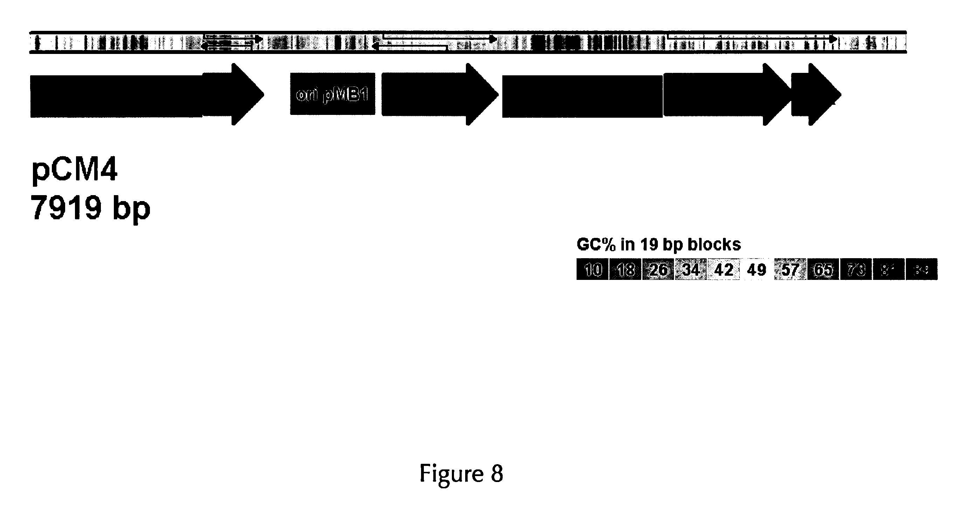

- Fig. 8:: depicts an exemplary vector used for transient and stable gene expression. The vector consists of two separate transcription units, one for the gene of interest (GOI) and one for the fusion of the suicide gene HSV thymidine kinase and the resistance gene blasticidin. For the first transcription unit, the human ubiquitin B promoter was used, and for the second transcription unit the human ferritin promoter was used (see also Example 9).

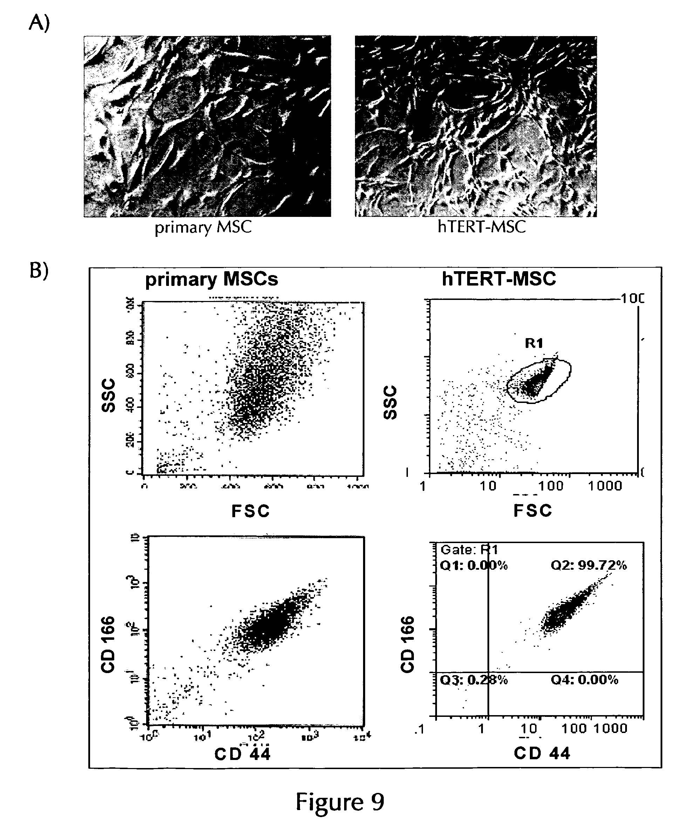

- Fig. 9:: illustrates characterization of cells used for (spherical) microcapsules as defined herein, after immortalising the cells in advance. As may be seen from Fig. 9 A, immortalised cells are still able to differentiate into adipocytes, osteocytes and chondrocytes as their non-immortalised counterparts (left, right). Immortalised cells have fibroblastic morphology and are more homogeneous regarding size and granularity as the mortal MSCs as shown by flow cytometry e.g. using CD 44 and CD166 epitope markers which are characteristic for the primary cells used here. Immortalised cells express the same CD markers as their non immortalised counterparts (see Figure 9B).

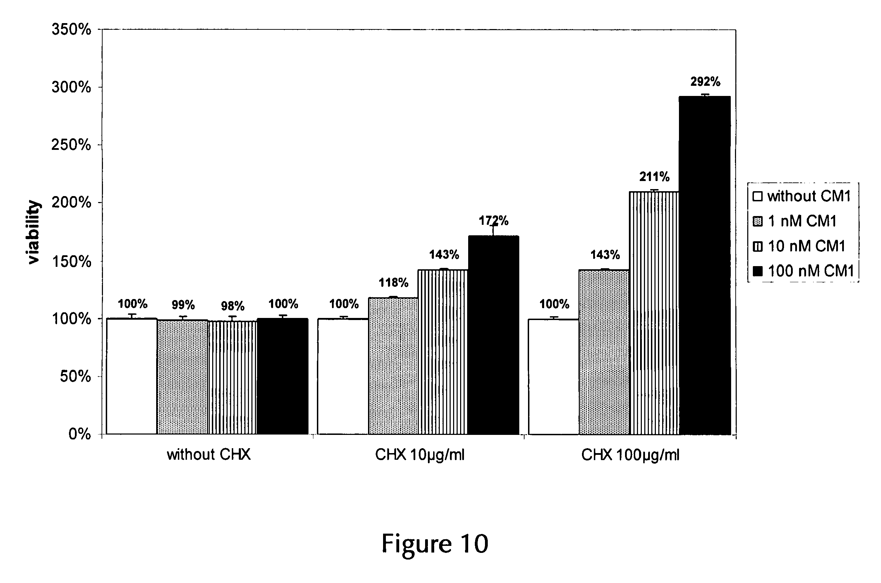

- Figs. 10:: shows the anti-apoptotic efficacy of the C-terminal elongated GLP-1 analogue CM1. Apoptosis is induced in RIN-5F cells by addition of the protein biosynthesis inhibitor cycloheximid (CHX) in a final of 10µg/ml and 100µg/ml respectively. The presence of different concentrations of the recombinantly in E. coli produced dimeric GLP-1 fusion protein CM1 result in an significant (p<0.01) increase of cell viability, which is quantified after an incubation period of 24 hours.

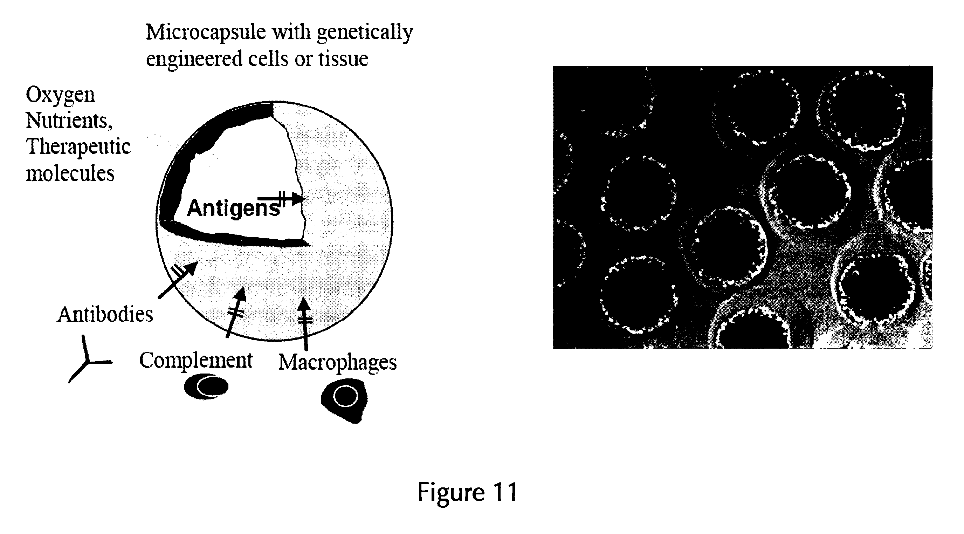

- Fig. 11:: is a schematic diagram of the inventive concept using (spherical) microcapsules encoding and secreting GLP-1 as utilized in the treatment of AMI and MI. Cells, e.g. mesenchymal stem cells, mesenchymal stromal cells or allogeneic cells are encapsulated in a thin selectively permeable alginate matrix forming (spherical) microcapsules encoding and secreting GLP-1. The alginate matrix is permeable for oxygen and nutrients supplying the encapsulated cells, as well as for GLP-1 or the GLP-1 fusion protein encoded and secreted by the cells. On the other hand, cells and components of the immune system cannot pass this barrier as depicted above. *Left:* schematic diagram of the inventive concept using (spherical) microcapsules encoding and secreting GLP-1. The cell containing corebead (cream-coloured) is surrounded by a layer of pure alginate (grey) *Right_{:}* (spherical) microcapsules encoding and secreting GLP-1 *in vitro.*

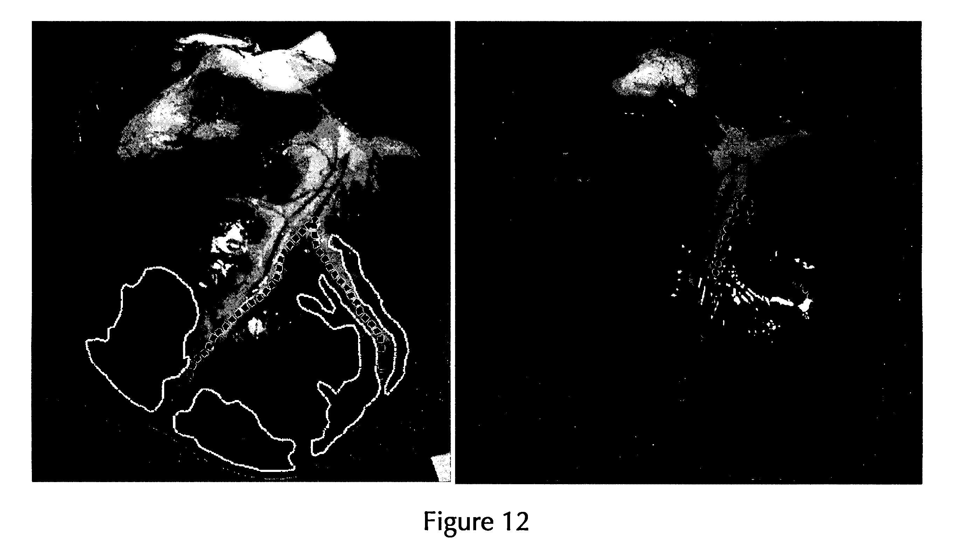

- Fig. 12: shows a gross pathology of a heart in which coronary arteries have been embolised (black dotted lines) with alginate beads (A) or inventive (spherical) microcapsules (CellBeads) (B). The patches outlines in white on (A) represent areas of visible infarction on the surface of the heart, whereas very little infarcted area is visible in (B).

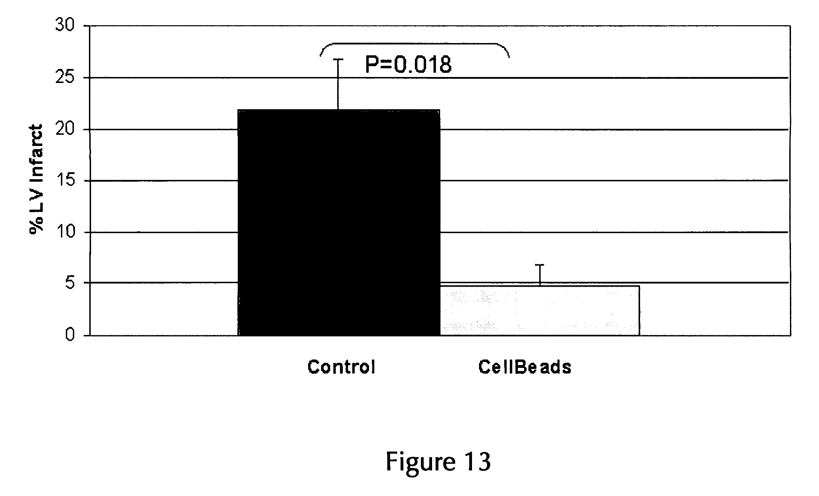

- Fig. 13:: shows the % of the LV infarct area on the surface of the hearts embolised with alginate control *versus* inventive (spherical) microcapsules (CellBeads).

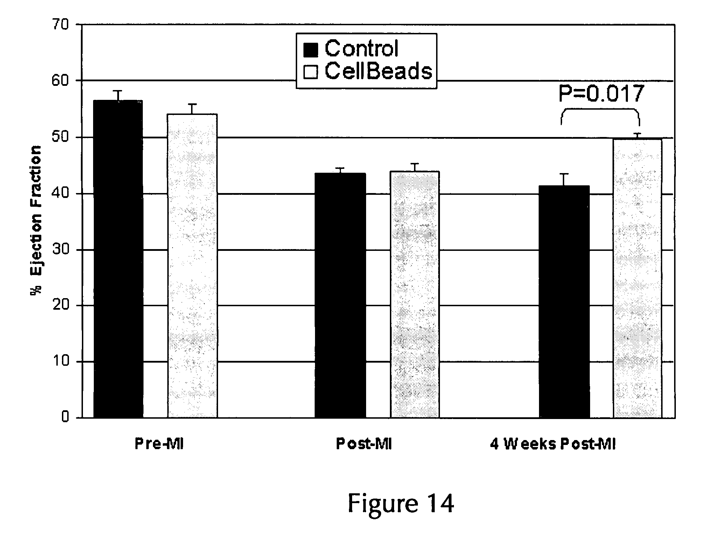

- Fig. 14:: shows the % ejection fraction pre-embolisation, immediately post embolisation and 4 weeks post embolisation demonstrating recovery of the inventive (spherical) microcapsules (CellBeads) group.

The invention is illustrated further in the accompanying examples. However, it is not intended to limit the scope of the invention to the content of the Examples as shown in the following.

### Examples

### Example 1

### Creation of genetic constructs

The coding sequence for GLP-1(7-37) cDNA was synthesized synthetically, in a sequence including Hincll and EcoRI sites as indicated in Fig. 1a. Separately the cDNA illustrated in Fig. 1b was synthesized, including the coding sequences for GLP-1 (7-37), IP2 and restriction sites for *Sfo*I, *Eco*RI and *Xba*I, as illustrated in Fig. 1b. To direct GLP-1 to the secretory pathway, the heterologous signal sequence of stromelysin 3 (Acc. No. NM_005940) was used. Therefore the cDNA, encoding stromelysin signal and leader sequence was reverse transcriptase PCR amplified from human RNA, and used with the construct of Fig. 1a or Fig. 1b to form the construct shown in Fig. 1c and Fig. 1d, respectively.

The *Hin*cII/*EcoR*I fragment of the Fig. 1a construct is cloned into the Sfol site of the sequence of Fig. 1d to form the construct Fig. 1e. Similarly, the *Eco*RI fragment of Fig. 1d is cloned into the *Eco*RI site of an eukaryotic expression plasmid, to produce the construct shown in Fig. 1f. To form the construct shown in Fig. 1g, the *Hinc*II/*Xba*I fragment of the construct shown in Fig. 1b is repetitively cloned into the *Sfo*I/*Xba*I site of the construct shown in Fig. 1d. Figure 1h shows a synthesized, codon optimized sequence encoding the stromelysin leader and signal sequences interrupted by a shortened endogenous intron sequence, fused to sequences encoding human GLP-1(7-37), IP2 and GLP-2(1-35). The DNA sequence of the construct Fig. 1h is SEQ ID NO: 16, while SEQ ID NO: 15 also shows the sequence of the translated peptide.

Also synthesized are the sequences in Figs 1i and 1j. These are then used to form the construct in Fig. 1k, by cloning the *Nae*I*lBss*HII fragment of Fig. 1j into the NaeI/BssHII linearised sequence of Fig. 1h. The DNA sequence of the construct Fig. 1k is SEQ ID NO: 14, while SEQ ID NO: 13 also shows the sequence of the translated peptide. The construct of Fig. 1l is formed by *Bss*I-III digest and religation of the sequence of Fig. 1h. The DNA sequence of the construct Fig. 1l is SEQ ID NO: 18, while SEQ ID NO: 17 also shows the sequence of the translated peptide. The construct of Fig. 1m is formed by cloning the AfeI/BssI-III fragment of the sequence of Fig. 1i into the *Afe*I*lBss*HII linearised sequence of Fig. 1h. The DNA sequence of the construct Fig. 1m is SEQ ID NO: 20, while SEQ ID NO:19 also shows the sequence of the translated peptide.

The above constructs may be made by a person skilled in the art using routine techniques.

### Example 2

### Transfection, clonal selection and GLP-1 expression of mammalian cells

Source of the cells: HEK293 (human embryonic kidney cell line, # ACC 305, DSMZ Cell Culture Collection, Germany), AtT20 (Mouse LAF1 pituitary gland tumour cell line, #87021902, European Cell Culture Collection, UK), hTERT-MSC cells are generated and provided by Prof. Kassem, University Hospital of Odense, Denmark.

For transfection of 10⁶ cells 0.5-2 µg plasmid DNA with different GLP-1 constructs was used. The constructs were generated as described in Example 1. HEK293 cells were transfected by standard calcium phosphate co-precipitation method as described in Current Protocols in Molecular Biology (Ausubel et al. 1994ff Harvard Medical School Vol2., Unit 9.1). AtT20 cells were transfected using FuGene (Roche) as described in Current Protocols in Molecular Biology (Ausubel et. al. 1994ff, Harvard Medical School Vol 2., Unit 9.4). Transfection of hTERT-MSC cells was performed using the Nucleofector technology (Amaxa), a non-viral method which is based on the combination of electrical parameters and cell-type specific solutions. Using the Nucleofector device (program C17) and the Nucleofector solution VPE-1001 transfection efficiencies >60% have been achieved. 48 hours after transfection selection of cell clones with stable integration of DNA into the chromosome was performed by adding the selective agent blasticidin (2 µg/ml) into the culture medium. 12-15 days later, stable transfected cell clones could be isolated and expanded for characterization.

Transient expression of different GLP-1 constructs was measured in hTERT-MSC and HEK293 cells. Whereas only marginal active GLP-1 level can be found in the monomeric GLP-1 constructs #103 and #317 (having just one copy of GLP-1(7-37) an enormous gain in expression can be found in the dimeric GLP-1 construct #217 (having GLP-1(7-37) as component (I) and as component (III)) both in hTERT-MSC and in HEK293 cells. Results are summarized in Figure 2. An elongation of the construct to the GLP-1 construct #159 (having four IP2 copies as component (II)) results in no further significant increase (not shown). After transfection of hTERT-MSC cells with different constructs clones were selected, which stably express GLP-1. The expression levels are shown in Table 1.

**Table 1**

| **construct** | **cell clone** | **active GLP per 10⁶ cells and hour [pmol]** |
|---|---|---|
| #103 GLP1₍₇₋₃₇₎ | 49TM113/13 | 0.4 |
| #317 GLP1₍₇₋₃₇₎-IP2-11aa | 71TM169/1 | 0.6 |
| #217 GLP1₍₇₋₃₇₎-IP2-GLP1₍₇₋₃₇₎ | 79TM217/13 | 2.7 |

### Example 3

### Western Blot Analysis of GLP-1 peptides, secreted from mammalian cells

Cell culture supernatant from GLP-1 secreting cells was separated in a 10%-20% gradient SDS PAGE (120V, 90 minutes) and transferred to a PVDF membrane (Immobilon-P Membrane 0.45 µm Millipore IPVH 00010) by semi-dry blotting (2.0 mA/cm2, 60 minutes). After methanol fixation and blocking (3% (w:v) BSA, 0.1% (v:v) Tween-20 in TBS) the membrane was immunoblotted with 1 µg/ml anti-GLP-1 antibody (HYB 147-12, Antibodyshop) at 4°C o/n. After washing and incubation with 0.02 µg/ml detection antibody (Anti Mouse IgG, HRP conjugated, Perkin Elmer PC 2855-1197) at RT for 4 hours, chemiluminescence detection reveals the location of the protein.

Western Blot Analysis is shown in Figure 3 (1: 100 ng synthetic GLP-1(7-37) dissolved in supernatant of mock transfected hTERT-MSC cells, 2: supernatant of hTERT-MSC cells (clone 79TM217/13) secreting dimeric GLP-1 from construct #217, 3: supernatant of AtT20 cells (clone 81-A-217/3) secreting dimeric GLP-1 from construct #217; M: prestained protein marker [kDa]). The results show that peptides containing GLP-1(7-37) and a C-terminal appendix (2 and 3 in Fig. 3) are secreted from the transfected cell lines and can be detected using an anti-GLP-1 antibody, which binds to the mid-molecular epitopes of GLP-1 (7-37).

### Example 4

### In vitro plasma stability of GLP-1 peptides secreted from human cells

HEK293 and hTERT-MSC cells were transfected with constructs, encoding the heterologous stromelysin signal sequence, which is linked to GLP-1 variants encoding the following peptides:
1: GLP-1(7-37) (construct #103)
2: GLP-1(7-3 7)-IP2-extended with 11 AA (construct #317)
3: GLP1(7-37)-IP2-GLPI (7-37) (construct #217)

Cell culture supernatant, containing GLP-1 peptides secreted from cells or synthetic GLP-1(7-37) (Bachem) was incubated with human lymphocyte enriched plasma containing dipeptidylpeptidase activity at 37°C and 5% CO₂, for 3 or additionaly 6 and 9 hours. Synthetic GLP-1 (7-37) in supernatant from mock transfected cells was used as a positive control for DPP-IV activity, which was shown to be inhibited by addition of a DPP-IV inhibitor (#DPP4, Biotrend). Active GLP was measured using the GLP-1 (Active) ELISA (#EGLP-35K, Biotrend), using an antibody which binds to the N-terminal epitope of GLP-1 (7-37) discriminating the DPP-IV degraded, inactive GLP-1 (9-37) peptide.

The results are shown in Figures 4 (HEK293 cells) and 5 (hTERT-MSC cells). HEK293 and hTERT-MSC cells are both effective hosts for the gene construct. The numbering of the results for the transfected cells is 1: supernatant of cells secreting GLP-1(7-37) from construct #103, 2: supernatant of cells secreting GLP-1 extended by IP2 and 11 aminoacids from construct #317, 3: supernatant of cells secreting dimeric GLP-1 from construct #217. While construct 1 produces wild type GLP-1 which is inactivated by DPP-IV in a similar way to synthetic GLP-1, the C-terminally elongated GLP-1 forms (2 and 3 in Figure 4, 3 in Figure 5) are more resistant to degradation. The C-terminal extended GLP-1 peptides are significantly stabilized in human plasma *in vitro.* The peptide with the dimeric GLP-1 sequence (3) is nearly fully stabilized to DPP-IV degradation *in vitro.*

### Example 5

### In vitro bioactivity of GLP-1 peptides measured by cAMP release

GLP-1(7-37) exerts its biological actions through the seven-transmembrane-spanning, G protein coupled GLP-1 receptor, which leads to activation of protein kinase A signalling through the second messenger cyclic AMP. To ensure that the C terminal elongation of CM1 does not interfere with GLP-1's mode of action, CM1 bioactivity was quantified in an in vitro bioassay, which determines cAMP increase in a GLP-1 receptor expressing cell line after incubation with different concentrations of the peptide. The GLP-1 receptor expressing cell line used for the study (clone 111CH0349/18) is a CHO (chinese hamster ovary) cell line stably transfected with the human GLP-1 receptor. The dose response curves for CM1 produced in the 79TM217/18K5 cells outline the bioactivity of the peptide is shown in figure 7. The peptide dose that produces a half maximal effect (ED50) in the cAMP bioassay has been determined to be 353 pM.

### Example 6

### In vitro human plasma stability of GLP-1^{CM} peptides

Synthetic GLP-1 peptides (SEQ ID NO:1_{syn}, SEQ ID NO:6_{syn}, SEQ ID NO:7_{rec}, SEQ ID NO:8_{syn}) were incubated at concentrations of 20 ng/ml with human plasma at 37°C and 5% CO₂ for 3 hours. Dipeptidylpeptidase activity of the plasma was inhibited by a DPP-IV inhibitor (#DPP4, Biotrend). Active GLP was measured using the GLP-1 (Active) ELISA (#EGLP-35K, Biotrend).

In contrast to the native GLP-1₍₇₋₃₇₎ (SEQ ID NO:1) the C-terminal elongated GLP-1 peptides SEQ ID NO:6, SEQ ID NO:7, and SEQ ID NO:8 are significantly stabilized in human plasma *in vitro* (Fig. 7). As control (on the right hand side) the results obtained for experiments with addition of DPP-IV inhibitor are shown. GLP-1 activity is completely maintained in these control experiments.

### Example 7

### Plasmid creation

The vector for transient and stable gene expression consists of two separate transcription units, one for the gene of interest (GOI) and one for the fusion of the suicide gene HSV thymidine kinase and the resistance gene blasticidin. For the first transcription unit, the human ubiquitin B promoter was used, and for the second transcription unit the human ferritin promoter was used. The plasmid is based on plasmid pCM4, having 7,919 base pairs, shown schematically in Figure 8.

As shown in Figure 8, transcription unit 1, comprises the following components:

**Transcription unit 2,**

| | |
|---|---|
| CMVenh: | immediate early enhancer human cytomegalovirus |
| ubiB human: | ubiquitin promoter B |
| Stro-GLP: | fusion gene, coding for signal peptide and leader sequence of stromelysin and GLPI constructs |
| ori pMBI: | E coli minimal origin of replication. |
| Hygro: | hygromycin B resistance gene. |

| | |
|---|---|
| SV 40 enh: | SV40 enhancer. |
| FerH: | Human ferritin H promoter combined with 5'UTR of the murine EFI gene. |
| Tk-bla: | fusion gene coding for herpes simplex virus type 1 thymidine kinase and blasticidine resistance gene. |

For transient expression the circular plasmid was used. For the selection of stable expressing cell clones, the plasmid was linearised and bacterial sequences (pMB1 origin and hygromycin gene) eliminated.

### Example 8

### Production of mesenchymal stem cell lines or mesenchymal stromal cell lines (MSC).

The *mesenchymal stem cell line* was generated by Prof. Kassem, University Hospital of Odense, Denmark (published in Simonsen et al., 2002, Nature Biotechnology 20m, 592-596) according to following criteria:

### Origin

The production cell line consists of mesenchymal stem cells (MSC), isolated from bone marrow aspirates of a healthy male donor (age 33).

### Immortalisation

Cells were immortalised by introduction of the coding sequence of the telomerase reverse transcriptase. Retroviral transduction was performed by packaging the GCsam retroviral vector in which the expression of the transgene is driven by the Moloney murine leukaemia virus long terminal repeat in PG13. Transduction was performed on day 9 (PDL 12) of culture. The cell line has so far been cultivated until population doubling level (PDL) of 260.

The insertion locus was tested by fluorescence *in situ* hybridization and southern blot. There is only one insertion locus of ecotopic hTERT on chromosome 5 (5q23-31). Analysis was performed at PDL 186. Giemsa banding and comparative genomic hybridization revealed that hMSC-TERT did not develop any numerical or structural chromosomal abnormalities at PDL 96 and maintained a normal diploid male karyotype. Tumourigeneity was tested in immunodeficient mice after subcutaneous implantation for six months and was found negative for PDL 80.

### Flow cytometry (FACS) analysis

Cells were cultured in standard growth medium to 80% confluence. Cells were trypsinised and assayed for size and granularity by FACScan flow cytometer (Becton-Dickinson). For surface marker studies typsinised cells were stained with antibodies directly conjugated to a fluorescent dye (FITC-conjugated mouse anti human CD44 monoclonal antibody, #CBL154F, Cymbus Biotechnology; phycoerythrin-conjugated mouse anti human CD166 monoclonal antibody, #559263, BD Pharmingen) for 30 min on ice. Samples were washed and fixed with 1% of paraformaldehyde until analysis with FACScan (Becton-Dickinson).

### Characterization

Immortalised cells are still able to differentiate into adipocytes, osteocytes and chondrocytes as their non-immortalised counterparts (see Figure 9A). Immortalised cells have fibroblastic morphology and are more homogeneous regarding size and granularity as the mortal MSCs as shown by flow cytometry e.g. using CD 44 and CD166 epitope markers which are characteristic of the primary cells used here. Immortalised cells express the same CD markers as their non immortalised counterparts (see Figure 9B).

**Cultivation**

| | |
|---|---|
| Serum containing medium: | 7% Earles MEM |
| | 10% FCS |
| | 2mM L-Glutamine |
| | 1 mM Sodiumpyruvate |
| | 100 U/ml Penicillin |
| | 0.1 mg/ml Streptomycin |

The population doubling is between 26 and 30 hours.

### Transfection and clonal selection

For transefection of 10⁶ cells 0.5-2µg plasmid DNA with different GLP1 constructs was used. HEK293 cells were transfected by standard calcium phosphate co-precipitation method. AtT20 cells were transfected using FuGene (Roche).

Transfection of hTERT-MSC cells was performed using the Nucleofector technology (amaxa), a non-viral method which is based on the combination of electrical parameters and cell-type specific solutions. Using the Nucleofector device (programme C17) and the Nucleofetor solutionVPE-1001 transfection efficiencies >60% have been achieved.

48 hours after transfection selection of cell clones with stable integration of DNA into the chromosome was performed by adding the selective agent blasticidin (2µg/ml) into the culture medium. 12-15 days later, stable transfected cell clones could be isolated and expanded for characterization.

### Expression

Transient expression of different GLP constructs was measured in hTERT-MSC and HEK293 cells. An active GLP1 level can be found in the monomeric GLP1 constructs #103 (Stro-GLP1₍₇₋₃₇₎) and #317 (Stro-GLP1₍₇₋₃₇₎-IP2-extended with 11aa) and an enormous gain in expression can be found in the dimeric GLP1 construct #217 (Stro-GLPI₍₇₋₃₇₎-IP2-GLP1₍₇₋₃₇₎) both in hTERT-MSC and in HEK293 cells. An elongation of construct #317 to the tetrameric GLP1 construct #159 (Stro-GLP1₍₇₋₃₇₎-IP2 (4x)-11aa) results in an similar activity (see also above Figure 2). After transfection of hTERT-MSC cells with different constructs clones were selected, which stably express GLP1 (see above Figures 4 and 5, Example 4).

### Example 9

### Encapsulation

The cultivated cells to be encapsulated were washed with PBS (PAA, Austria) and separated using trypsin/EDTA (PAA, Austria). The reaction was quickly stopped using medium (dependent on cell type, for example RPMI, PAA, Austria) and the cell suspension centrifuged off (8 min at 1,200 rpm) The pellet was resuspended in PBS and the cell count determined. The desired quantity of 4 x 10⁷ cells was centrifuged off again (8 min at 1,200 rpm). The PBS was then completely removed by suction and 50 µl pellet was resuspended without air bubbles in 50 µl 0.9% saline buffered by 5 mM I-histidine to a pH of 7.4. This cell suspension was taken up in 900 µl of 1.5 - 1.7 % (w/v) sodium alginate solution (an alginate with a viscosity of approximately 5 mPa.s of 0.2 % (w/v) aqueous solution at room temperature was used).

To mix the resuspended cells with the alginate solution, the solution was drawn up in a 1 ml syringe with cannulas and homogeneously mixed with the cells by way of repeated slow drawing up and drawing off. A cell concentration of 4 x 10⁷ cells/ml resulted. For producing the microcapsules with a diameter of about 200 µm, a cannula with an internal diameter of 120 µm was used in an air-charged spray nozzle. An air ring with an opening of 2.0 mm was screwed over the inner cannula. The device is an adapted version of the device described in WO 00/09566. The homogeneous cell/alginate solution mixture was dripped through the described spray nozzle. For this purpose, the 1 ml syringe containing the mixture was placed on the cannula by means of a luer connector. The cell/alginate solution mixture was pressed through the cannula at a speed of 50 µl/min. The airflow was conveyed though the outer air ring at a speed of 2.5 I/min. The resulting microcapsules precipitated into a barium-containing precipitation bath (20 mM BaCl, 5 mM L-histidine, 124 mM NaCl, pH 7.0 ± 0.1, 290 mOsmol ± 3) which was constructed approximately 10 cm below the spray nozzle. After a dwell time of 5 min in the barium-containing precipitation bath the microcapsules were washed five times with 20 ml PBS in each case.

500 µl of the single-layer microcapsules were then taken up in 500 µl of a 1.5 - 1.7 % (w/v) alginate solution the same as used for the core, above and homogeneously mixed. This suspension was taken up in a 1 ml syringe and connected by means of a luer connector to the inner channel (internal diameter: 200 µm) of the spray nozzle and pressed at a speed of 50 µl/min therethrough. A 5 ml syringe with a 1.5 - 1.7 % alginate solution was connected by means of a luer connector to the second inner channel (internal diameter: 700 µm) and pressed there through at a speed of 250 µl/min. The airflow was conveyed through the outer air ring at a speed of 2.9 I/min. The resultant microcapsules precipitated into a barium-containing precipitation bath (20 mM BaCl, 5 mM L-histidine, 124 mM NaCl, pH 7.0 I 0.1, 290 mOsmol ± 3) which is constructed approximately 10 cm below the spray nozzle. After a dwell time of 5 min in the barium-containing precipitation bath, the microcapsules were washed four times with 20 ml PBS in each case and once with medium. Two-layer microcapsules with a total diameter of approximately 180 - 200 µm (including the alginate layer) were produced by this process, wherein the diameter of the inner, cell containing core is 120 - 150 µm.
The concentration of cell in the core is about 4 x 10⁷ cell/ml alginate. This results in (spherical) microcapsules (CellBeads) with a bead volume of 0.002 - 0.004 µl containing approximately 100 cells per bead. A (spherical) microcapsule encoding and secreting GLP-1 produces on average 0.2 fmol active GLP-1 per hour.

A micrograph of Cellbeads containing encapsulated GLP-1 secreting hTERT-MSC cells in the core are shown in Figure 10.

### Example 10

### Anti-apoptotic efficacy of C-terminally elongated GLP- 1

The cytoprotecitve efficacy of the C-terminally elongated GLP-1 analougue CM1 was tested *in vitro* using the rat insulinoma cell line Rin-5F. 40.000 Rin-5F cells were seeded per 96 well and cultivated for 2 days in RPMI supplemented with 1 % L-Glutamin and 10 % fetal calf serum. Apoptosis is induced after change to serumfree conditions (RPMI supplemented with 1 % L-Glutamin) by addition of the protein biosynthesis inhibitor cycloheximid (CHX) in the presence of different concentrations of the recombinantly in E. coli produced dimeric GLP-1 fusion protein CM1. After 24 hours cell viability is quantified using AlamarBlue. A significant anti-apoptotic effect (p<0.01) was observed already in the presence of 1 nM GLP-1 analouge CM1. The results are given in Figure 10.

### Example 11

### Cytokine profile of the GLP-1 producing hTERT-MSC cell line

To investigate GLP-1 independent, cytoprotective effects, the GLP-1 secreting cell line 79TM217/18K5 cell line was examined for the secretion of cytokines, chemokines and growth factors.

The cell line originates from a human stromal cell and therefore secretes a characteristic cytokine profile. A multiplex assay kit (Biosource Cytokine 30-plex) was used for measuring the 30 most abundant human cytokines, chemokines and growth factors simultaneously. No expression was found regarding the cytokines IL-1RA, IL-1β, IL-2, IL-2R, IL-4, IL-5, IL 7, IL-10, IL-12(p40/p70), IL-13, IL-15, IL-17, IP-10, EGF, Eotaxin, FGF-basic, IFN-a, IFNγ, GM CSF, G-CSF, HGF, MIG, MIP-b, MIP-1α, RANTES and TNFα (detection limit of each analyte 20 pg per 10⁵ cells and 24h). The cytokines, which are expressed at detctable levels are summarized in table 1.

Table 1: Expression level of growth factors Vascular endothelial growth factor (VEGF), neurotrophin-3 (NT-3), glial cell line-derived neurotrophic factor (GDNF) and the cytokines Interleukin 6 (IL-6), Interleukin 8 (IL-8) and Monocyte chemotactic protein 1 (MCP-1). The factors have been quantified in cell culture supernatant of the CM1 secreting cell line 79TM217/18K5 using the VEGF ELISA (#ELH-VEGF-001; RayBio), NT-3 ELISA (#TB243, Promega), GDNF ELISA (#TB221, Promega) and the the human IL-6, IL-8 and MCP-1 ELISA Kits (RayBio).

| Growth factor / Cytokine [pg / 10⁶ cells and hour] | 79TM217/18K5 |
|---|---|
| VEGF | 973.0 ± 78.3 |
| NT-3 | 20.9 ± 3.5 |
| GDNF | 10.7 ± 1.5 |
| IL-6 | 378.4 ± 4.0 |
| IL-8 | 3608.7 ± 53.8 |
| MCP-1 | 16.8 ± 0.1 |

### Example 12

### Preparation of Cell Beads for a novel cell-based therapy for myocardial ischemia and preservation

Cells encoding and secreting GLP-1 as defined above and (spherical) microbeads containing these cells (GLP-1 CellBeads^{®}) for implantation into the myocardium of patients affected by acute myocardial infarction are being developed according to good manufacturing practice (GMP) according to the experiments as shown above in previous Examples 1 to 11. The intention of this experiment is to limit damage of heart tissue by extended local delivery of GLP-1 to the myocardium. GLP-1 CellBeads^{®} may be directly delivered to the affected site of the heart by interventional means, using a catheter to navigate to the affected area and implant the beads by injection into the myocardial tissue. Implantation could be performed during routine angioplasty post AMI. The *in situ* cardioprotective effect of GLP-1 CellBeads^{®} is due to the local secretion of a glucagon-like peptide-1 (GLP-1) fusion protein and other paracrine factors that are continuously delivered to the site of injury.

The GLP-1 CellBeads^{®} as used for this experiment consist of cells from a human mesenchymal stromal cell line, embedded in a spherical shaped alginate matrix (180 - 200 µm in diameter). The cells are designed to secrete GLP-1 fusion protein which seem to have an antiapoptotic effect. The alginate matrix, which entraps the cells, is generated during the herein described production process by cross-linking of the alginate with barium ions. The alginate itself has no pharmacological effect but provides a mechanical scaffold for the cells and protects them against attacks of the patient's immune system (see Figure 11). Therefore, the alginate is regarded as an excipient. From the patient's view, the alginate matrix is considered as a safety component, because it restricts the cells locally to the point of application and prevents a free floating of the cells. To fulfil these functions properly, GLP-1 CellBeads^{®} are composed of a corebead, which is an alginate matrix enclosing the GLP-1 fusion protein secreting cells. This corebead again is surrounded by a shell consisting of pure alginate to assure the complete encapsulation of all cells.

The cells, which are encapsulated within the alginate and secrete the GLP-1 fusion protein are derived from the clone 79TM217/18K5 and defined as the drug substance. The cells are human mesenchymal stromal cells (hMSC), which are genetically modified to produce the anti apoptotic glucagon-like peptide-1 (GLP-1) fusion protein as defined herein. The parental cell line is a non-tumourigenic cell line with normal karyotype. The cell line 79TM217/18K5 was generated at CellMed AG by genetically modifying the parental cell line. Therefore, a transfection with a plasmid vector encoding the GLP-1 fusion protein was performed.

The GLP-1 fusion protein is a dimeric GLP-1 construct, which is arranged in analogy to the native preproglucagon gene. It is a 79 amino acid dimeric GLP-1 / Intervening Peptide 2 (IP-2) / GLP-1 protein with a molecular weight of 8.7 kDa and corresponds to SEQ ID No 10 in the accompanied sequence listing.

The advantages of this C-terminally elongated GLP-1 fusion protein in comparison to the native GLP-1 are the higher expression levels and the decreased susceptibility to naturally occurring degradation by dipeptidyl peptidase IV. Bioactivity of the fusion protein is maintained. For safety reasons, the plasmid used allows co-expression of the GLP-1 fusion protein and a suicide gene. The suicide gene codes for the most widely used Herpes simplex virus Type 1 Thymidine Kinase (HSV1tk). This enzyme converts intracellularly the non-toxic prodrug Ganciclovir into a toxic product, therefore allowing the destruction of transfected cells in the case of unexpected cell proliferation. Thus, systemic application of Ganciclovir to a patient treated with GLP-1 CellBeads^{®} containing degenerated cells leads to destruction of the transplanted cells. The characterization of the cell line 79TM217/18K5 was performed taking into account the recommendations of the ICH Q5B as set forth in:
"Analysis of the Expression Construct in Cells Used for Production of r-DNA Derived Protein Products"; and
"Q5D Derivations and Characterisation of Cell Substrates Used for Production of Biotechnological /Biological Products"; and the
"Note for Guidance on the Quality, Preclinical and Clinical Aspects of Gene Transfer Medicinal Products" (EMEA/273974/2005).

### Example 13

### Proof of Concept Study: Early evaluation of genetically engineered mesenchymal stromal stem cell therapy for prevention of LV dysfunction in pigs

**Background:** Heart Failure (HF) is still associated with a poor prognosis with almost 50% of patients dying within five years of diagnosis. Glucagon-like Peptide-1 (GLP-1) is a naturally occurring gut incretin hormone that stimulates insulin secretion and has anti-apoptotic properties. Infusion of GLP-1 after successful primary angioplasty has previously been shown to reduce left ventricular systolic dysfunction (LVSD). We hypothesised that a prolonged delivery of GLP-1 expressing cells at the time of an MI would improve LV function and significantly reduce infarct size in a porcine model of early LV dysfunction.

Previous work has shown that bead embolisation of the coronary arteries creates a reproducible infarct. In order to evaluate the effects of locally delivered GLP-1 on the size and nature of the infarct, GLP-1 secreting CellBeads^{®} were compared to control (non-GLP-1 secreting beads of the same size and number).

**Methods:** Human mesenchymal stromal cells immortalised and engineered to produce GLP-1 as defined above in Experiments 1-12 were encapsulated into alginate beads in order to immuno-isolate the cells (GLP-1 CellBeads^{®}). These beads were selectively delivered to branches of left anterior descending coronary artery in Yorkshire White pigs (n=6), with the control group receiving cell-free alginate beads (n=6). Four weeks after intervention, hearts were explanted for morphometric quantification of infarcted surface area and histological analysis. **Results:** Acute animal loss was 2 in treatment and 1 in control group. In the surviving animals, transthoracic echocardiography confirmed onset of mild LVSD (EF 40-45%) in both groups. LV surface area morphometry showed significantly decreased infarction area in the treatment group, compared to control group (21.8 ± 4.8% vs 4.7±2.1%; p<0.018). Histological analysis showed similar patterns of fibrosis and moderately enhanced inflammation in the treatment group. Further work is aimed at investigating the effect of GLP-1 CellBeads^{®} on calcium regulatory proteins, apoptosis and angiogenesis.

**Conclusions:** Delivery of alginate encapsulated mesenchymal stromal cells expressing GLP-1 (GLP-1 CellBeads^{®}) as described according to the present invention significantly reduces infarct size and LVSD in a porcine model of early LV dysfunction.

The results of example 13 are shown in Figures 12, 13 and 14. Fig. 12 shows a gross pathology of a heart in which coronary arteries have been embolised (black dotted lines) with alginate beads (A) or inventive (spherical) microcapsules (CellBeads) (B). The patches outlines in white on (A) represent areas of visible infarcted area on the surface of the heart, whereas very little infarcted area in visible in (B). Fig. 13 shows the % of the LV infarct area on the surface of the hearts embolised with alginate control *versus* inventive (spherical) microcapsules (CellBeads). Fig. 14 shows the % ejection fraction pre-embolisation, immediately post embolisation and 4 weeks post embolisation demonstrating recovery of the inventive (spherical) microcapsules (CellBeads) group.

## Claims

1. Use of cells, encoding and secreting GLP-1, a fragment or variant thereof or a fusion peptide comprising GLP-1 or a fragment or variant thereof, for the treatment of acute myocardial infarction (AMI or MI), wherein the cells, encoding and secreting GLP-1, a fragment or variant thereof or a fusion peptide comprising GLP-1 or a fragment or variant thereof, are encapsulated in a spherical microcapsule to prevent a response of the immune system of the patient to be treated.

2. Use according to claim 1, wherein the spherical microcapsule preferably comprises a spherical core and at least one surface coating,
wherein the spherical core comprises or consists of a mixture of cross-linked polymers and cells, encoding and secreting GLP-1, a fragment or variant thereof or a fusion peptide comprising GLP-1 or a fragment or variant thereof; and
wherein the at least one surface coating comprises or consists of cross-linked polymers.

3. Use according to claim 1 or 2, wherein the spherical microcapsule has a total diameter of about 120 µm to about 300 µm, a total diameter of about 150 µm to about 250 µm, a total diameter of about 165 µm to about 225 µm, or a total diameter of about 180 µm to about 200 µm, including a total diameter of about 180, 185, 190 or 200 µm.

4. Use according to any of claims 1 to 3, wherein the cells are mesenchymal stem cells, mesenchymal stromal cells, human mesenchymal stem cells, differentiated cells derived from human mesenchymal stem cells, allogenic cells or autologous cells encoding and secreting GLP-1, a fragment or variant thereof or a fusion peptide comprising GLP-1 or a fragment or variant thereof.

5. Use according to any of claims 2 to 4, wherein the cross-linked polymer of the core and/or the at least one surface coating comprises biopolymers.

6. Use according to any of claims 2 to 5, wherein the cross-linked polymer of the core and/or the at least one surface coating comprises an alginate.

7. Use according to any of claims 2 to 6, wherein the cross-linked polymer of the core and/or the at least one surface coating comprises a chemically identical polymer in identical or differing concentrations, wherein the polymers further may have different molecular weights and/or may be cross-linked differently.

8. Use according to any of claims 1 to 7, wherein the spherical microcapsule according comprises 1, 2, 3, 4, 5, 5-10 or more surface coatings.

9. Use according to any of claims 1 to 8, wherein the spherical microcapsule comprises an additional external surface coating consisting of polycations.

10. Use according to any of claims 1 to 9, wherein the GLP-1 is a peptide selected from group consisting of:
a) a peptide comprising aa 7 - 35 of GLP-1; or
b) a peptide comprising aa 1 - 37 of GLP-1; or
c) a peptide comprising aa 7 - 37 of GLP-1; or
d) a peptide comprising aa 7 - 36 of GLP-1 or GLP-1 (7-36)amide; or
e) a peptide comprising the sequence according to formula II:
Xaa7-Xaa8-Glu-Gly-Thr-Phe-Thr-Ser-Asp-Xaa 16-Ser-Xaa 18-Xaa 19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is Ser, Lys or Arg; Xaa19 is Tyr or Gln; Xaa20 is Leu or Met; Xaa22 is Gly, Glu or Aib; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent; or
f) a peptide comprising a peptide according to formula III:
Xaa7-XaaB-Glu-Gly-Thr-Phe-Thr-Ser-Asp-Val-Ser-Xaa8-Tyr-Leu-Glu-Xaa22-Xaa23-Ala-Ala-Xaa26-Glu-Phe-Ile-Xaa30-Trp-Leu-Val-Xaa34-Xaa35-Xaa36-Xaa37,
wherein Xaa7 is L-histidine, D-histidine, desamino-histidine, 2-amino-histidine, -hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent, or
g) a peptide showing a homology of at least 80 % with any of the above peptides according to a) to f).

11. Use according to any of claims 1 to 9, wherein the GLP-1 fusion peptide or a fragment or variant thereof comprises components (I) and (II),
wherein component (I) N-terminally is selected from consisting or comprising
a) a GLP-1(7-35, 7-36 or 7-37) sequence, or
b) a sequence according to SEQ ID NO: 1; or
b) a peptide comprising or consisting of the sequence according to formula II:
Xaa7-Xaa8-Glu-Gly-Thr-Phe-Thr-Ser-Asp-Xaa 16-Ser-Xaa 18-Xaa 19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is Ser, Lys or Arg; Xaa19 is Tyr or Gln; Xaa20 is Leu or Met; Xaa22 is Gly, Glu or Aib; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent; or
c) a peptide comprising or consisting of the sequence according to formula III:
Xaa7-Xaa8-Glu-Gly-Thr-Phe-Thr-Ser-Asp-Val-Ser-Xaa8-Tyr-Leu-Glu-Xaa22 -Xaa23-Ala-Ala-Xaa26-Glu-Phe-Ile-Xaa30-Trp-Leu-Val-Xaa34-Xaa35-Xaa36-Xaa37,
wherein Xaa7 is L-histidine, D-histidine, desamino-histidine, 2-amino-histidine, -hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg; Xaa26 is Lys, Glu or Arg;Xaa30 is Ala, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent; or
d) a sequence selected from a sequence according to SEQ ID NO: 8 (HAEGTFTSDVSSYLEGQAAKEFIAWLVKGRGRRDFPEEVAIAEELG); or
e) a sequence selected from a sequence according to SEQ ID NO: 12 (HAEGTFTSDVSSYLEGQAAKEFIAWLVKGRGRRDFPEEVAIVEELG)
d) or a sequence having at least 80 % sequence homology with a sequence of any of sequence according to a) to e); and
component (II) C-terminally is selected from a peptide sequence of at least 9 amino acids or a functional fragment or variant thereof.

12. Use according to claim 11, wherein component (II) of the GLP-1 fusion peptide, is selected from:
a) a peptide sequence containing a sequence according to SEQ ID NO: 22 (RRDFPEEVAI) or a sequence having at least 80% sequence homology with SEQ ID NO: 22; or
b) a peptide sequence containing a sequence according to SEQ ID NO: 23 (RRDFPEEVAIVEEL) or SEQ ID NO: 24 (RRDFPEEVAIAEEL) or a sequence having at least 80% sequence homology with SEQ ID NOs: 23 or 24; or
c) a peptide sequence containing a sequence according to SEQ ID NO: 2 (RRDFPEEVAIVEELG) or SEQ ID NO: 3 (RRDFPEEVAIAEELG) or a sequence having at least 80% sequence homology with SEQ ID NOs: 2 or 3.

13. Use according to any of claims 11 or 12, wherein component (I) and component (II) of the GLP-1 fusion peptide are directly linked or linked *via* a linker sequence.

14. Use according to any of claims 11 to 13, wherein the GLP-1 fusion peptide contains alternatively or additionally to components (I) and (II) a component (III), wherein component (III) may be linked to the C-terminus of component (I) and/or to the N-terminus of component (I), if components (I) and (III) are present in the fusion protein, or wherein component (III) may be linked to the C-terminus of component (II) and/or to the N-terminus of component (I), if components (I), (II) and (III) are present in the fusion protein.

15. Use according to any claim 14, wherein component (III) comprises at least four amino acid residues, preferably at least 10 additional amino acid residues, more preferably at least 20, or most preferably at least 30.

16. Use according to any claim 15, wherein component (III) comprises at least 4, preferably at least 10, more preferably at least 20 additional amino acid residues of
a) the N-terminal sequence of GLP-2 as in proglucagon, or
b) a GLP-1 (5-37, 6-37, or 7-37) sequence, or
c) a peptide comprising the sequence according to formula II:
Xaa7-Xaa8-Glu-Gly-Thr-Phe-Thr-Ser-Asp-Xaa16-Ser-Xaa18-Xaa19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine, D-histidine, desamino-histidine, 2-amino-histidine, 3-hydroxy-histidine, homohistidine, N-acetyl-histidine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa is Ser, Lys or Arg; Xaa19 is Tyr or Gln; Xaa20 is Leu or Met; Xaa22 is Gly, Glu or Aib; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent; or
d) a peptide comprising a sequence according to formula III: 
Xaa7-Xaa8-Glu-Gly-Thr-Phe-Thr-Ser-Asp-Val-Ser-Xaa8-Tyr-Leu-Glu-
Xaa22-Xaa23-Ala-Ala-Xaa26-Glu-Phe-Ile-Xaa30-Trp-Leu-Val-Xaa34-
Xaa35-Xaa36-Xaa37,
 wherein Xaa7 is L-histidine, D-histidine, desamino-histidine, 2-amino-histidine, -hydroxy-histidine, homohistidine, N-acetyl-histdine, α-fluoromethyl-histidine, α-methyl-histidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent.
e) or a sequence having at least 80 % sequence homology with a sequence of any of sequence according to a) to d).

17. Use according to any claim 14, wherein component (III) contains the sequence of SEQ ID NOs: 4 or 5 or a sequence having at least 80% sequence homology with SEQ ID NOs: 4 or 5.

18. Use according to any of claims 11 to 17, wherein the GLP-1 fusion peptide additionally contains or comprises a carrier protein, in particular transferrin or albumin, as component (IV).

19. Use according to any of claims 1 to 18, wherein the fusion peptide contains or consists of a peptide sequence selected from a group consisting of: SEQ ID NO: 6, SEQ ID NO: 7, SEQ ID NO: 10, SEQ ID NO: 11 and SEQ ID NO: 26, or a sequence having at least 80% sequence homology with SEQ ID NOs: 6, 7, 10, 11 or 26.

20. Use according to any of claims 1 to 19, wherein the cells in the core of the spherical microcapsules are engineered to additionally secrete a factor selected from the group consisting of anti-apoptotic factors, growth factors, VEGF and erythropoietin (EPO), anti-platelet drugs, anti-coagulant drugs, and anti-thrombotic drugs.

21. Use according to any of claims 1 to 19, wherein the cells in the core of the spherical microcapsules secrete endogenous proteins or peptides as paracrine factors that are released through the capsule in therapeutic levels selected from VEGF, IL6, IL8, GDNF, NT3, and MCPI.

22. Use according to any of claims 1 to 21, wherein the spherical microcapsules are implanted into the affected area of myocardium of a mammalian AMI or post AMI patient by direct injection into the heart tissue, or by intravascular delivery through the arterioles feeding the affected heart tissue.
